(19) Europäisches Patentamt
European Patent Office
Office européen des brevets

(11) **EP 4 214 335 B1**

(12) **EUROPEAN PATENT SPECIFICATION**

(45) Date of publication and mention
of the grant of the patent:
**17.06.2026 Bulletin 2026/25**

(21) Application number: **21819526.1**

(22) Date of filing: **10.12.2021**

(51) International Patent Classification (IPC):
**C12Q 1/6886** (2018.01)

(52) Cooperative Patent Classification (CPC):
**C12Q 1/6886; C12Q 2600/118; C12Q 2600/178**

(86) International application number:
**PCT/EP2021/085141**

(87) International publication number:
**WO 2022/122994 (16.06.2022 Gazette 2022/24)**

(54) **PROGNOSTIC METHOD FOR AGGRESSIVE LUNG ADENOCARCINOMAS**

PROGNOSTISCHES VERFAHREN FÜR AGGRESSIVE LUNGENADENOKARZINOME

PROCÉDÉ DE PRONOSTIC D'ADÉNOCARCINOMES PULMONAIRES AGRESSIFS

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR

(30) Priority: **11.12.2020 EP 20213323**

(43) Date of publication of application:
**26.07.2023 Bulletin 2023/30**

(73) Proprietor: **Fondazione di Religione e di Culto
"Casa Sollievo Della Sofferenza" - Opera di San
Pio da Pietrelcina
71013 San Giovanni Rotondo (FG) (IT)**

(72) Inventors:
• **BIANCHI, Fabrizio**
23900 Lecco (LC) (IT)
• **DAMA, Elisa**
13878 Candelo (BI) (IT)
• **MELOCCHI, Valentina**
24010 Ponteranica (BG) (IT)

(74) Representative: **Dragotti & Associati S.P.A. et al
Via Nino Bixio, 7
20129 Milano (MI) (IT)**

(56) References cited:
**AU-B2- 2012 238 336     CN-B- 104 818 334**

• XUELIAN LI ET AL: "An eight-miRNA signature as a potential biomarker for predicting survival in lung adenocarcinoma", JOURNAL OF TRANSLATIONAL MEDICINE, BIOMED CENTRAL, vol. 12, no. 1, 4 June 2014 (2014-06-04), pages 159, XP021188127, ISSN: 1479-5876, DOI: 10.1186/1479-5876-12-159
• SIRIWARDHANA CHATHURA ET AL: "Development of a miRNA-seq based prognostic signature in lung adenocarcinoma", vol. 19, no. 1, 1 December 2019 (2019-12-01), XP055803659, Retrieved from the Internet <URL:https://bmccancer.biomedcentral.com/track/pdf/10.1186/s12885-018-5206-8.pdf> DOI: 10.1186/s12885-018-5206-8
• PENG ZHUO ET AL: "Identification of microRNAs as potential biomarkers for lung adenocarcinoma using integrating genomics analysis", ONCOTARGET, vol. 8, no. 38, 8 September 2017 (2017-09-08), United States, pages 64143 - 64156, XP055803811, ISSN: 1949-2553, DOI: 10.18632/oncotarget.19358
• YERUKALA SATHIPATI SRINIVASULU ET AL: "Identifying the miRNA signature associated with survival time in patients with lung adenocarcinoma using miRNA expression profiles", vol. 7, no. 1, 1 December 2017 (2017-12-01), XP055804115, Retrieved from the Internet <URL:https://www.nature.com/articles/s41598-017-07739-y.pdf> DOI: 10.1038/s41598-017-07739-y

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **ANONYMOUS: "miProfile TM Human miRNome miRNA qPCR Array(96-well)", 1 January 2016 (2016-01-01), XP055803970, Retrieved from the Internet <URL:https://www.biocat.com/ genomics/microrna/microrna-qpcr-arrays> [retrieved on 20210512]**

Remarks:
    The complete document including Reference Table(s) and the Sequence Listing(s) can be downloaded from the EPO website

## Description

The present invention relates to methods based on miRNA biomarkers for the prognosis of aggressive lung adenocarcinoma (ADC) including early-stage (stage I) disease, preferably in fresh-frozen or in formalin-fixed, paraffin-embedded (FFPE) specimens. More in particular, the invention refers to the use of 7-miRNA, 14-miRNA or 19-miRNA prognostic signatures in a method for prognostic risk stratification of ADC, preferably for identify patients with aggressive early-stage lung adenocarcinoma (namely C1-ADC).

## Background of the invention

Latest global lung cancer data indicate a burden of 2.09 million new cases and 1.76 million deaths in 2018 [1]. The main type of lung cancer is represented by Non-Small-Cell Lung Cancer (NSCLC) (80-85%) including several heterogeneous tumor subtypes, among which lung adenocarcinoma (ADC) accounts for ~40% of all lung cancer cases. Primary and secondary prevention strategies such as anti-smoking campaigns and the implementation of large CT screening programs resulted in a reduction of lung cancer mortality of a ~20% (as observed in NELSON and NLST trials) and progressive lung cancer stage-shift [2,3]. However, the high level of molecular heterogeneity of lung cancer enhances the metastatic dissemination of a large fraction of aggressive early stage tumors (~30-50%) [4].

In-depth molecular and functional characterization of ADC could help to contextualize tumor heterogeneity in specific molecular subtypes which may suggest alternative therapeutic options. We recently described a 10-gene prognostic signature for stage I ADC which identified a subset of tumors, namely C1-ADC [5,6], with peculiar gene/protein expression and genetic alterations resembling more advanced cancer.

This prognostic gene signature can be measured by quantitative real-time PCR (qRT-PCR) or digital PCR (dPCR), Affymetrix or RNA-sequencing, or direct digital detection (e.g. Nanostring technology), in fresh-frozen or in formalin-fixed, paraffin-embedded (FFPE) specimens [6].

To foster clinical translation of this 10-gene signature, here we present a miRNA signature as a surrogate of the 10 genes, for prognostic risk stratification of ADC, in particular to identify patients with aggressive early-stage lung adenocarcinomas. A miRNA-based prognostic signature would overcome the problem of using low-quality mRNA when extracted from FFPE samples, which are routinely used for diagnostic purposes. Indeed, shorter non-coding RNA molecules such as miRNA are more resistant to harsh conditions [7,8] and compatible with most of the expression profiling methods including qRT-PCR.

Some prior art discloses the use of detecting miRNAs to diagnose cancer, such as lung cancer. However, no prior art document discloses a prognostic method based on a specific miRNA signature that effectively works for detecting patients with the aggressive ADC subtype i.e. the C1-ADC, and that can be applied also by using fresh-frozen or in formalin-fixed, paraffin-embedded (FFPE) specimens.

For example, WO2012/089630A1 discloses a method to identify asymptomatic high-risk individual with early-stage lung cancer in biologic fluids, by means of detecting at least 5 miRNAs within a list of 34 miRNA.

WO2016/038119 and Bianchi F. et al. [14] discloses a method for diagnosing lung cancer in a subject by detecting a decrease and an increased abundance of different miRNAs in a blood sample obtained from that patient, the presence of which provides an earlier indication of cancer than alternative art-recognized methods, including, but not limited to, low-dose computed tomography (LDCT).

The use of microRNA markers for the prognosis of lung adenocarcinoma is described in AU 2012238336 B2, CN 104818334 B, Xuelian Li et al., Journal of Translational Medicine, 4 June 2014; Siriwardhana Chathura et al., BMC Cancer, 1 December 2019; Peng Zhuo et al., Oncotarget, 8 September 2017; Yerukala Sathipati Srinivasulu et al., Scientific Reports, 1 December 2017.

There is therefore an urgent need of prognostic biomarkers and of a method to identify patients with early-stage aggressive lung cancer, who could eventually benefit from systemic adjuvant chemotherapy (i.e. platinum-based) rather than molecular targeted/immune-therapies and that can be applied to different kind of body fluids or tissue samples, including FFPE specimens.

## Brief description of the figures.

**Figure 1.**
Flow chart of study design with data sets and analysis.

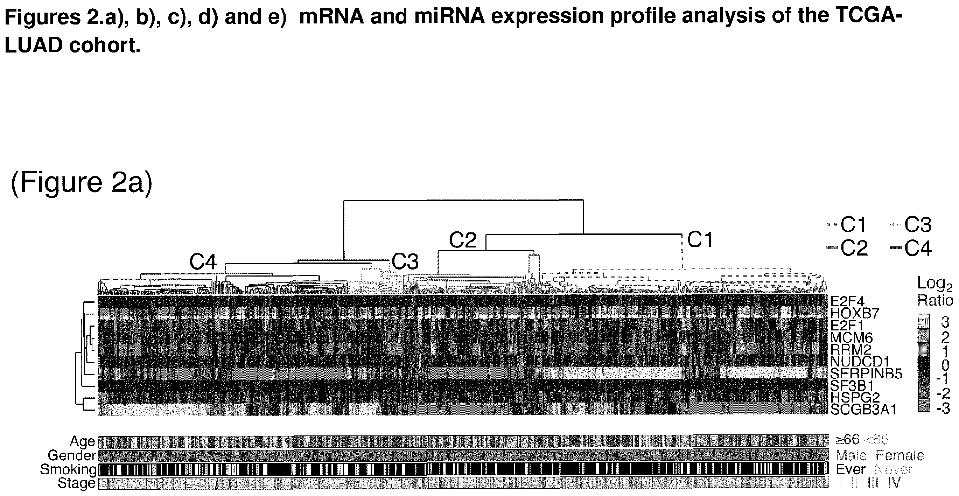

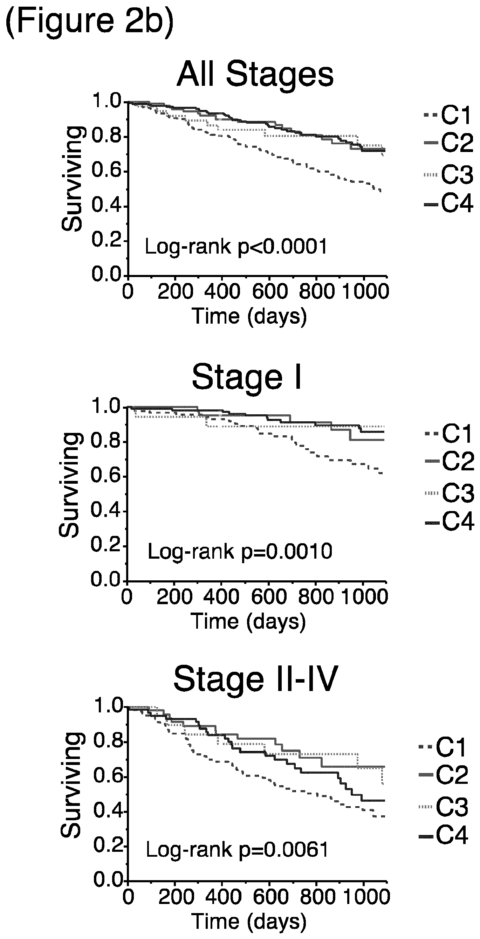

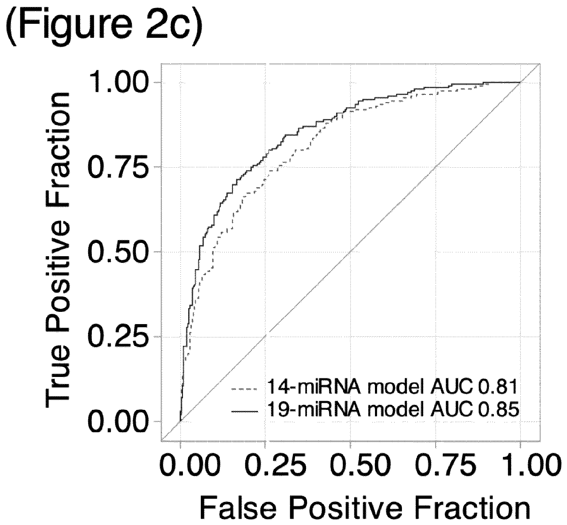

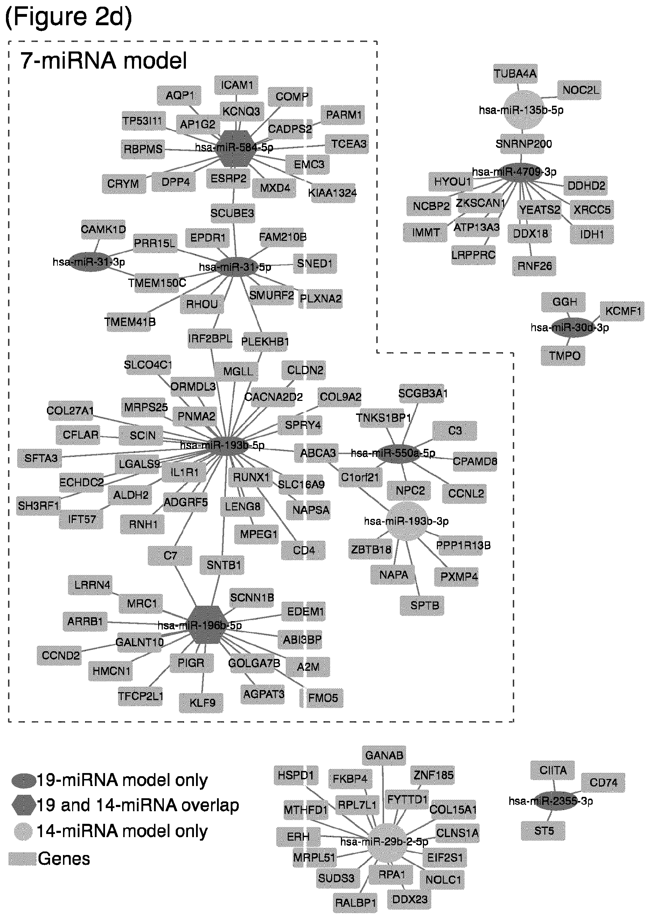

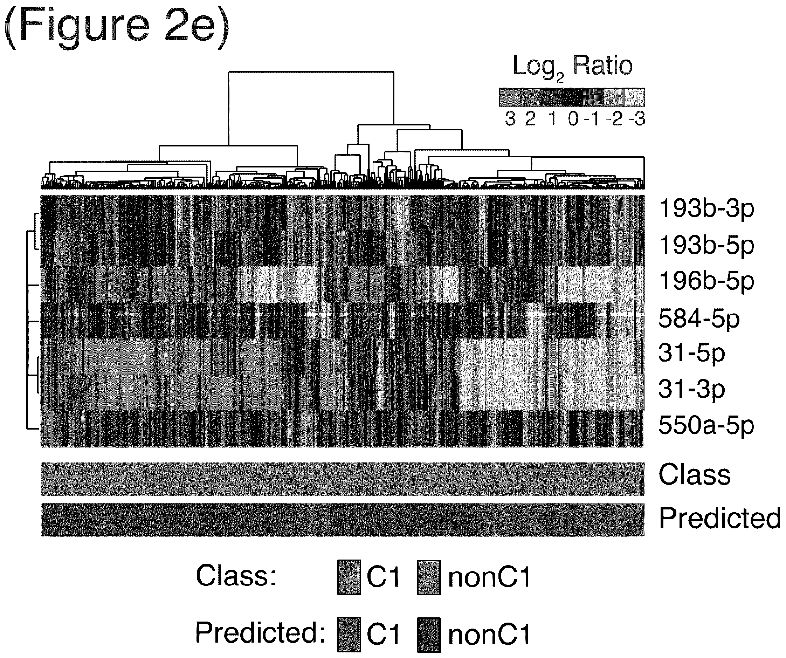

**Figure 2. mRNA and miRNA expression profile analysis of the TCGA-LUAD cohort.**
(a) Hierarchical clustering analysis of the 10-gene expression signature. C1-C4 clusters are colored as per the legend. Age, gender, smoking status and stage are colored as per the legend. (b) Kaplan-Meier curves for 3-years overall survival stratified by C1-C4 clusters. Log-rank p-values are shown for C1 vs nonC1 patients (C2-C4) comparison. (c) Receiver operating characteristic (ROC) curves showing the False Positive Fraction and True Positive Fraction of the 19- (solid line) and 14-miRNA (dashed line) models. The areas under curve (AUC) are reported. (d) Networks of miRNA derived from 19-, 14- and 7-miRNAs model and corresponding target genes. Rectangles represent genes (mRNA); ellipses represent miRNA from 19-miRNA model; circles represent miRNA from 14-miRNA model; hexagons represent miRNA from both 14- and 19-miRNA model. (e) Hierarchical clustering of 7-miRNAs in the TCGA-LUAD cohort. C1 and nonC1 tumors (defined according to the 10-gene signature) are colored as per the legend. Predicted C1 and nonC1 tumors (defined according the 7-miRNA logistic model) are colored as per the legend.

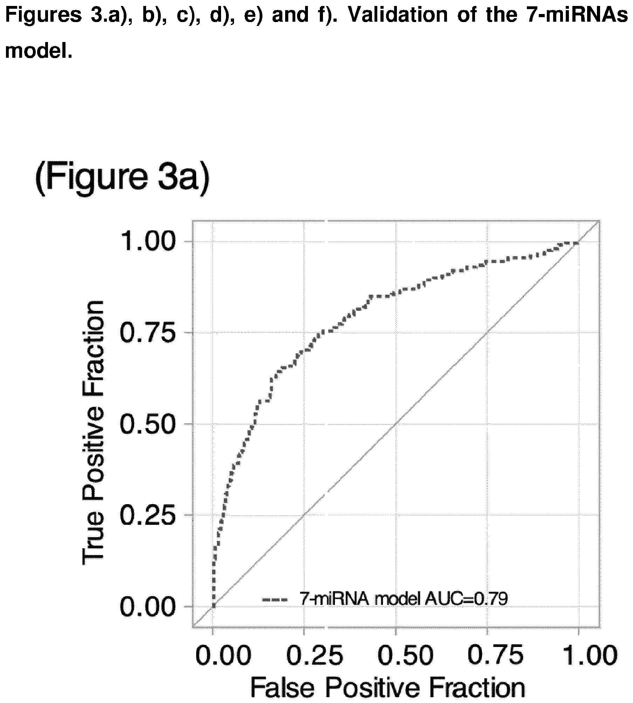

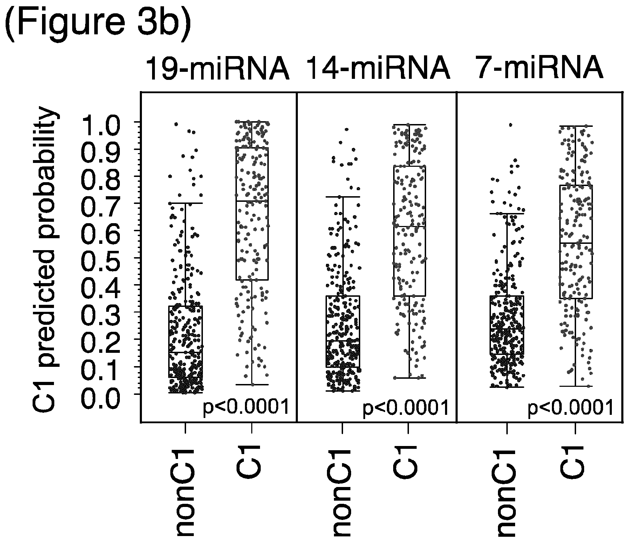

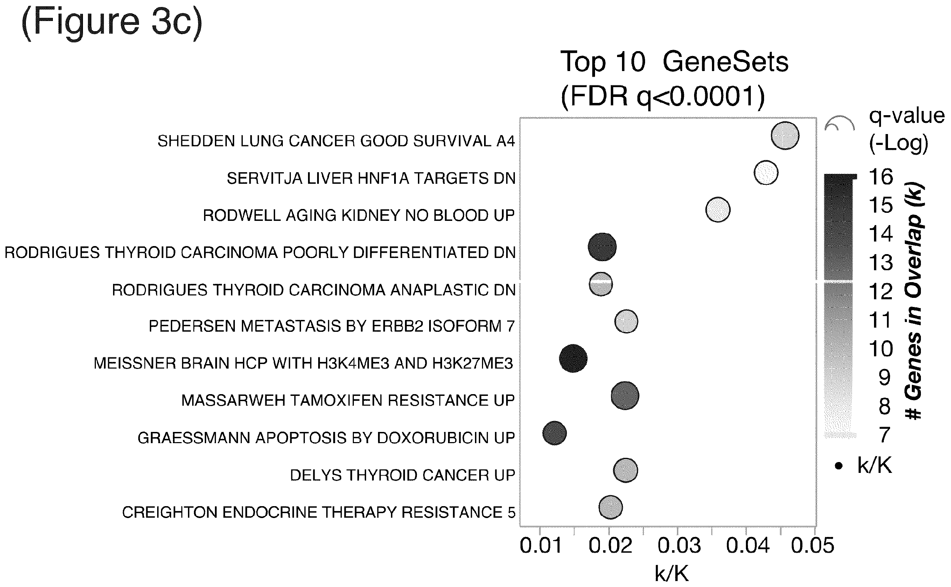

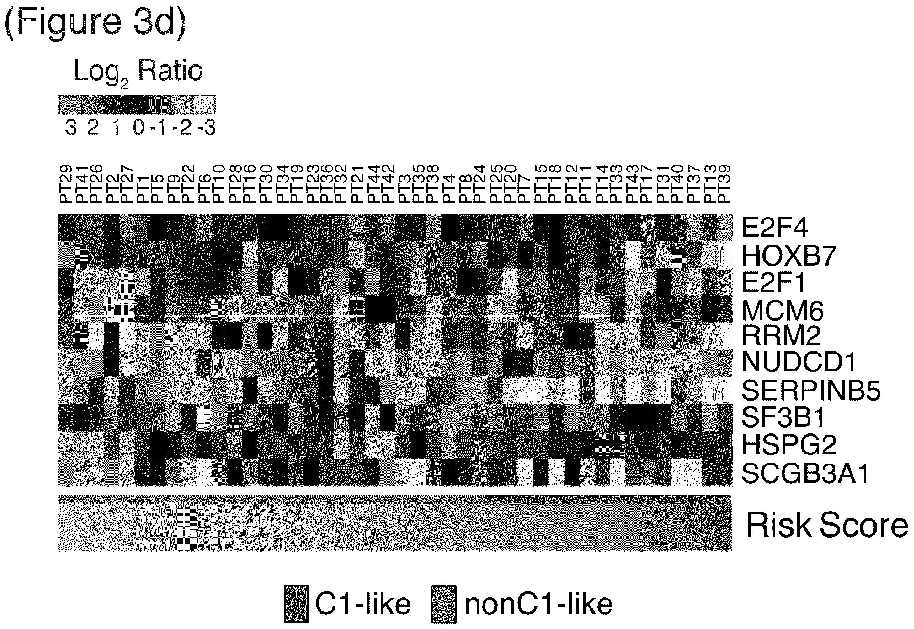

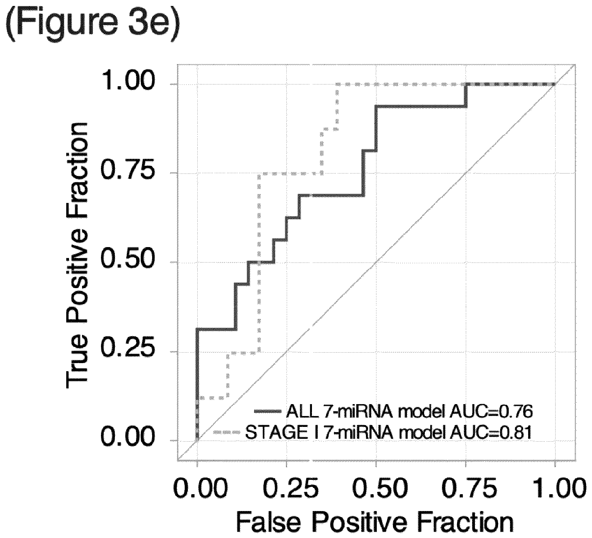

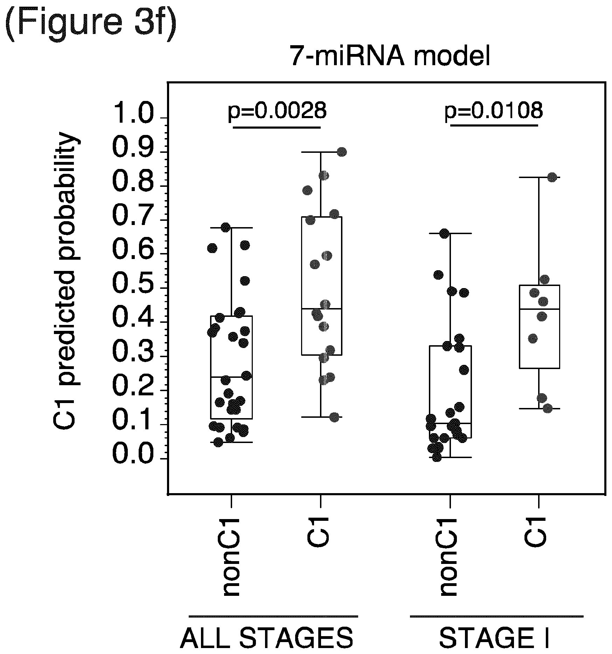

**Figure 3. Validation of the 7-miRNA model.**

(a) ROC curve showing the False Positive Fraction and True Positive Fraction of the 7-miRNA model. The AUC is reported. (b) Box-plot for C1 predicted probability in C1 (light grey dots) and nonC1 (black dots) patients. Predicted probabilities are calculated through the 19-, 14- and 7-miRNA models. Wilcoxon-Mann-Whitney test p-values are reported. (c) Bubble plot of top 10 GeneSets found significantly overlapping with gene networks targeted by the 7-miRNA signature. Bubbles size is proportional to statistical significance (-Log of FDR q-value) and color codes refer to number of genes found in the overlap. In X-axis, ratios (k/K) of overlap of the query set of genes (k) with overlapping GeneSet size (K). (d) Heatmap of the 10-gene expression of CSS cohort. C1 and nonC1 tumors are colored as per the legend. Risk scores are calculated based on the 10-gene risk model. (e) ROC curves showing the False Positive Fraction and True Positive Fraction of the 7-miRNA model in the CSS cohort, for all stages (solid black line) or only stage I tumors (dashed light grey line). The AUC are reported. (f) Box-plot for C1 predicted probability in C1 (light grey dots) and nonC1 (black dots) tumors in CSS cohort, for all stages tumors and stage I tumors. Predicted probabilities are calculated through the 7-miRNA model. Wilcoxon-Mann-Whitney test p-values are reported.

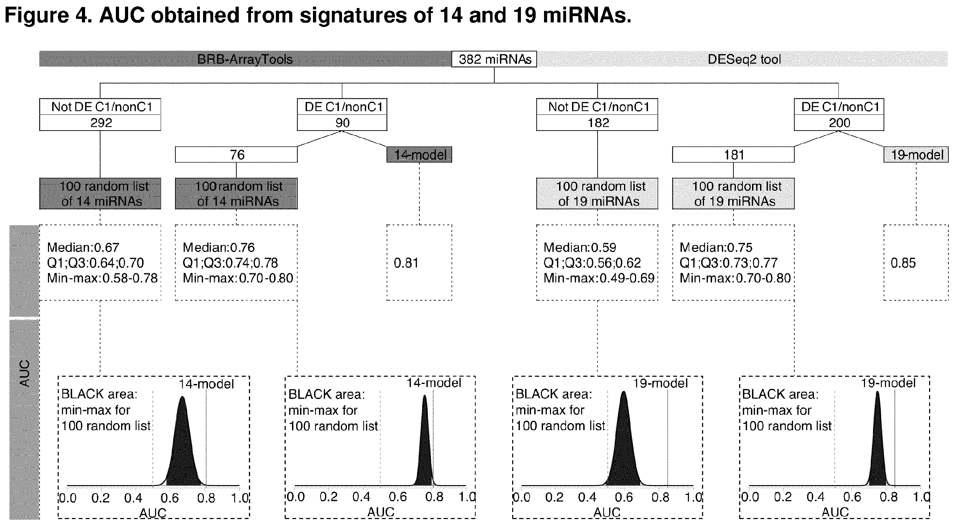

**Figure 4. AUC obtained from signatures of 14 and 19 miRNAs.**

Flow-chart of the design applied to compare the AUC of the 14- and 19-miRNA models to random signatures of equal lengths. For random list of 14 and 19 miRNAs: median, first quartile (Q1), third quartile (Q3), minimum (min) and maximum (max) AUC are reported. Bell distributions of AUC for 100 random signatures; dashed vertical line at AUC = 0.5; solid vertical line at AUC of 14- and 19-miRNA models; filled black area corresponds to min-max AUC range for 100 random signatures. DE stands for differentially expressed.

**Figure 5. Table S1A.**

TCGA-LUAD cohort. 200 miRNAs significantly regulated by DESeq2 in C1 vs. nonC1 patients comparison.

**Figure 6. Table S1B.**

TCGA-LUAD cohort. 90 miRNAs significantly regulated by BRB-ArrayTools in C1 vs. nonC1 patients comparison.

**Definitions**

Unless otherwise defined, all terms of art, notations and other scientific terminology used herein are intended to have the meanings commonly understood by those persons skilled in the art to which this disclosure pertains. In some cases, terms with commonly understood meanings are defined herein for clarity and/or for ready reference; thus, the inclusion of such definitions herein should not be construed to represent a substantial difference over what is generally understood in the art.

The term *"microRNA"* or *"miRNA"* used herein refers to a small non-coding RNA molecule of about 22 nucleotides found in plants, animals and some viruses, that has role in RNA silencing and post-transcriptional regulation of gene expression. miRNA exerts its functions via base-pairing with complementary sequences within mRNA molecules.

The term *"signature"* herein refers to an expression pattern derived from combination of several miRNA (i.e. transcripts) used as biomarkers.

The term *"FFPE specimen"* herein refers to a tissue sample fixed in formalin and embedded in paraffin.

The term *"AUC"* herein refers to the area under the ROC curve (Receiver Operating Characteristic curve), that is a graph showing the performance of a binary classification model at various classification thresholds.

The term patients in group *"C1"* or *"C1-ADC"* used herein include patients affected by aggressive lung adenocarcinoma with experience poor-prognosis patients (i.e. with shorter overall survival, and/or with shorter disease-free survival, and/or responsive to a treatment, and/or with metastatic disease) which can include, but not limited to, patients with early-stage disease (i.e. stage I); while patients included in the group *"nonC1"* or *"nonC1-ADC"* are affected by a non-aggressive lung adenocarcinoma or good-prognosis patients (i.e. with longer overall survival, and/or with longer disease-free survival, and/or responsive to treatment, and/or without metastatic disease) which can include, but not limited to, patients with early-stage disease (i.e. stage I).

The term "aggressive" herein refers to a cancer diagnosed in patients with an adverse prognosis (i.e. with shorter overall survival, and/or with shorter disease-free survival, and/or responsive to a treatment, and/or with metastatic disease).

The term "prognostic" herein refers to the ability to discriminate patients with good/poor prognosis.

The term "biomarkers" (short for biological markers) herein refers to biological indicators (for example a transcript, i.e. miRNA) and/or measures of some biological state or condition.

The terms *"comprising", "having", "including"* and *"containing"* should be understood as 'open' terms (i.e. meaning "including, but not limited to") and should also be deemed a support for terms such as *"consist essentially of', "consisting essentially of', "consist of',* or *"consisting of"*.

The term "TCGA" herein refers to The Cancer Genome Atlas database, where molecular data (e.g. gene and protein expression, gene mutations, methylation profile, copy number variation) for a total of 33 different type of tumors were made available to public (https://www.cancer.gov/about-nci/organization/ccg/research/structural-genomics/tcga).

The term "TCGA-LUAD" herein refers to the specific cohort of lung adenocarcinoma (LUAD) patients which data are available in The Cancer Genome Atlas database.

The term "CSS" herein refers to the cohort of lung adenocarcinoma patients enrolled in IRCSS Casa Sollievo della Sofferenza Hospital.

## Description of the invention

It has been surprisingly found that a 19-miRNA or 14-miRNA signature, or preferably a 7-miRNA signature, is able to identify patients affected by aggressive lung adenocarcinoma including early-stage disease, otherwise stated as "C1-ADC" or "C1". The expression profile and therefore the quantity of such miRNAs can be measured by RNA sequencing (RNA-seq), by quantitative real-time PCR (qRT-PCR) or digital PCR (dPCR), by Affymetrix or direct digital detection (e.g. Nanostring technology), and can be applied to different body fluids or tissue samples, also to FFPE samples, overcoming the drawbacks related to instability/degradation of mRNA mainly in FFPE samples.

Here, the inventors applied a multi-tiered approach relying on RNA-seq (mRNA and miRNA profile) data analysis of a large cohort of lung cancer patients (TCGA-LUAD; N=510), which enabled them to identify prognostic miRNA signatures in lung adenocarcinoma patients. Such signatures showed high accuracy (AUC ranging between 0.79 and 0.85) in scoring aggressive disease and can be used in a molecular multi-biomarkers classifier method. Importantly, using a network-based approach the inventors rewired miRNA-mRNA regulatory networks, identifying a minimal signature of 7 miRNAs, which works also in FFPE samples, and controls a variety of genes overlapping with cancer relevant pathways.

The obtained results further demonstrate the reliability of miRNA-based biomarkers for lung cancer prognostication and their use in a classification method based on the application of miRNA biomarkers in the clinical routine.

More in details, the inventors obtained surrogate miRNA signatures of 7-, 14- and 19-miRNA which recapitulate a previously described 10-gene prognostic signature in ADC including stage I disease [6]. The 7-, 14- and 19-miRNA signatures were all effective to identify aggressive "C1-ADC" disease (AUC=0.79-0.85). Notably, all miRNAs in the 7-miRNA signature were detected in most of the FFPE samples (Ct<40; Table S2) which confirmed the proven higher stability when used in low-quality mRNA [9].

Importantly, in their approach the inventors adopted a network-rewiring strategy by specifically select miRNA-mRNA pairs characterizing aggressive stage I tumors ("C1"). Such approach allowed to select a core of 7 miRNAs capable to stratify C1 from nonC1 tumors with an accuracy comparable to the 14- and 19-miRNA models (Figure 2c and Figure 3a), and, importantly, interacting with the "C1" transcriptome. This is relevant for capturing molecular mechanisms controlled by miRNAs which are associated to lung cancer progression.

As a matter of fact, the inventors observed a large overlap between the '7-miRNA networks' with several gene sets representing cancer relevant pathways (Figure 3c).

The advantages of the new method identified is that of being a reliable prognostic method for the screening of aggressive lung adenocarcinoma including early-stage disease. In particular, said method displays several characteristics that are desirable in a routine clinical setting:

- it can overcome the problem of using low-quality mRNA when extracted for example from FFPE samples, which are routinely used for diagnostic purposes;
- it could distinguish between a fraction of patients with ADC after treatment (i.e. surgery, or surgery and adjuvant chemotherapy, or surgery and radiotherapy, or a combination thereof) that could experience relapse and an adverse prognosis, with subsequent reduction of mortality;
- it can be used with multiple gene expression profiling platforms (i.e. RNAseq, quantitative real-time PCR, digital PCR, Affymetrix, digital detection through molecular barcoding).
- it analyses specific transcript sequences which augment the specificity of the said method.
- it can be implemented in a point-of-care testing (POCT) for miRNA-based diagnostics.
- It can measure quantities of transcripts using small amount of biological material (up to 1-10 nanograms of purified nucleic acids).

It is therefore an embodiment of the present invention a method *in-vitro* or *ex-vivo* for identifying patients affected by aggressive lung adenocarcinoma (C1-ADC), comprising the steps of:

a. detecting the amount of each of the 19 miRNAs having sequence hsa-miR-193b-5p (SEQ ID NO. 3), hsa-miR-31-3p (SEQ ID NO. 11), hsa-miR-31-5p (SEQ ID NO. 12), hsa-miR-550a-5p (SEQ ID NO. 15), hsa-miR-196b-5p (SEQ ID NO. 5), hsa-miR-584-5p (SEQ ID NO. 17), hsa-miR-30d-5p (SEQ ID NO. 10), hsa-miR-582-3p (SEQ ID NO. 16), hsa-miR-9-5p (SEQ ID NO. 19), hsa-let-7c-3p (SEQ ID NO. 1), hsa-miR-138-5p (SEQ ID NO. 2), hsa-miR-196a-5p (SEQ ID NO. 4), hsa-miR-203a-3p (SEQ ID NO. 6), hsa-miR-215-5p (SEQ ID NO. 7), hsa-miR-2355-3p (8SEQ ID NO. 3); hsa-miR-30d-3p (SEQ ID NO. 9), hsa-miR-4709-3p (SEQ ID NO. 13), hsa-miR-548b-3p (SEQ ID NO. 14) and hsa-miR-675-3p (SEQ ID NO. 18); or of each of the 14 miRNA having sequence hsa-miR-196b-5p (SEQ ID NO. 5), hsa-miR-584-5p (SEQ ID NO. 17), hsa-miR-30d-5p (SEQ ID NO. 10), hsa-miR-582-3p (SEQ ID NO. 16), hsa-miR-9-5p (SEQ ID NO. 19), hsa-miR-193b-3p (SEQ ID NO. 23), hsa-miR-135b-5p (SEQ ID NO. 20), hsa-miR-187-3p (SEQ ID NO. 21), hsa-miR-192-5p (SEQ ID NO. 22), hsa-miR-210-3p (SEQ ID NO. 24), hsa-miR-29b-2-5p (SEQ ID NO. 25), hsa-miR-3065-3p (SEQ ID NO. 26), hsa-miR-375-3p (SEQ ID NO. 27) and hsa-miR-708-5p (SEQ ID NO. 28); or of each of the 7 mi-RNA having sequence hsa-miR-31-5p (SEQ ID NO. 12), hsa-miR-31-3p (SEQ ID NO. 11), hsa-miR-193b-3p (SEQ ID NO. 23), hsa-miR-193b-5p (SEQ ID NO. 3), hsa-miR-196b-5p (SEQ ID NO. 5), hsa-miR-550a-5p (SEQ ID NO. 15) and hsa-miR-584-5p (SEQ ID NO. 17) in a biological sample from a subject.

According to a further preferred embodiment, the method of the present invention further comprises step b) wherein the data obtained in step a) is normalized.

According to a further preferred embodiment, the method of the present invention further comprises step c) wherein the patients are classified either in the class of subjects affected by aggressive lung adenocarcinoma, or in the class of subjects affected by non-aggressive lung adenocarcinoma (nonC1-ADC).

Preferably, the class of subjects affected by aggressive lung adenocarcinoma comprises patients at an early-stage disease (stage I).

More preferably, the lung adenocarcinoma is a non-small cell lung adenocarcinoma (NSCLC).

According to the present invention, the detection of said miRNA is performed by means on hybridization with primers and/or probes, each one selective for the sequence of one miRNA.

According to a preferred embodiment of the present invention, the quantity of said miRNAs in step a) is calculated by quantitative RT-PCR (qRT-PCR), digital PCR, RNA sequencing, Affymetrix microarray, custom microarray or digital detection through molecular barcoding, selected from NanoString technology.

Preferably, the quantitative RT-PCR (qRT-PCR) of step a) is performed by using specific primers and/or probers for each of miRNA to be detected.

Preferably, said specific primers and probes are designed in order to retro-transcribe (i.e. RT reaction) and then amplify (i.e. qPCR) each miRNA present in the 19-miRNA, 14-miRNA o 7-miRNA signature. The RT reaction can be based on 3' poly-A tailing and 5' ligation of an adaptor sequence to extend the mature miRNAs present in the sample [16], or by using a miRNA specific stem-loop primers [17].

Preferably, miRNA quantities are measured as total RNA, comprising mRNA and miRNA, extracted using conventional RNA extraction methods, selected from AllPrep DNA/RNA FFPE kit (QIAGEN) or other RNA extraction methods from FFPE blocks and RNA extractions methods from other body fluid samples or tissue samples.

Preferably, the RNA sequencing is performed using 10ng of total RNA, from which miRNAs are selected according to size. MiRNA sequencing libraries are constructed ligating miRNAs with specific sequencing adapters and converting them into cDNA. Sequencing by synthesis, using preferably Illumina sequencing platform, is then applied to the miRNA library preparation.

Preferably, qRT-PCR analysis is performed using 10 ng of total RNA which is reverse-transcribed using 3' poly-A tailing and 5' ligation of an adaptor sequence, or using specific stem-loop primers, followed by qRT-PCR analysis using miRNA specific primers and probes selected for any miRNA signature analyzed according to the present invention.

More preferable, the qRT-PCR analysis is performed by using the TaqMan Advanced miRNA cDNA Synthesis Kit (ThermoFisher) and TaqMan Advanced miRNA Assays or with an analogue method of quantitative RT-PCR, by using the specific primers and probes selected for any miRNA signature analyzed according to the present invention

Preferably, Poly(A) tailing, adapter ligation, RT reaction and miR-Amp are performed following instructions of TaqMan Advanced miRNA Assay (ThermoFisher) or of an analogue method.

Preferably, the hsa-miR16-5p (MIMAT0000069; SEQ ID N: 29 UAGCAGCACGUAAAUAUUGGCG) is used as standard reference in the qRT-PCR reaction.

According to the present invention, the normalization of the data reported in step b) is made according to the scheme reported in below, when the miRNAs are quantified by RNA sequencing:

| | RNA sequencing | | |
|---|---|---|---|
| | For 14-miRNAs signature | For 19-miRNAs signature | For 7-miRNA signature |
| STEP b- raw data normalization | For miRNA $i$ in sample $j$: Normalized count$_{ij}$=raw count$_{ij}$/SizeFactor$_j$ Where: SizeFactor$_j$=median$_j$ | For miRNA $i$ in sample $j$: Normalized count$_{ij}$=raw count$_{ij}$/SizeFactor$_j$ Where: SizeFactor$_j$=median$_j$ | For miRNA $i$ in sample $j$: Normalized count$_{ij}$=raw count$_{ij}$/SizeFactor$_j$ Where: SizeFactor$_j$=median$_j$ |
| | (across all RatioFactor$_{ij}$), RatioFactor$_{ij}$=raw count$_{ij}$/geomean$_i$ (across all samples$_{ij}$) | (across all RatioFactor$_{ij}$), RatioFactor$_{ij}$=raw count$_{ij}$/geomean$_i$ (across all samples$_{ij}$) | (across all RatioFactor$_{ij}$), RatioFactor$_{ij}$=raw count$_{ij}$/geomean$_i$ (across all samples$_{ij}$) |

wherein

- the normalized count$_{ij}$ of each miRNA ($i$) of each sample ($j$) is calculated as ratio of the raw count$_{ij}$ and a size factor$_j$ specific for each sample$_j$; the size factor$_j$ specific for each sample is calculated as the median of all ratio factors$_{ij}$ of that sample; ratio factors$_{ij}$ represents the ratio between raw count$_{ij}$ and the geometric mean$_j$ of all raw count$_{ij}$ relative to a specific miRNA $_I$,

According to the present invention, the normalization of the data reported in step b) is made according to the scheme reported in below, when the miRNAs are quantified by RT-PCR:

| | qRT-PCR | | |
|---|---|---|---|
| | For 14-miRNAs signature | For 19-miRNAs signature | For 7-miRNAs signature |
| *STEP b- raw data normalization* | For miRNA $i$ in sample $j$: Normalized Ct$_{ij}$=raw Ct$_{ij}$-SF$_j$ where SF$_j$=hsa-miR-16-5p$_j$- 21.87 | For miRNA $i$ in sample $j$: Normalized Ct$_{ij}$=raw Ct$_{ij}$-SF$_j$ where SF$_j$ =hsa-miR-16-5p$_j$- 21.87 | For miRNA $i$ in sample $j$: Normalized Ct$_{ij}$=raw Ct$_{ij}$-SF$_j$ where SF$_j$=hsa-miR-16-5p$_j$- 21.87 |

wherein:

- the normalized Ct$_{ij}$ (cycle threshold) of each miRNA ($i$) of each sample ($j$) is calculated as difference between the raw Ct$_{ij}$ and a scaling factor$_j$ (SF) specific for each sample$_j$; the scaling factor represents the difference between the raw Ct of the miRNA "hsa-miR-16-5p" used as a reference in the sample and a constant equal to 21.87.

According to the present invention, the normalization of the raw data reported in step b) when the miRNAs are quantified by Affymetrix microarray is made according to Gene Chip miRNA Arrays, where Affymetrix oligonucleotide microarrays are used to interrogate the expression of all mature miRNA sequences in last miRBase Release. According to the present invention, the normalization of the raw data reported in step b) when the miRNAs are quantified by direct digital quantification (e.g. Nanostring, nCounter analysis) is made according to digital color-coded barcode technology that is based on direct multiplexed measurement of miRNA expression.

According to the method of the present invention, the classification of the predicted class of subjects affected by aggressive lung adenocarcinoma or of subjects affected by non-aggressive lung adenocarcinoma of step c) is calculated by the following formula:

$$\text{predicted class} = \begin{cases} \text{C1}, & \dfrac{1}{1 + e^{-z}} \geq 0.5 \\ \text{nonC1}, & \dfrac{1}{1 + e^{-z}} < 0.5 \end{cases}$$

wherein

- in a model of 19-miRNAs analyzed by RNA sequencing:

z = -8.2029+(-0.2651*hsa-let-7c-3p)+(0.1709*hsa-miR-138-5p)+(0.1443*hsa-miR-193b-5p)+(0.0200*hsa-miR-196a-5p)+(0.0464*hsa-miR-196b-5p)+(0.2297*hsa-miR-203a-3p)+(0.1285*hsa-miR-215-5p)+(0.3933*hsa-miR-2355-3p)+(-0.2220*hsa-miR-30d-3p)+(-0.1874*hsa-miR-30d-5p)+(0.1535*hsa-miR-31-3p)+(0.0326*hsa-miR-31-5p)+(-0.3032*hsa-miR-4709-3p)+(-0.1672*hsa-miR-548b-3p)+(0.1529*hsa-miR-550a-5p)+(0.1132*hsa-miR-582-3p)+(0.5229*hsa-miR-584-5p)+(0.1314*hsa-miR-675-3p)+(0.0497*hsa-miR-9-5p);

- in a model of 14-miRNAs analyzed by RNA sequencing:

z = -5.4414+(-0.1028*hsa-miR-135b-5p)+(-0.0486*hsa-miR-187-3p)+(0.2828*hsa-miR-192-5p)+(0.1977*hsa-miR-193b-3p)+(0.0201*hsa-miR-196b-5p)+(0.1908*hsa-miR-210-3p)+(-0.5074*hsa-miR-29b-2-5p)+(0.0384*hsa-miR-3065-3p)+(-0.3312*hsa-miR-30d-5p)+(-0.0475*hsa-miR-375-3p)+(0.1895*hsa-miR-582-3p)+(0.5606*hsa-miR-584-5p)+(0.2663*hsa-miR-708-5p)+(0.0435*hsa-miR-9-5p); and

- in a model 7-miRNAs analyzed by RNA sequencing:

z = -8.5210+(0.1171*hsa-miR-193b-3p)+(0.2233*hsa-miR-193b-5p)+(0.1341*hsa-miR-196b-5p)+(0.1554*hsa-miR-31-3p)+(0.0584*hsa-miR-31-5p)+(0.3622*hsa-miR-550a-5p)+(0.4683*hsa-miR-584-5p);

or in alternative,
- in a model of 7-miRNAs analyzed by qRT-PCR :

z= 2.2920 +(hsa-miR-193b-3p*0.1295)+(hsa-miR-193b-5p*0.0920)+(hsa-miR-196b-5p*-0.1310)+(hsa-miR-31-3p*-0.2116)+(hsa-miR-31-5p*-0.2724)+(hsa-miR-550a-5p*0.2717)+(hsa-miR-584-5p*0.0413).

According to a preferred embodiment, step c) classify the patients in the predicted class of subjects affected by aggressive lung adenocarcinoma which can include, but not limited to, early-stage disease (stage I).
According to a preferred embodiment, the method of the present invention identifies patients included in the group of aggressive lung adenocarcinoma with a poor-prognosis, selected from patients with shorter overall survival and and/or patients with shorter disease-free survival, and/or patients responsive to a treatment, and/or with patients with metastatic disease which can include, but not limited to, patients with early-stage disease (stage I).
According to a further preferred embodiment, the method of the present invention identifies patients included in the group of non aggressive lung adenocarcinoma with a good-prognosis, selected from patients with longer overall survival, and/or patients with longer disease-free survival, and/or patients responsive to treatment, patients and/or without metastatic disease which can include, but not limited to, patients with early-stage disease (stage I).
According to a further preferred embodiment, the method of the present invention is used for the prognostic risk stratification of patients with lung adenocarcinoma and/or to identify alternative therapeutic options after surgery, selected from systemic adjuvant chemotherapy, selected from platinum-based combinations, preferably cisplatin, carboplatin plus a third generation agents such as gemcitabine, vinorelbine, a taxane or camptothecin, molecular targeted therapeutics, immunotherapeutic, radiotherapy, or a combination thereof.
Preferably, in the method of the present invention the biological sample is a tissue sample or a body fluid.
More preferably said tissue sample is a fresh tissue sample, a frozen tissue sample or a FFPE tissue sample.
More preferably said body fluid is serum or plasma.
A further embodiment is a microarray, a quantitative polymerase chain reaction, a sequencing-based technology or a digital molecular barcoding-based technology, to perform the method according to the present invention.
Preferably the primers and probes used in the method of the present invention to amplify each of miRNAs to be detected correspond to the primers and probes used in the assays listed in Table 12.

**Results**

*1. miRNA-signature identification*

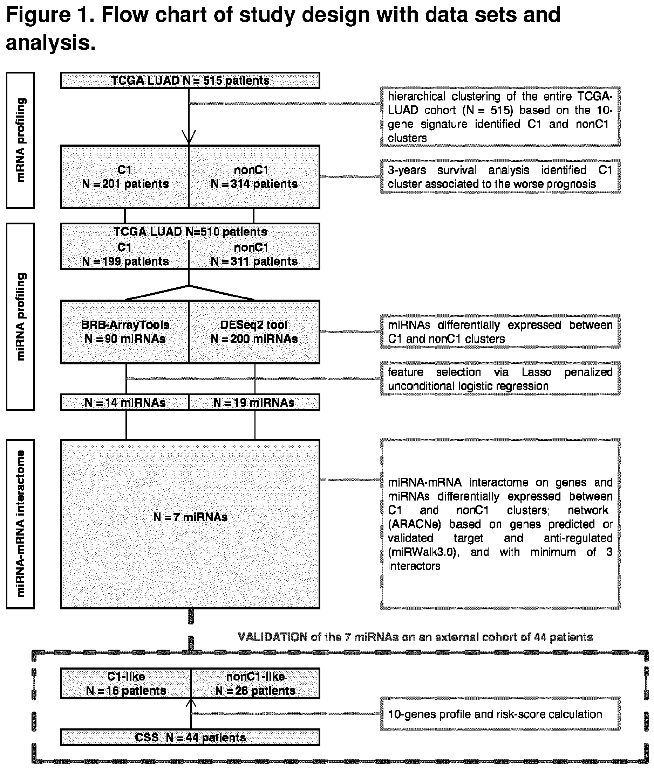

We developed a multi-tiered approach summarized in Figure 1, which allowed us to identify a surrogate miRNA-based signature for prognostication of ADC patients.

**Table 1. Patients and tumors characteristics.**

| | TCGA-LUAD cohort N=515 | CSS cohort N=44 |
|---|---|---|
| **Age [years]** | | |
| Median (first quartile;third quartile) | 66 (59;73) [1] | 73 (67;77) |
| **Gender** | | |
| Male | 238 (46.2%) | 27 (61.4%) |
| Female | 277 (53.8%) | 17 (38.6%) |
| **Smoking status** | | |
| Current/former smoker | 367 (71.3%) | 20 (45.5%) |
| Never smoker | 63 (12.2%) | 11 (25.0%) |
| Missing smoking status | 85 (16.5%) | 13 (29.5%) |
| **Stage** | | |
| Stage I | 279 (54.2%) | 31 (70.5%) [2] |
| Stage II-IV | 235 (45.6%) | 13 (29.5%) |
| Missing stage | 1 (0.2) | - |
| **Follow-up** [3] | | |
| Survivors length of follow-up | | |
| <1 yr | 52 (10.3%) | 13 (31.7%) |
| 1-2 yrs | 128 (25.3%) | 11 (26.8%) |
| 2-3 yrs | 56 (11.1%) | 10 (24.4%) |
| >3 yrs | 133 (26.3%) | 5 (12.2%) |
| Deaths within 3 years | 137 (27.1%) | 2 (4.9%) [4] |

Percentages could not add up to 100 due to rounding; [1] 19 patients with missing information on age; [2] 1 patient with adenocarcinoma *in situ;* [3] 9 patients with missing follow-up in the TCGA-LUAD cohort; [4] 3 deaths were excluded: 1 without date of dead, and 2 within 30 days from surgery.

Hierarchical clustering analysis using the 10-gene signature of the TCGA-LUAD cohort (N=515) patients revealed 4 main branches, namely C1 (N=201), C2 (N=98), C3 (N=39), and C4 (N=177) clusters (Figure 2a) that are consistent with previous findings [6]. Analysis of the 3-years overall survival showed non-significant differences between C2, C3 and C4 clusters (log-rank test p-value=0.90 and p=0.48 in stage I and advanced stages, respectively), that were therefore collapsed into nonC1 clusters. C1 patients displayed the worse prognosis both in stage I (p-value=0.0010) and in more advanced stages (p=0.0061) (Figure 2b).

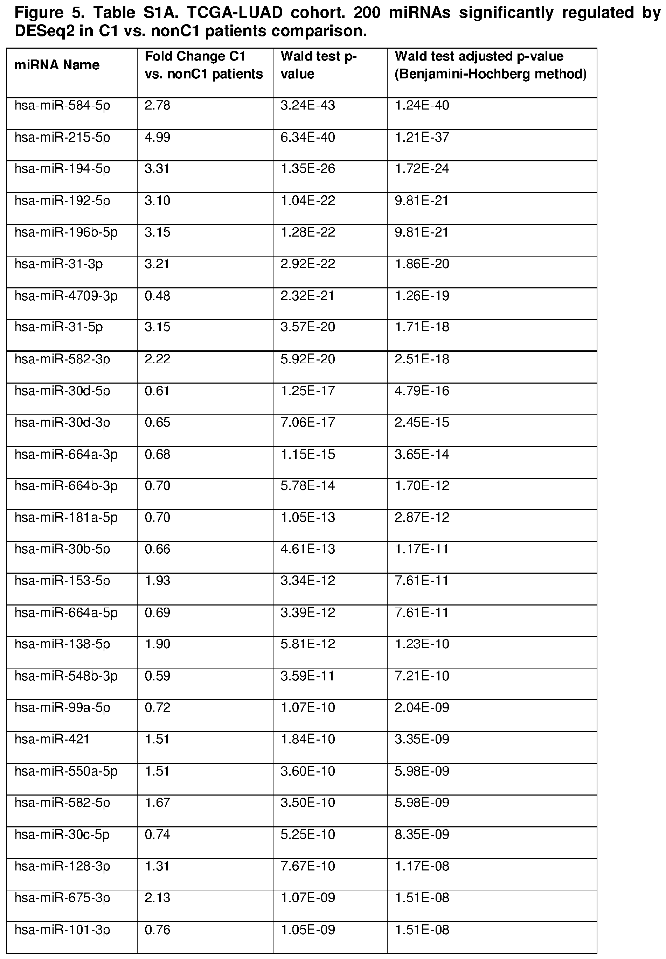

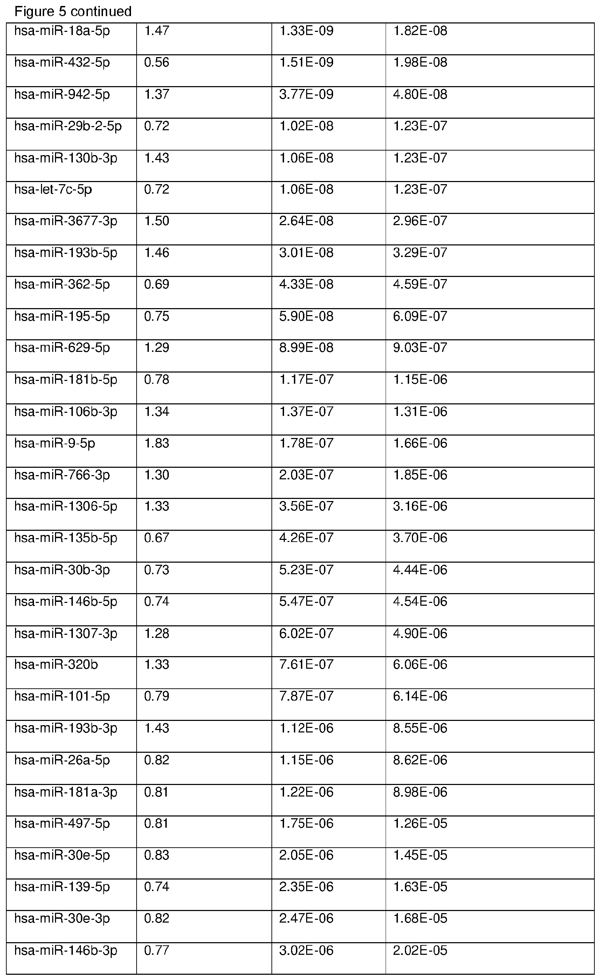

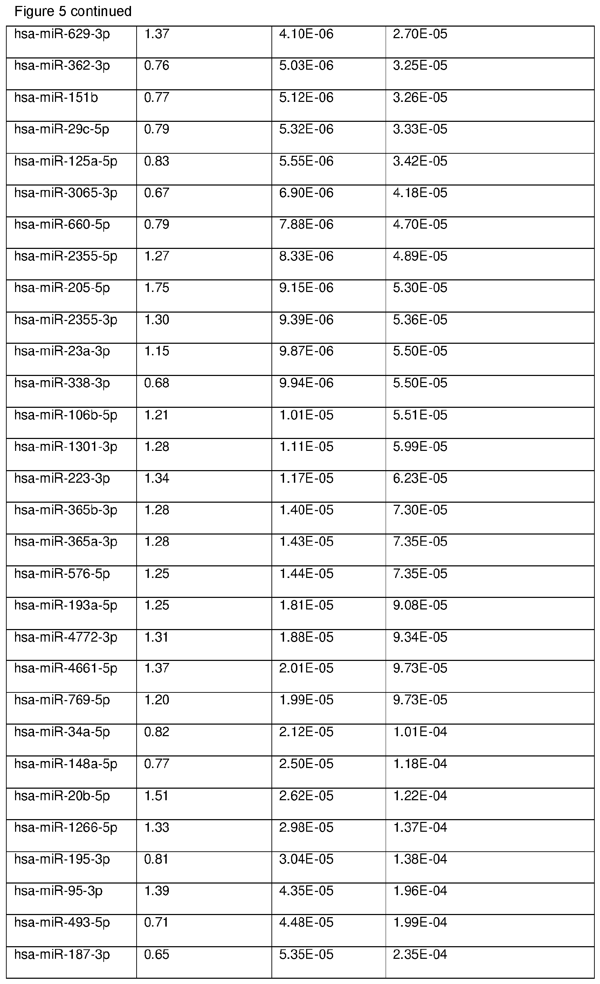

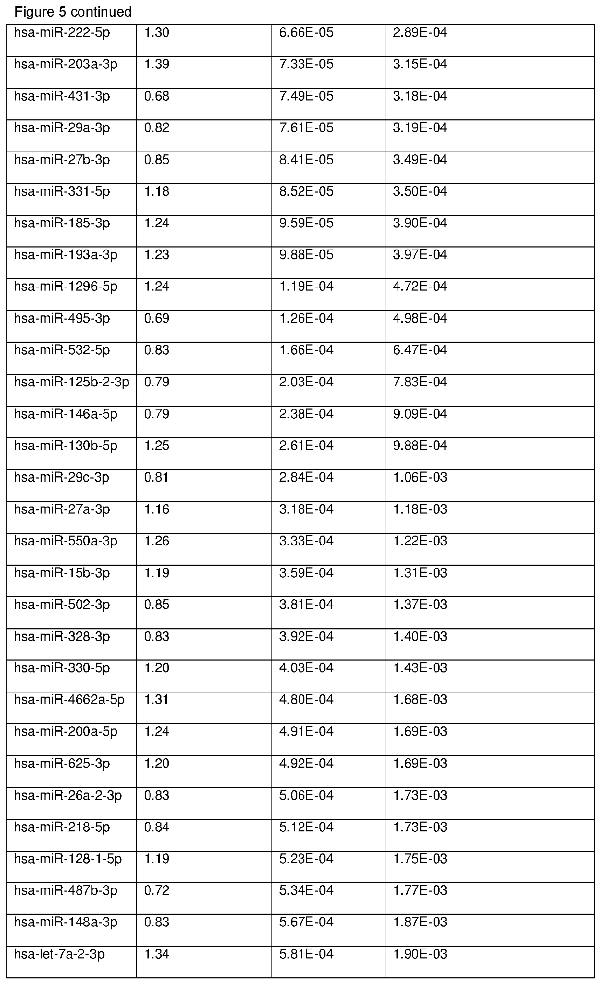

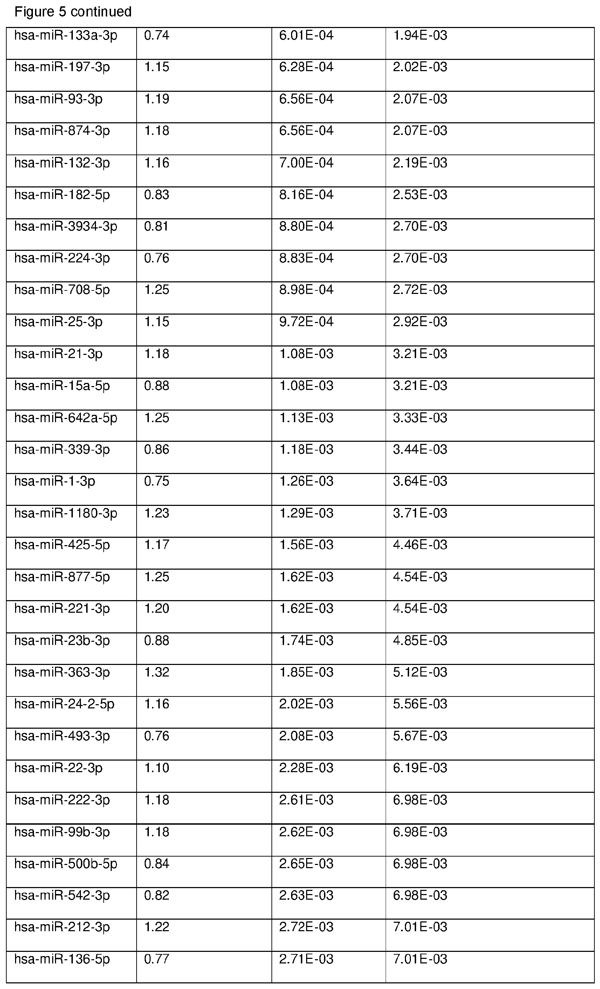

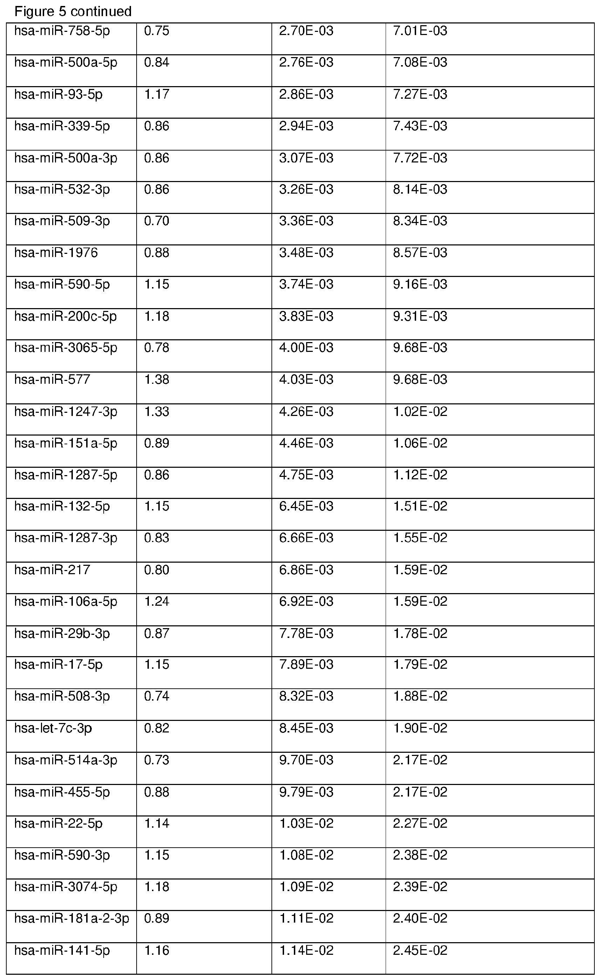

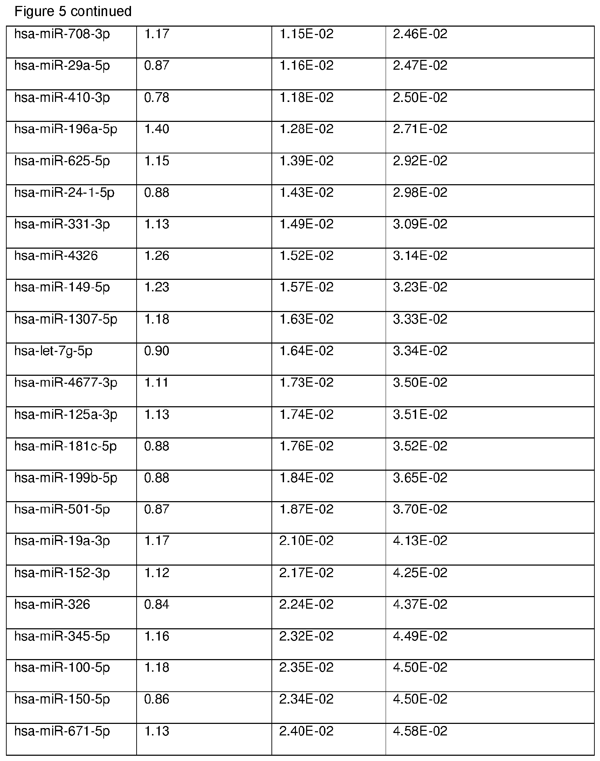

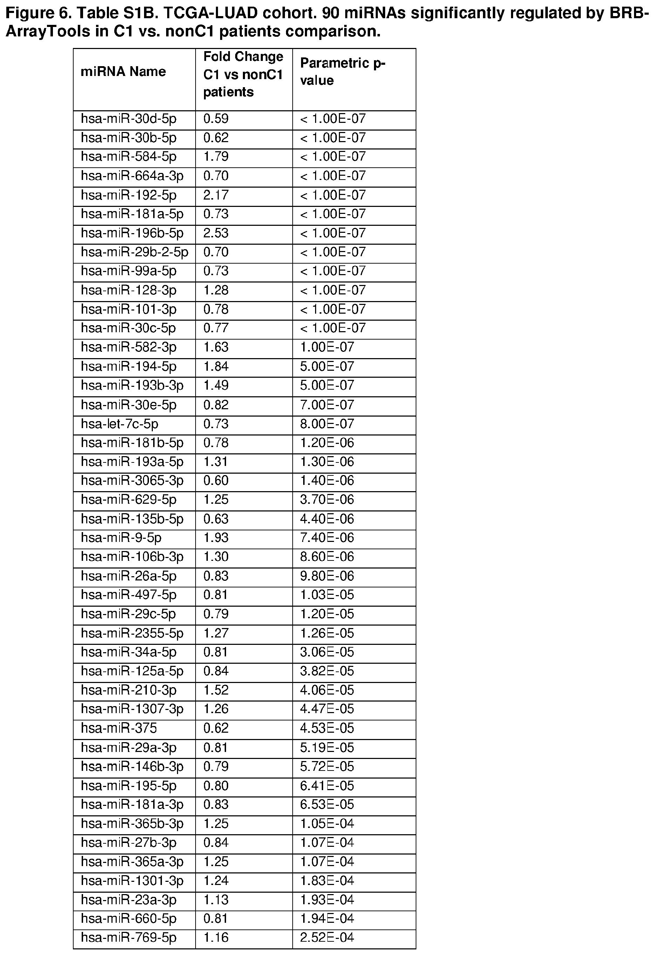

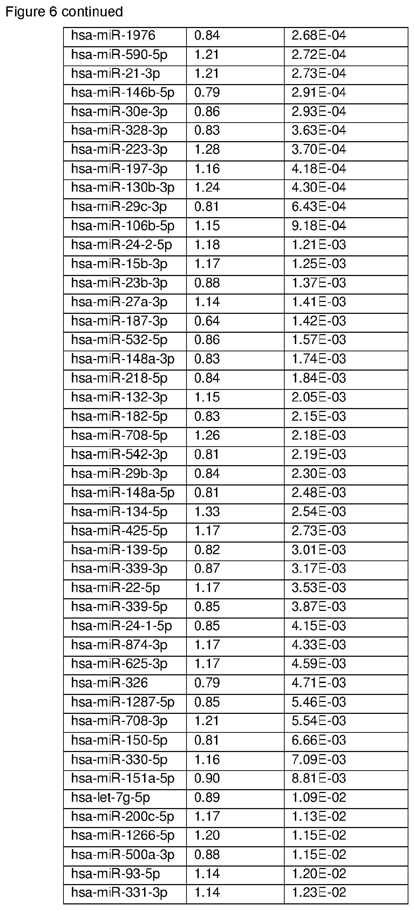

We then performed miRNA expression profile of 510 out of the 515 ADC of the TCGA-LUAD cohort, with miRNAs expression data available. We used both DESeq2 R package and BRB-ArrayTools (see methods) as alternative statistical approaches in order to identify differentially expressed miRNAs in C1 and nonC1 patients. We analyzed a total of 382 miRNAs of which 200 were found differentially expressed by DESeq2 and 90 by BRB-ArrayTools (Table S1A and Table S1B, respectively, see Figures 5 and 6).

A total of 87 miRNAs were overlapping in the two sets. Lasso regularization was then applied to identify optimized miRNA-based signatures capable of stratifying C1 from nonC1 tumors. Two signatures of 14-miRNA (from the 90 miRNA set) and 19-miRNA (from the 200 miRNA set) were derived (5 miRNA overlapping; Table 2), which displayed an high accuracy in C1/nonC1 cancer patients stratification (cross-validated AUC=0.81 and AUC=0.85, respectively; Figure 2c).

To further reduce complexity of these miRNA-based biomarkers, we looked for a minimal set of miRNAs capable of the same accuracy of the 14- and 19-miRNA signatures to identify C1 aggressive disease.

The following assumptions were made: i) the molecular function of a miRNA is dependent to the network of targeted mRNAs which, in this case, are those differentially expressed in C1/nonC1 tumors; ii) a prognostic biomarkers should be functionally linked to mechanisms involved in tumor progression. Accordingly, we explored the miRNA-mRNA interactome characterizing C1 tumors by performing ARACNe (Algorithm for the Reconstruction of Accurate Cellular Networks)(see methods) using the set of 200 miRNA, and a set of 2900 mRNA genes found significantly regulated in C1-ADC (p<0.05) by DESeq2 (see methods). Our analysis was restricted to genes identified by DESeq2 in order to reduce technical variability. The following rules were applied to rewire C1 miRNA-mRNA interactome: 1) we selected miRNA-mRNA pairs generated in only C1 tumors and specific, but not exclusive, for stage I (N=2858); 2) we selected miRNA predicted to target C1-genes (N=1787), and 3) with an opposite trend of expression than C1-genes (N=598); 4) we selected miRNA interacting with a least three C1-genes (N=528).

Among the miRNA-mRNA networks identified, we found a set of interacting networks with 7 miRNA as "HUBs" which derived from both the 19-miRNA and 14-miRNA signatures (Table 2 and Figure 2d). Hierarchical clustering analysis of this 7-miRNA signature (Table S2) showed an overall increased expression in the more aggressive C1 tumors (Figure 2e). Importantly, the 7-miRNA signature had a cross-validated AUC of 0.79 in C1/nonC1 patients stratification, which is comparable to the other two signatures (Figure 3a) as well as when we considered differences in C1 predicted probability (Figure 3b). The predicted C1 class from all the 3 signatures (7-, 14- and 19-miRNA) presented significantly increased hazard of death at 3 years in patients of all stages, with an increased risk comparable to the C1 patients identified by the 10 genes (Table 3). However, when we focused the analysis to stage I ADC patients, we scored that the best risk-stratification was held by the 7-miRNA signature with approximately 2-fold increased risk of death for C1 patients (HR=2.11; 95% Confidence Interval: 1.11-4.00; p=0.0223) (Table 3). Interestingly enough, the networks of genes targeted by these 7 miRNAs were found significantly (FDR q-value<0.0001) enriched in gene sets representing molecular mechanisms related to cancer progression, which fulfilled our initial hypotheses (Figure 3c).

Despite most of 90 miRNAs identified by BRB-ArrayTools (87/90, 97%) were comprised in the 200-miRNA set found by DESeq2, including 12 out of 14 miRNAs of the BRB-derived model, we performed ARACNe as well by using this 90-miRNAs set. Among the three not overlapping miRNAs, only hsa-miR-210-3p passed all the selection filters we described previously. However, when we added this additional miRNA to the 7-miRNA signature and perfomed cross-validation in C1/nonC1 patients stratification, the prediction performance remained the same (i.e. AUC=0.79).

**Table 2. TCGA-LUAD cohort. Differentially expressed miRNAs composing the 3 signatures of 19, 14 and 7 miRNAs.**

| miRNA | Signature | TCGA-LUAD cohort-C1 vs nonC1 patients | | |
|---|---|---|---|---|
| | | Fold Change | Wald test adjusted p-value [1] | C1 regulation |
| hsa-miR-193b-5p | 19- and 7-miRNA | 1.5 | 3.3E-07 | ↑ |
| hsa-miR-31-3p | 19- and 7-miRNA | 3.2 | 1.9E-20 | ↑ |
| hsa-miR-31-5p | 19- and 7-miRNA | 3.1 | 1.7E-18 | ↑ |
| hsa-miR-550a-5p | 19- and 7-miRNA | 1.5 | 6.0E-09 | ↑ |
| hsa-miR-196b-5p | 19-, 14-miRNA and 7-miRNA | 3.2 | 9.8E-21 | ↑ |
| hsa-miR-584-5p | 19-, 14-miRNA and 7-miRNA | 2.8 | 1.2E-40 | ↑ |
| hsa-miR-30d-5p | 19- and 14-miRNA | 0.6 | 4.8E-16 | ↓ |
| hsa-miR-582-3p | 19- and 14-miRNA | 2.2 | 2.5E-18 | ↑ |
| hsa-miR-9-5p | 19 and 14-miRNA | 1.8 | 1.7E-06 | ↑ |
| hsa-let-7c-3p | 19-miRNA | 0.8 | 1.9E-02 | ↓ |
| hsa-miR-138-5p | 19-miRNA | 1.9 | 1.2E-10 | ↑ |
| hsa-miR-196a-5p | 19-miRNA | 1.4 | 2.7E-02 | ↑ |
| hsa-miR-203a-3p | 19-miRNA | 1.4 | 3.1E-04 | ↑ |
| hsa-miR-215-5p | 19-miRNA | 5.0 | 1.2E-37 | ↑ |
| hsa-miR-2355-3p | 19-miRNA | 1.3 | 5.4E-05 | ↑ |
| hsa-miR-30d-3p | 19-miRNA | 0.6 | 2.5E-15 | ↓ |
| hsa-miR-4709-3p | 19-miRNA | 0.5 | 1.3E-19 | ↓ |
| hsa-miR-548b-3p | 19-miRNA | 0.6 | 7.2E-10 | ↓ |
| hsa-miR-675-3p | 19-miRNA | 2.1 | 1.5E-08 | ↑ |

(continued)

| miRNA | Signature | TCGA-LUAD cohort-C1 vs nonC1 patients | | |
|---|---|---|---|---|
| | | Fold Change | Wald test adjusted p-value [1] | C1 regulation |
| hsa-miR-193b-3p | 14- and 7-miRNA | 1.4 | 8.6E-06 | ↑ |
| hsa-miR-135b-5p | 14-miRNA | 0.7 | 3.7E-06 | ↓ |
| hsa-miR-187-3p | 14-miRNA | 0.6 | 2.3E-04 | ↓ |
| hsa-miR-192-5p | 14-miRNA | 3.1 | 9.8E-21 | ↑ |
| hsa-miR-210-3p | 14-miRNA | 1.2 | 6.4E-02 | ↑ |
| hsa-miR-29b-2-5p | 14-miRNA | 0.7 | 1.2E-07 | ↓ |
| hsa-miR-3065-3p | 14-miRNA | 0.7 | 4.2E-05 | ↓ |
| hsa-miR-375-3p | 14-miRNA | 1.2 | 1.7E-01 | ↑ |
| hsa-miR-708-5p | 14-miRNA | 1.3 | 2.7E-03 | ↑ |
| [1] Benjamini-Hochberg method from DESeq2 tool | | | | |

**Table S2. Distributions of expression for the 7 miRNAs in TCGA-LUAD and CSS cohorts.**

| | TCGA-LUAD N=510 | | | CSS cohort N=44 | | |
|---|---|---|---|---|---|---|
| | normalized counts (log2) | | | normalized CT | | |
| miRNA | Median | Q1 | Q3 | Median | Q1 | Q3 |
| hsa-miR-193b-3p | 7.70 | 6.90 | 8.62 | 23.58 | 21.94 | 24.38 |
| hsa-miR-193b-5p | 4.27 | 3.55 | 5.00 | 30.44 | 24.59 | 36.55 |
| hsa-miR-196b-5p | 6.42 | 5.14 | 8.67 | 29.10 | 27.96 | 30.19 |
| hsa-miR-31-3p | 2.94 | 1.53 | 4.95 | 26.40 | 25.14 | 27.53 |
| hsa-miR-31-5p | 3.62 | 1.87 | 5.75 | 26.88 | 24.21 | 28.15 |
| hsa-miR-550a-5p | 4.01 | 3.33 | 4.67 | 24.86 | 23.89 | 25.83 |
| hsa-miR-584-5p | 6.46 | 5.87 | 7.29 | 23.02 | 21.64 | 24.72 |
| Q1, first quartile of distribution; Q3, third quartile of distribution | | | | | | |

**Table 3. TCGA-LUAD cohort. Univariate and multivariable Cox regression analyses for 3-years overall survival in patients of all stages and stratified by stage. Hazard Ratio (HR) and 95% Confidence Interval (95% CI) are reported.**

| | N (Number of deaths) | Univariate analysis | | Multivariable analysis [1] | |
|---|---|---|---|---|---|
| | | HR (95% CI) | Wald test p-value | HR (95% CI) | Wald test p-value |
| ALL STAGES | 501 (135) [2] | | | | |
| 10-gene | 194 (75) | 2.21 (1.57-3.10) | <0.0001 | 2.03 (1.43-2.87) | <0.0001 |
| 19-miRNA | 169 (66) | 2.13 (1.52-2.99) | <0.0001 | 1.85 (1.31-2.61) | 0.0005 |
| 14-miRNA | 165 (67) | 2.17 (1.55-3.04) | <0.0001 | 2.06 (1.46-2.91) | <0.0001 |
| 7-miRNA | 146 (67) | 2.90 (2.07-4.06) | <0.0001 | 2.69 (1.91-3.78) | <0.0001 |
| STAGE I | 274 (40) | | | | |
| 10-gene | 92 (23) | 2.86 (1.53-5.36) | 0.0010 | 2.96 (1.55-5.65) | 0.0010 |
| 19-miRNA | 73 (11) | 1.07 (0.54-2.15) | 0.8462 | 1.12 (0.55-2.26) | 0.7529 |
| 14-miRNA | 79 (17) | 1.90 (1.01-3.56) | 0.0451 | 1.99 (1.05-3.79) | 0.0359 |

(continued)

| | N (Number of deaths) | Univariate analysis | | Multivariable analysis [1] | |
|---|---|---|---|---|---|
| | | HR (95% CI) | Wald test p-value | HR (95% CI) | Wald test p-value |
| ALL STAGES | 501 (135) [2] | | | | |
| 7-miRNA | 65 (15) | 2.11 (1.11-4.00) | 0.0223 | 2.14 (1.11-4.12) | 0.0235 |
| STAGE II-IV | 226 (95) | | | | |
| 10-gene | 101 (52) | 1.69 (1.13-2.54) | 0.0108 | 1.64 (1.08-2.49) | 0.0207 |
| 19-miRNA | 95 (55) | 2.27 (1.51-3.41) | <0.0001 | 2.18 (1.43-3.31) | 0.0003 |
| 14-miRNA | 86 (50) | 2.00 (1.34-3.00) | 0.0007 | 2.04 (1.35-3.08) | 0.0007 |
| 7-miRNA | 80 (52) | 2.89 (1.93-4.33) | <0.0001 | 2.91 (1.93-4.39) | <0.0001 |
| [1] all stages analyses were adjusted for age, sex, smoking status and stage; analyses stratified by stage were adjusted for age, sex and smoking status; [2] 1 patient with missing stage and 9 patients with missing follow-up. | | | | | |

*2. Seven-miRNA-signature validation*

Finally, we performed a validation of the 7 miRNA-signature in an external cohort of 44 lung adenocarcinoma patients, which was collected at the IRCCS Casa Sollievo della Sofferenza Hospital (CSS). Table 1 shows patients and tumors characteristics of CSS cohort, highlighting an overrepresentation of stage I tumors in CSS (70%) with respect to the TCGA-LUAD cohort (54%). We performed qRT-PCR analysis of FFPE samples using the 10-gene signature and calculated relative risk-score to stratify the cohort into a C1 (N=16) and nonC1 (N=28) groups (Figure 3d) (see methods). Next, we performed qRT-PCR analysis using TaqMan Advanced miRNA Assay to profile also the 7-miRNA signature in the same cohort of 44 ADC and, using logistic regression, we rederived a model based on the expression profile of the 7-miRNA signature (Table S2). The 7-miRNA model stratified C1 from nonC1 tumors with an AUC of 0.76 (Figure 3e) and with significant difference (p=0.0028) in C1 predicted probability (Figure 3f). Remarkably, when we limited the analysis to stage I tumors, we scored an AUC of 0.81 (Figure 3e) and a significant difference (p=0.0108) in C1 predicted probability (Figure 3f). Tables 4-7 reported below presents the details of the miRNAs signatures identified, their accession number (mirbase.org), their sequences, the regulation trends (C1 versus nonC1) and the model weight used.

**Table 4. The 19-miRNA signature (detected by RNA sequencing).**

Assay refers to the code used to identify each miRNA in the TCGA-LUAD RNA sequencing experiment; Acc. mature-miRNA refers to the miRBase accession number of the mature miRNA; Sequence refers to the nucleotide sequence of the mature miRNA; miRbase ID refers to the name of the mature miRNA in the miRbase database; Ratio refers to the fold change calculated dividing the median expression of each miRNA in C1-ADC samples by the median expression in nonC1-ADC samples; p-values were calculated from Wald test of DESeq2 comparing C1-ADC vs. nonC1-ADC samples. All miRNAs have q-value < 0.05 based on Benjamini-Hochberg adjustment; Model weight refers to the coefficient in the equation used to calculate the predicted class.

**Table 5. The 14-miRNA signature (detected by RNA sequencing).**

| The 19-miRNA signature (RNA-seq) | | | | | | |
|---|---|---|---|---|---|---|
| Assay | Acc. mature-miRNA | Sequence (SEQ ID N.) | miRbase ID | Ratio | P-value | Model weight |
| hsa-let-7c-3p | MIMAT0026472 | CUGUACAACCUUCUAGCUUUCC (SEQ ID NO. 1) | hsa-let-7c-3p | 0.82 | 8.45E-03 | -0.2651 |
| hsa-mi-R-138-5p | MIMAT0000430 | AGCUGGUGUUGUGAAUCAGGCCG (SEQ ID NO. 2) | hsa-mi-R-138-5p | 1.76 | 5.81E-12 | 0.1709 |
| hsa-miR-193-b-5p | MIMAT0004767 | CGGGGUUUUGAGGGCGAGAUGA (SEQ ID NO. 3) | hsa-miR-193-b-5p | 1.44 | 3.01E-08 | 0.1443 |

(continued)

| The 19-miRNA signature (RNA-seq) | | | | | | |
|---|---|---|---|---|---|---|
| Assay | Acc. mature-miRNA | Sequence (SEQ ID N.) | miRbase ID | Ratio | P-value | Model weight |
| hsa-miR-196-a-5p | MIMAT0000226 | UAGGUAGUUUCAUGUUGUUGGG (SEQ ID NO. 4) | hsa-miR-196-a-5p | 2.82 | 1.28E-02 | 0.0200 |
| hsa-miR-196-b-5p | MIMAT0001080 | UAGGUAGUUUCCUGUUGUUGGG (SEQ ID NO. 5) | hsa-miR-196-b-5p | 3.15 | 1.28E-22 | 0.0464 |
| hsa-miR-203-a-3p | MIMAT0000264 | GUGAAAUGUUUAGGACCACUAG (SEQ ID NO. 6) | hsa-miR-203-a-3p | 1.48 | 7.33E-05 | 0.2297 |
| hsa-mi-R-215-5p | MIMAT0000272 | AUGACCUAUGAAUUGACAGAC (SEQ ID NO. 7) | hsa-mi-R-215-5p | 1.57 | 6.34E-40 | 0.1285 |
| hsa-mi-R-2355--3p | MIMAT0017950 | AUUGUCCUUGCUGUUUGGAGAU (SEQ ID NO. 8) | hsa-mi-R-2355-3-p | 1.34 | 9.39E-06 | 0.3933 |
| hsa-miR-30-d-3p | MIMAT0004551 | CUUUCAGUCAGAUGUUUGCUGC (SEQ ID NO. 9) | hsa-miR-30-d-3p | 0.63 | 7.06E-17 | -0.2220 |
| hsa-miR-30-d-5p | MIMAT0000245 | UGUAAACAUCCCCGACUGGAAG (SEQ ID NO. 10) | hsa-miR-30-d-5p | 0.57 | 1.25E-17 | -0.1874 |
| hsa-mi-R-31-3p | MIMAT0004504 | UGCUAUGCCAACAUAUUGCCAU (SEQ ID NO. 11) | hsa-miR-31-3-p | 5.96 | 2.92E-22 | 0.1535 |
| hsa-mi-R-31-5p | MIMAT0000089 | AGGCAAGAUGCUGGCAUAGCU (SEQ ID NO. 12) | hsa-miR-31-5-p | 5.39 | 3.57E-20 | 0.0326 |
| hsa-mi-R-4709--3p | MIMAT0019812 | UUGAAGAGGAGGUGCUCUGUAGC (SEQ ID NO. 13) | hsa-mi-R-4709-3-p | 0.44 | 2.32E-21 | -0.3032 |
| hsa-miR-548-b-3p | MIMAT0003254 | CAAGAACCUCAGUUGCUUUUGU (SEQ ID NO. 14) | hsa-miR-548-b-3p | 0.54 | 3.59E-11 | -0.1672 |
| hsa-miR-550-a-5p | MIMAT0004800 | AGUGCCUGAGGGAGUAAGAGCCC (SEQ ID NO. 15) | hsa-miR-550-a-5p | 1.48 | 3.60E-10 | 0.1529 |
| hsa-mi-R-582-3p | MIMAT0004797 | UAACUGGUUGAACAACUGAACC (SEQ ID NO. 16) | hsa-mi-R-582-3p | 1.45 | 5.92E-20 | 0.1132 |
| hsa-mi-R-584-5p | MIMAT0003249 | UUAUGGUUUGCCUGGGACUGAG (SEQ ID NO. 17) | hsa-mi-R-584-5p | 1.50 | 3.24E-43 | 0.5229 |
| hsa-mi-R-675-3p | MIMAT0006790 | CUGUAUGCCCUCACCGCUCA (SEQ ID NO. 18) | hsa-mi-R-675-3p | 1.75 | 1.07E-09 | 0.1314 |

(continued)

| The 19-miRNA signature (RNA-seq) | | | | | | |
|---|---|---|---|---|---|---|
| Assay | Acc. mature-miRNA | Sequence (SEQ ID N.) | miRbase ID | Ratio | P-value | Model weight |
| hsa-miR-9-5p | MIMAT0000441 | UCUUUGGUUAUCUAGCUGUAUGA (SEQ ID NO. 19) | hsa-miR-9-5p | 2.42 | 1.78E-07 | 0.0497 |

Assay refers to the code used to identify each miRNA in the TCGA-LUAD RNA sequencing experiment; Acc. mature-miRNA refers to the miRBase accession number of the mature miRNA; Sequence refers to the nucleotide sequence of the mature miRNA; miRbase ID refers to the name of the mature miRNA in the miRbase database; Ratio refers to the fold change calculated dividing the median expression of each miRNA in C1-ADC samples by the median expression in nonC1-ADC samples; p-values were calculated from parametric test of BRB-ArrayTools comparing C1-ADC vs. nonC1-ADC samples. All miRNAs have q-value < 0.05 based on 100 permutation; Model weight refers to the coefficient in the equation used to calculate the predicted class.

**Table 6. The 7-miRNA signature (detected by qRT-PCR).**

| The 14-miRNA signature (RNA-seq) | | | | | | |
|---|---|---|---|---|---|---|
| Assay | Acc. mature-miRNA | Sequence | miRbase ID | Ratio | P-value | Model weight |
| hsa-miR-135-b-5p | MIMAT0000758 | UAUGGCUUUUCAUUCCUAUGUGA (SEQ ID NO. 20) | hsa-miR-135-b-5p | 0.62 | 4.40E-06 | -0.1028 |
| hsa-mi-R-187-3p | MIMAT0000262 | UCGUGUCUUGUGUUGCAGCCGG (SEQ ID NO. 21) | hsa-mi-R-187-3p | 0.56 | 1.42E-03 | -0.0486 |
| hsa-mi-R-192-5p | MIMAT0000222 | CUGACCUAUGAAUUGACAGCC (SEQ ID NO. 22) | hsa-mi-R-192-5p | 1.45 | 1.00E-07 | 0.2828 |
| hsa-miR-193-b-3p | MIMAT0002819 | AACUGGCCCUCAAAGUCCCGCU (SEQ ID NO. 23) | hsa-miR-193-b-3p | 1.57 | 5.00E-07 | 0.1977 |
| hsa-miR-196-b-5p | MIMAT0001080 | UAGGUAGUUUCCUGUUGUUGGG (SEQ ID NO. 5) | hsa-miR-196-b-5p | 3.15 | 1.00E-07 | 0.0201 |
| hsa-mi-R-210-3p | MIMAT0000267 | CUGUGCGUGUGACAGCGGCUGA (SEQ ID NO. 24) | hsa-mi-R-210-3p | 1.43 | 4.06E-05 | 0.1908 |
| hsa-miR-29-b-2-5p | MIMAT0004515 | CUGGUUUCACAUGGUGGCUUAG (SEQ ID NO. 25) | hsa-miR-29-b-2-5p | 0.72 | 1.00E-07 | -0.5074 |
| hsa-mi-R-3065--3p | MIMAT0015378 | UCAGCACCAGGAUAUUGUUGGAG (SEQ ID NO. 26) | hsa-mi-R-3065-3-p | 0.56 | 1.40E-06 | 0.0384 |
| hsa-miR-30-d-5p | MIMAT0000245 | UGUAAACAUCCCCGACUGGAAG (SEQ ID NO. 10) | hsa-miR-30-d-5p | 0.57 | 1.00E-07 | -0.3312 |
| hsa-mi-R-375-3p | MIMAT0000728 | UUUGUUCGUUCGGCUCGCGUGA (SEQ ID NO. 27) | hsa-mi-R-375-3p | 0.66 | 4.53E-05 | -0.0475 |

(continued)

| The 14-miRNA signature (RNA-seq) | | | | | | |
|---|---|---|---|---|---|---|
| Assay | Acc. mature-miRNA | Sequence | miRbase ID | Ratio | P-value | Model weight |
| hsa-mi-R-582-3p | MIMAT0004797 | UAACUGGUUGAACAACUGA ACC (SEQ ID NO. 16) | hsa-mi-R-582-3p | 1.45 | 1.00E-07 | 0.1895 |
| hsa-mi-R-584-5p | MIMAT0003249 | UUAUGGUUUGCCUGGGAC UGAG (SEQ ID NO. 17) | hsa-mi-R-584-5p | 1.50 | 1.00E-07 | 0.5606 |
| hsa-mi-R-708-5p | MIMAT0004926 | AAGGAGCUUACAAUCUAGC UGGG (SEQ ID NO. 28) | hsa-mi-R-708-5p | 1.28 | 2.18E-03 | 0.2663 |
| hsa-miR-9-5p | MIMAT0000441 | UCUUUGGUUAUCUAGCUG UAUGA (SEQ ID NO. 19) | hsa-miR-9-5p | 2.42 | 7.40E-06 | 0.0435 |

Assay refers to the code used to identify each miRNA in the CSS qRT-PCR sequencing experiment; Acc. mature-miRNA refers to the miRBase accession number of the mature miRNA; Sequence refers to the nucleotide sequence of the mature miRNA; miRbase ID refers to the name of the mature miRNA in the miRbase database; Ratio refers to the fold change calculated dividing the median expression of each miRNA in C1-ADC samples by the median expression in nonC1-ADC samples; p-values were calculated from Wilcoxon test comparing C1-ADC vs. nonC1-ADC samples; Model weight refers to the coefficient in the equation used to calculate the predicted class.

**Table 7. The miRNA signature (detected by RNA sequencing).**

| The 7-miRNA signature (qRT-PCR) | | | | | | |
|---|---|---|---|---|---|---|
| Assay | Acc. mature-miRNA | Sequence | miRbase ID | Ratio | P-value | Model weight |
| 478314_mir | MIMAT0002819 | AACUGGCCCUCAAAGUCCCGC U (SEQ ID NO. 23) | hsa-miR-193-b-3p | 0.79 | 0.8933 | 0.1295 |
| 478742_mir | MIMAT0004767 | CGGGGUUUUGAGGGCGAGAU GA (SEQ ID NO. 3) | hsa-miR-193-b-5p | 0.005 | 0.0482 | 0.0920 |
| 478585_mir | MIMAT0001080 | UAGGUAGUUUCCUGUUGUUG GG (SEQ ID NO. 5) | hsa-miR-196-b-5p | 1.60 | 0.2670 | -0.1310 |
| 478012_mir | MIMAT0004504 | UGCUAUGCCAACAUAUUGCCA U (SEQ ID NO. 11) | hsa-mi-R-31-3p | 1.84 | 0.2089 | -0.2116 |
| 478015_mir | MIMAT0000089 | AGGCAAGAUGCUGGCAUAGCU (SEQ ID NO. 12) | hsa-mi-R-31-5p | 6.89 | 0.0769 | -0.2724 |
| 477852_mir | MIMAT0004800 | AGUGCCUGAGGGAGUAAGAGC CC (SEQ ID NO. 15) | hsa-miR-550-a-5p | 1.19 | 0.5830 | 0.2717 |
| 478167_mir | MIMAT0003249 | UUAUGGUUUGCCUGGGACUG AG (SEQ ID NO. 17) | hsa-mi-R-584-5p | 0.60 | 0.1643 | 0.0413 |

Assay refers to the code used to identify each miRNA in the TCGA-LUAD RNA sequencing experiment; Acc. mature-miRNA refers to the miRBase accession number of the mature miRNA; Sequence refers to the nucleotide sequence of the mature miRNA; miRbase ID refers to the name of the mature miRNA in the miRbase database; Ratio refers to the fold change calculated dividing the median expression of each miRNA in C1-ADC samples by the median expression in nonC1-ADC samples; p-values were calculated from Wald test of DESeq2 comparing C1-ADC vs. nonC1-ADC samples. All miRNAs have q-value < 0.05 based on Benjamini-Hochberg adjustment; Model weight refers to the coefficient in the equation used to calculate the predicted class.

| The 7-miRNA signature (RNAseq) | | | | | | |
|---|---|---|---|---|---|---|
| Assay | Acc. mature-miRNA | Sequence | miRbase ID | Ratio | P-value | Model weight |
| hsa-miR-193-b-3p | MIMAT0002819 | AACUGGCCCUCAAAGUCC CGCU (SEQ ID NO. 23) | hsa-miR-193-b-3p | 1.57 | 1.12E-06 | 0.1171 |
| hsa-miR-193-b-5p | MIMAT0004767 | CGGGGUUUUGAGGGCGA GAUGA (SEQ ID NO. 3) | hsa-miR-193-b-5p | 1.44 | 3.01E-08 | 0.2233 |
| hsa-miR-196-b-5p | MIMAT0001080 | UAGGUAGUUUCCUGUUG UUGGG (SEQ ID NO. 5) | hsa-miR-196-b-5p | 3.15 | 1.28E-22 | 0.1341 |
| hsa-mi-R-31-3p | MIMAT0004504 | UGCUAUGCCAACAUAUUG CCAU (SEQ ID NO. 11) | hsa-miR-31-3p | 5.96 | 2.92E-22 | 0.1554 |
| hsa-mi-R-31-5p | MIMAT0000089 | AGGCAAGAUGCUGGCAU AGCU (SEQ ID NO. 12) | hsa-miR-31-5p | 5.39 | 3.57E-20 | 0.0584 |
| hsa-miR-550-a-5p | MIMAT0004800 | AGUGCCUGAGGGAGUAA GAGCCC (SEQ ID NO. 15) | hsa-miR-550-a-5p | 1.48 | 3.60E-10 | 0.3622 |
| hsa-mi-R-584-5p | MIMAT0003249 | UUAUGGUUUGCCUGGGA CUGAG (SEQ ID NO. 17) | hsa-mi-R-584-5p | 1.50 | 3.24E-43 | 0.4683 |

The parameters of logistic regression of the three signatures and the formula used to include the groups of patients in C1 or nonC1 group are represented here below:

**Model 19-miRNA (RNA sequencing):**

z=-8.2029+(-0.2651*hsa-let-7c-3p)+(0.1709*hsa-miR-138-5p)+(0.1443*hsa-miR-193b-5p)+(0.0200*hsa-miR-196a-5p)+(0.0464*hsa-miR-196b-5p)+(0.2297*hsa-miR-203a-3p)+(0.1285*hsa-miR-215-5p)+(0.3933*hsa-miR-2355-3p)+(-0.2220*hsa-miR-30d-3p)+(-0.1874*hsa-miR-30d-5p)+(0.1535*hsa-miR-31-3p)+(0.0326*hsa-miR-31-5p)+(-0.3032*hsa-miR-4709-3p)+(-0.1672*hsa-miR-548b-3p)+(0.1529*hsa-miR-550a-5p)+(0.1132*hsa-miR-582-3p)+(0.5229*hsa-miR-584-5p)+(0.1314*hsa-miR-675-3p)+(0.0497*hsa-miR-9-5p)

**Model 14-miRNA (RNA sequencing):**

z=-5.4414+(-0.1028*hsa-miR-135b-5p)+(-0.0486*hsa-miR-187-3p)+(0.2828*hsa- miR-192-5p)+(0.1977*hsa-miR-193b-3p)+(0.0201*hsa-miR-196b-5p)+(0.1908*hsa-         miR-210-3p)+(-0.5074*hsa-miR-29b-2-5p)+(0.0384*hsa-miR-3065-3p)+(-0.3312*hsa-miR-30d-5p)+(-0.0475*hsa-miR-375-3p)+(0.1895*hsa-miR-582-3p)+(0.5606*hsa-miR-584-5p)+(0.2663*hsa-miR-708-5p)+(0.0435*hsa-miR-9-5p)

**Model 7-miRNA (RNA sequencing):**

z=-8.5210+(0.1171*hsa-miR-193b-3p)+(0.2233*hsa-miR-193b-5p)+(0.1341*hsa-miR-196b-5p)+(0.1554*hsa-miR-31-3p)+(0.0584*hsa-miR-31-5p)+(0.3622*hsa-miR-550a-5p)+(0.4683*hsa-miR-584-5p)

**Model 7-miRNA (qRT-PCR):**

z=2.2920    +(hsa-miR-193b-3p*0.1295)+(hsa-miR-193b-5p*0.0920)+(hsa-miR-196b-5p*-0.1310)+(hsa-miR-31-3p*-0.2116)+(hsa-miR-31-5p*-0.2724)+(hsa-miR-550a-5p*0.2717)+(hsa-miR-584-5p*0.0413)

The formula shown herein is used to identify the predicted class of patient selected by using the models reported above:

$$Predicted\ class = \begin{cases} C1, & \dfrac{1}{1+e^{-z}} \geq 0.5 \\ nonC1, & \dfrac{1}{1+e^{-z}} < 0.5 \end{cases}$$

*4. Evaluation of the AUC obtained from other 14- and 19-miRNAs signatures.*

To demonstrate that the 14- and 19-miRNAs signatures, from which the 7 miRNAs signature derived, are the most effective to identify the more aggressive subtype of lung cancer (C1), we compared the AUC of the 14- and 19-miRNA models to the expected AUC distribution derived from random signatures of equal lengths (14 and 19 miRNAs length) (Figure 4). In particular, we generated:

- 100 random lists of 14 miRNAs from 292 miRNAs not differentially expressed according to BRB-Array Tools (Table 8);
- 100 random lists of 14 miRNAs from 76 miRNAs differentially expressed according to BRB-Array Tools excluding the 14 miRNAs of the model (Table 9);
- 100 random lists of 19 miRNAs from 182 miRNAs not differentially expressed according to DESeq2 tool (Table 10);
- 100 random lists of 19 miRNAs from 181 miRNAs differentially expressed according to DESeq2 tool excluding the 19 miRNAs of the model (Table 11).

**Table 8. List of 292 miRNAs not differentially expressed according to BRB-Array Tools.**

| miRNA ID | | | | |
|---|---|---|---|---|
| hsa-miR-215-5p | hsa-miR-22-3p | hsa-miR-501-3p | hsa-miR-324-3p | hsa-let-7a-2-3p |
| hsa-miR-4709-3p | hsa-miR-222-3p | hsa-miR-27a-5p | hsa-miR-378a-5p | hsa-miR-133a-3p |
| hsa-miR-30d-3p | hsa-miR-99b-3p | hsa-miR-454-3p | hsa-miR-503-5p | hsa-miR-93-3p |
| hsa-miR-138-5p | hsa-miR-500b-5p | hsa-miR-758-3p | hsa-miR-320a | hsa-miR-3934-3p |
| hsa-miR-548b-3p | hsa-miR-212-3p | hsa-miR-3615 | hsa-miR-16-2-3p | hsa-miR-224-3p |
| hsa-miR-675-3p | hsa-miR-136-5p | hsa-miR-653-5p | hsa-miR-30a-5p | hsa-miR-25-3p |
| hsa-miR-2355-3p | hsa-miR-758-5p | hsa-let-7f-5p | hsa-let-7b-3p | hsa-miR-15a-5p |
| hsa-miR-203a-3p | hsa-miR-500a-5p | hsa-let-7d-5p | hsa-miR-3913-5p | hsa-miR-642a-5p |
| hsa-let-7c-3p | hsa-miR-532-3p | hsa-miR-382-5p | hsa-let-7a-5p | hsa-miR-1-3p |
| hsa-miR-196a-5p | hsa-miR-509-3p | hsa-miR-151a-3p | hsa-let-7b-5p | hsa-miR-1180-3p |
| hsa-miR-31-3p | hsa-miR-3065-5p | hsa-miR-221-5p | hsa-miR-214-5p | hsa-miR-877-5p |
| hsa-miR-31-5p | hsa-miR-577 | hsa-miR-424-3p | hsa-miR-200c-3p | hsa-miR-221-3p |
| hsa-miR-550a-5p | hsa-miR-1247-3p | hsa-miR-24-3p | hsa-miR-374b-5p | hsa-miR-363-3p |
| hsa-miR-193b-5p | hsa-miR-132-5p | hsa-miR-744-3p | hsa-miR-3607-3p | hsa-miR-493-3p |
| hsa-miR-664b-3p | hsa-miR-1287-3p | hsa-miR-369-3p | hsa-miR-142-3p | hsa-miR-337-3p |
| hsa-miR-153-5p | hsa-miR-217 | hsa-miR-200a-3p | hsa-miR-451a | hsa-miR-32-5p |
| hsa-miR-664a-5p | hsa-miR-106a-5p | hsa-miR-3130-5p | hsa-miR-143-3p | hsa-miR-5698 |

(continued)

| miRNA ID | | | | |
|---|---|---|---|---|
| hsa-miR-421 | hsa-miR-17-5p | hsa-miR-342-5p | hsa-miR-150-3p | hsa-miR-26b-5p |
| hsa-miR-582-5p | hsa-miR-508-3p | hsa-miR-214-3p | hsa-miR-20a-5p | hsa-miR-99b-5p |
| hsa-miR-18a-5p | hsa-miR-514a-3p | hsa-miR-574-3p | hsa-miR-361-3p | hsa-miR-889-3p |
| hsa-miR-432-5p | hsa-miR-455-5p | hsa-let-7e-3p | hsa-miR-425-3p | hsa-miR-185-5p |
| hsa-miR-942-5p | hsa-miR-590-3p | hsa-miR-5586-5p | hsa-miR-30c-2-3p | hsa-miR-3127-5p |
| hsa-miR-3677-3p | hsa-miR-3074-5p | hsa-miR-16-5p | hsa-miR-33a-5p | hsa-miR-28-3p |
| hsa-miR-362-5p | hsa-miR-181a-2-3p | hsa-miR-378a-3p | hsa-miR-144-5p | hsa-miR-370-3p |
| hsa-miR-766-3p | hsa-miR-141-5p | hsa-miR-103a-3p | hsa-miR-199a-5p | hsa-miR-139-3p |
| hsa-miR-1306-5p | hsa-miR-29a-5p | hsa-miR-505-5p | hsa-miR-598-3p | hsa-miR-98-5p |
| hsa-miR-30b-3p | hsa-miR-410-3p | hsa-miR-3200-3p | hsa-let-7f-1-3p | hsa-miR-27b-5p |
| hsa-miR-320b | hsa-miR-625-5p | hsa-miR-181d-5p | hsa-miR-301a-5p | hsa-miR-10b-5p |
| hsa-miR-101-5p | hsa-miR-4326 | hsa-miR-378c | hsa-miR-452-3p | hsa-miR-96-5p |
| hsa-miR-629-3p | hsa-miR-149-5p | hsa-miR-15b-5p | hsa-miR-19b-1-5p | hsa-miR-429 |
| hsa-miR-362-3p | hsa-miR-1307-5p | hsa-miR-3614-5p | hsa-miR-224-5p | hsa-miR-29b-1-5p |
| hsa-miR-151b | hsa-miR-4677-3p | hsa-miR-3613-5p | hsa-miR-301a-3p | hsa-miR-34b-5p |
| hsa-miR-205-5p | hsa-miR-125a-3p | hsa-miR-203b-3p | hsa-miR-744-5p | hsa-miR-145-3p |
| hsa-miR-338-3p | hsa-miR-181c-5p | hsa-miR-1269a | hsa-let-7e-5p | hsa-miR-34c-5p |
| hsa-miR-576-5p | hsa-miR-199b-5p | hsa-miR-7-1-3p | hsa-miR-107 | hsa-miR-10a-5p |
| hsa-miR-4772-3p | hsa-miR-501-5p | hsa-miR-142-5p | hsa-miR-145-5p | hsa-miR-16-1-3p |
| hsa-miR-4661-5p | hsa-miR-19a-3p | hsa-miR-376c-3p | hsa-miR-330-3p | hsa-miR-423-3p |
| hsa-miR-20b-5p | hsa-miR-152-3p | hsa-miR-190a-5p | hsa-miR-335-5p | hsa-miR-379-5p |
| hsa-miR-195-3p | hsa-miR-345-5p | hsa-miR-409-3p | hsa-miR-21-5p | hsa-miR-1247-5p |
| hsa-miR-95-3p | hsa-miR-100-5p | hsa-let-7i-5p | hsa-miR-424-5p | hsa-let-7a-3p |
| hsa-miR-493-5p | hsa-miR-671-5p | hsa-miR-92a-3p | hsa-miR-140-5p | hsa-miR-126-5p |
| hsa-miR-222-5p | hsa-miR-671-3p | hsa-miR-450b-5p | hsa-miR-181c-3p | hsa-miR-505-3p |
| hsa-miR-431-3p | hsa-miR-148b-3p | hsa-miR-1468-5p | hsa-miR-3653-3p | hsa-miR-183-5p |
| hsa-miR-331-5p | hsa-miR-338-5p | hsa-miR-452-5p | hsa-miR-181b-3p | hsa-miR-374a-5p |
| hsa-miR-185-3p | hsa-miR-136-3p | hsa-miR-34c-3p | hsa-miR-26b-3p | hsa-miR-144-3p |
| hsa-miR-193a-3p | hsa-miR-200b-5p | hsa-miR-155-5p | hsa-miR-340-3p | hsa-miR-33b-5p |
| hsa-miR-1296-5p | hsa-miR-140-3p | hsa-miR-129-5p | hsa-miR-6842-3p | hsa-miR-126-3p |
| hsa-miR-495-3p | hsa-miR-204-5p | hsa-miR-409-5p | hsa-miR-374a-3p | hsa-miR-342-3p |
| hsa-miR-125b-2-3p | hsa-miR-937-3p | hsa-miR-127-3p | hsa-miR-423-5p | hsa-miR-17-3p |
| hsa-miR-146a-5p | hsa-miR-484 | hsa-let-7i-3p | hsa-miR-92b-3p | hsa-miR-181b-2-3p |
| hsa-miR-130b-5p | hsa-miR-450a-5p | hsa-miR-28-5p | hsa-miR-651-5p | hsa-miR-652-3p |
| hsa-miR-550a-3p | hsa-miR-455-3p | hsa-miR-141-3p | hsa-miR-130a-3p | hsa-miR-34b-3p |
| hsa-miR-502-3p | hsa-miR-20a-3p | hsa-miR-628-5p | hsa-miR-381-3p | hsa-miR-296-5p |
| hsa-miR-4662a-5p | hsa-miR-10a-3p | hsa-let-7d-3p | hsa-miR-19b-3p | hsa-miR-511-5p |
| hsa-miR-200a-5p | hsa-miR-335-3p | hsa-miR-199a-3p | hsa-let-7g-3p | hsa-miR-191-5p |
| hsa-miR-26a-2-3p | hsa-miR-589-5p | hsa-miR-199b-3p | hsa-miR-361-5p | hsa-miR-30a-3p |

(continued)

| miRNA ID | | | | |
|---|---|---|---|---|
| hsa-miR-128-1-5p | hsa-miR-340-5p | hsa-miR-143-5p | hsa-miR-186-5p | |
| hsa-miR-487b-3p | hsa-miR-127-5p | hsa-miR-654-3p | hsa-miR-200b-3p | |
| hsa-miR-486-5p | hsa-miR-324-5p | hsa-miR-125b-5p | hsa-miR-411-5p | |

**Table 9. List of 76 miRNAs differentially expressed according to BRB-Array Tools excluding the 14 miRNAs of the model.**

| miRNA ID | | | | |
|---|---|---|---|---|
| hsa-miR-326 | hsa-miR-330-5p | hsa-miR-30c-5p | hsa-miR-30e-3p | hsa-miR-769-5p |
| hsa-miR-1266-5p | hsa-miR-195-5p | hsa-miR-34a-5p | hsa-miR-365a-3p | hsa-miR-139-5p |
| hsa-miR-1976 | hsa-miR-331-3p | hsa-miR-181a-5p | hsa-miR-146b-5p | hsa-miR-328-3p |
| hsa-miR-1301-3p | hsa-miR-22-5p | hsa-miR-181b-5p | hsa-miR-500a-3p | hsa-miR-148a-5p |
| hsa-miR-590-5p | hsa-miR-2355-5p | hsa-miR-182-5p | hsa-miR-532-5p | hsa-miR-101-3p |
| hsa-miR-24-1-5p | hsa-miR-660-5p | hsa-miR-181a-3p | hsa-miR-542-3p | hsa-miR-29b-3p |
| hsa-miR-874-3p | hsa-miR-15b-3p | hsa-miR-223-3p | hsa-miR-425-5p | hsa-miR-197-3p |
| hsa-miR-130b-3p | hsa-miR-29c-5p | hsa-let-7g-5p | hsa-miR-21-3p | hsa-miR-148a-3p |
| hsa-miR-24-2-5p | hsa-let-7c-5p | hsa-miR-23b-3p | hsa-miR-193a-5p | hsa-miR-194-5p |
| hsa-miR-497-5p | hsa-miR-23a-3p | hsa-miR-27b-3p | hsa-miR-106b-3p | hsa-miR-106b-5p |
| hsa-miR-664a-3p | hsa-miR-26a-5p | hsa-miR-30b-5p | hsa-miR-151a-5p | hsa-miR-29c-3p |
| hsa-miR-200c-5p | hsa-miR-27a-3p | hsa-miR-128-3p | hsa-miR-146b-3p | hsa-miR-30e-5p |
| hsa-miR-339-3p | hsa-miR-29a-3p | hsa-miR-132-3p | hsa-miR-625-3p | |
| hsa-miR-339-5p | hsa-miR-93-5p | hsa-miR-125a-5p | hsa-miR-629-5p | |
| hsa-miR-218-5p | hsa-miR-99a-5p | hsa-miR-150-5p | hsa-miR-1287-5p | |
| hsa-miR-134-5p | hsa-miR-365b-3p | hsa-miR-1307-3p | hsa-miR-708-3p | |

**Table 10. List of 182 miRNAs not differentially expressed according to DESeq2 tool.**

| miRNA ID | | | | |
|---|---|---|---|---|
| hsa-miR-210-3p | hsa-miR-24-3p | hsa-miR-29b-1-5p | hsa-miR-744-5p | hsa-miR-758-3p |
| hsa-miR-375 | hsa-miR-744-3p | hsa-miR-34b-5p | hsa-let-7e-5p | hsa-miR-3615 |
| hsa-miR-134-5p | hsa-miR-369-3p | hsa-miR-145-3p | hsa-miR-107 | hsa-miR-653-5p |
| hsa-miR-671-3p | hsa-miR-200a-3p | hsa-miR-34c-5p | hsa-miR-145-5p | hsa-let-7f-5p |
| hsa-miR-148b-3p | hsa-miR-3130-5p | hsa-miR-10a-5p | hsa-miR-330-3p | hsa-let-7d-5p |
| hsa-miR-338-5p | hsa-miR-342-5p | hsa-miR-16-1-3p | hsa-miR-335-5p | hsa-miR-382-5p |
| hsa-miR-136-3p | hsa-miR-214-3p | hsa-miR-423-3p | hsa-miR-21-5p | hsa-miR-151a-3p |
| hsa-miR-200b-5p | hsa-miR-574-3p | hsa-miR-379-5p | hsa-miR-424-5p | hsa-miR-221-5p |
| hsa-miR-140-3p | hsa-let-7e-3p | hsa-miR-1247-5p | hsa-miR-140-5p | hsa-miR-424-3p |
| hsa-miR-204-5p | hsa-miR-5586-5p | hsa-let-7a-3p | hsa-miR-181c-3p | hsa-let-7i-3p |
| hsa-miR-937-3p | hsa-miR-16-5p | hsa-miR-126-5p | hsa-miR-3653-3p | hsa-miR-28-5p |
| hsa-miR-484 | hsa-miR-378a-3p | hsa-miR-505-3p | hsa-miR-181b-3p | hsa-miR-141-3p |
| hsa-miR-450a-5p | hsa-miR-103a-3p | hsa-miR-183-5p | hsa-miR-26b-3p | hsa-miR-628-5p |
| hsa-miR-455-3p | hsa-miR-505-5p | hsa-miR-374a-5p | hsa-miR-340-3p | hsa-let-7d-3p |

(continued)

| miRNA ID | | | | |
|---|---|---|---|---|
| hsa-miR-20a-3p | hsa-miR-3200-3p | hsa-miR-324-3p | hsa-miR-6842-3p | hsa-miR-199a-3p |
| hsa-miR-10a-3p | hsa-miR-181d-5p | hsa-miR-378a-5p | hsa-miR-374a-3p | hsa-miR-199b-3p |
| hsa-miR-335-3p | hsa-miR-378c | hsa-miR-503-5p | hsa-miR-423-5p | hsa-miR-143-5p |
| hsa-miR-589-5p | hsa-miR-15b-5p | hsa-miR-320° | hsa-miR-92b-3p | hsa-miR-654-3p |
| hsa-miR-340-5p | hsa-miR-3614-5p | hsa-miR-16-2-3p | hsa-miR-651-5p | hsa-miR-144-5p |
| hsa-miR-127-5p | hsa-miR-3613-5p | hsa-miR-30a-5p | hsa-miR-130a-3p | hsa-miR-199a-5p |
| hsa-miR-32-5p | hsa-miR-203b-3p | hsa-let-7b-3p | hsa-miR-381-3p | hsa-miR-598-3p |
| hsa-miR-5698 | hsa-miR-1269a | hsa-miR-3913-5p | hsa-miR-19b-3p | hsa-let-7f-1-3p |
| hsa-miR-26b-5p | hsa-miR-7-1-3p | hsa-let-7a-5p | hsa-let-7g-3p | hsa-miR-301a-5p |
| hsa-miR-99b-5p | hsa-miR-142-5p | hsa-let-7b-5p | hsa-miR-361-5p | hsa-miR-452-3p |
| hsa-miR-889-3p | hsa-miR-376c-3p | hsa-miR-214-5p | hsa-miR-186-5p | hsa-miR-19b-1-5p |
| hsa-miR-185-5p | hsa-miR-190a-5p | hsa-miR-200c-3p | hsa-miR-200b-3p | hsa-miR-224-5p |
| hsa-miR-3127-5p | hsa-miR-409-3p | hsa-miR-374b-5p | hsa-miR-33b-5p | hsa-miR-301a-3p |
| hsa-miR-28-3p | hsa-let-7i-5p | hsa-miR-3607-3p | hsa-miR-126-3p | hsa-miR-486-5p |
| hsa-miR-370-3p | hsa-miR-92a-3p | hsa-miR-142-3p | hsa-miR-342-3p | hsa-miR-324-5p |
| hsa-miR-139-3p | hsa-miR-450b-5p | hsa-miR-451° | hsa-miR-17-3p | hsa-miR-125b-5p |
| hsa-miR-98-5p | hsa-miR-1468-5p | hsa-miR-143-3p | hsa-miR-181 b-2-3p | hsa-miR-337-3p |
| hsa-miR-27b-5p | hsa-miR-452-5p | hsa-miR-150-3p | hsa-miR-652-3p | hsa-miR-429 |
| hsa-miR-10b-5p | hsa-miR-34c-3p | hsa-miR-20a-5p | hsa-miR-34b-3p | hsa-miR-144-3p |
| hsa-miR-96-5p | hsa-miR-155-5p | hsa-miR-361-3p | hsa-miR-296-5p | hsa-miR-411-5p |
| hsa-miR-501-3p | hsa-miR-129-5p | hsa-miR-425-3p | hsa-miR-511-5p | |
| hsa-miR-27a-5p | hsa-miR-409-5p | hsa-miR-30c-2-3p | hsa-miR-191-5p | |
| hsa-miR-454-3p | hsa-miR-127-3p | hsa-miR-33a-5p | hsa-miR-30a-3p | |

**Table 11. List of 181 miRNAs differentially expressed according to DESeq2 tool excluding the 19 miRNAs of the model.**

| miRNA ID | | | | |
|---|---|---|---|---|
| hsa-miR-192-5p | hsa-miR-362-3p | hsa-miR-4662a-5p | hsa-miR-590-5p | hsa-miR-101-5p |
| hsa-miR-29b-2-5p | hsa-miR-151b | hsa-miR-200a-5p | hsa-miR-200c-5p | hsa-miR-26a-5p |
| hsa-miR-135b-5p | hsa-miR-29c-5p | hsa-miR-625-3p | hsa-miR-3065-5p | hsa-miR-181a-3p |
| hsa-miR-3065-3p | hsa-miR-125a-5p | hsa-miR-26a-2-3p | hsa-miR-577 | hsa-miR-497-5p |
| hsa-miR-187-3p | hsa-miR-660-5p | hsa-miR-218-5p | hsa-miR-1247-3p | hsa-miR-30e-5p |
| hsa-miR-708-5p | hsa-miR-2355-5p | hsa-miR-128-1-5p | hsa-miR-151a-5p | hsa-miR-139-5p |
| hsa-miR-193b-3p | hsa-miR-205-5p | hsa-miR-487b-3p | hsa-miR-1287-5p | hsa-miR-30e-3p |
| hsa-miR-194-5p | hsa-miR-23a-3p | hsa-miR-148a-3p | hsa-miR-132-5p | hsa-miR-146b-3p |
| hsa-miR-664a-3p | hsa-miR-338-3p | hsa-let-7a-2-3p | hsa-miR-1287-3p | hsa-miR-629-3p |
| hsa-miR-664b-3p | hsa-miR-106b-5p | hsa-miR-133a-3p | hsa-miR-217 | hsa-miR-146a-5p |
| hsa-miR-181a-5p | hsa-miR-1301-3p | hsa-miR-197-3p | hsa-miR-106a-5p | hsa-miR-130b-5p |
| hsa-miR-30b-5p | hsa-miR-223-3p | hsa-miR-93-3p | hsa-miR-29b-3p | hsa-miR-29c-3p |
| hsa-miR-153-5p | hsa-miR-365b-3p | hsa-miR-874-3p | hsa-miR-17-5p | hsa-miR-27a-3p |

(continued)

| miRNA ID | | | | |
|---|---|---|---|---|
| hsa-miR-664a-5p | hsa-miR-365a-3p | hsa-miR-132-3p | hsa-miR-508-3p | hsa-miR-550a-3p |
| hsa-miR-99a-5p | hsa-miR-576-5p | hsa-miR-182-5p | hsa-miR-514a-3p | hsa-miR-15b-3p |
| hsa-miR-421 | hsa-miR-193a-5p | hsa-miR-3934-3p | hsa-miR-455-5p | hsa-miR-502-3p |
| hsa-miR-582-5p | hsa-miR-4772-3p | hsa-miR-224-3p | hsa-miR-22-5p | hsa-miR-328-3p |
| hsa-miR-30c-5p | hsa-miR-4661-5p | hsa-miR-25-3p | hsa-miR-590-3p | hsa-miR-330-5p |
| hsa-miR-128-3p | hsa-miR-769-5p | hsa-miR-21-3p | hsa-miR-3074-5p | hsa-miR-136-5p |
| hsa-miR-101-3p | hsa-miR-34a-5p | hsa-miR-15a-5p | hsa-miR-181a-2-3p | hsa-miR-758-5p |
| hsa-miR-18a-5p | hsa-miR-148a-5p | hsa-miR-642a-5p | hsa-miR-141-5p | hsa-miR-500a-5p |
| hsa-miR-432-5p | hsa-miR-20b-5p | hsa-miR-339-3p | hsa-miR-708-3p | hsa-miR-93-5p |
| hsa-miR-942-5p | hsa-miR-1266-5p | hsa-miR-1-3p | hsa-miR-29a-5p | hsa-miR-339-5p |
| hsa-miR-130b-3p | hsa-miR-195-3p | hsa-miR-1180-3p | hsa-miR-410-3p | hsa-miR-500a-3p |
| hsa-let-7c-5p | hsa-miR-95-3p | hsa-miR-425-5p | hsa-miR-625-5p | hsa-miR-532-3p |
| hsa-miR-3677-3p | hsa-miR-493-5p | hsa-miR-877-5p | hsa-miR-24-1-5p | hsa-miR-509-3p |
| hsa-miR-362-5p | hsa-miR-222-5p | hsa-miR-221-3p | hsa-miR-331-3p | hsa-miR-1976 |
| hsa-miR-195-5p | hsa-miR-431-3p | hsa-miR-23b-3p | hsa-miR-4326 | hsa-miR-152-3p |
| hsa-miR-629-5p | hsa-miR-29a-3p | hsa-miR-363-3p | hsa-miR-149-5p | hsa-miR-326 |
| hsa-miR-181b-5p | hsa-miR-27b-3p | hsa-miR-24-2-5p | hsa-miR-1307-5p | hsa-miR-345-5p |
| hsa-miR-106b-3p | hsa-miR-331-5p | hsa-miR-493-3p | hsa-let-7g-5p | hsa-miR-100-5p |
| hsa-miR-766-3p | hsa-miR-185-3p | hsa-miR-22-3p | hsa-miR-4677-3p | hsa-miR-150-5p |
| hsa-miR-1306-5p | hsa-miR-193a-3p | hsa-miR-222-3p | hsa-miR-125a-3p | hsa-miR-671-5p |
| hsa-miR-30b-3p | hsa-miR-1296-5p | hsa-miR-99b-3p | hsa-miR-181c-5p | |
| hsa-miR-146b-5p | hsa-miR-495-3p | hsa-miR-500b-5p | hsa-miR-199b-5p | |
| hsa-miR-1307-3p | hsa-miR-532-5p | hsa-miR-542-3p | hsa-miR-19a-3p | |
| hsa-miR-320b | hsa-miR-125b-2-3p | hsa-miR-212-3p | hsa-miR-501-5p | |

The same logistic regression used for the selected signature was applied to each random list, and corresponding model performances were evaluated through AUC.

As shown in Figure 4, none of the AUC obtained using these random signatures were as high as the AUC obtained using the 14- and 19-miRNAs models, which demonstrate that the method applied for model selection was the most effective to identify the best set of miRNAs diagnostic for C1 subtype of lung cancer (Figure 4).

## MATERIALS AND METHODS

We selected the cohort of 515 patients with lung adenocarcinomas from the TCGA data portal (https://portal.gdc.cancer.gov/) at 2018. A total of 510 tumors were profiled for both gene and miRNA expression. Log2 read counts were used for expression analysis. Patients follow-up information was used for survival analysis: overall survival was defined as the time from the date of tumor resection until death from any cause.

Follow-up was truncated at 3 years to reduce the potential overestimation of overall mortality with respect to lung cancer-specific mortality.

Hierarchical clustering analysis was performed on the 10-gene signature for the entire cohort of 510 patients. Clustering was done by using Cluster 3.0 for Mac OS X (C Clustering Library 1.56) with uncentered correlation and centroid linkage, and Java TreeView software environment (version 1.1.6r4; http://jtreeview.sourceforge.net). Four main branches were selected to build clusters. Kaplan-Meier survival curves were stratified by clusters and log-rank test p-values were calculated. C1 cluster was associated to the worse prognosis, and all other clusters were pooled together (nonC1 clusters).

**EP 4 214 335 B1**

To reduce the complexity of the TCGA-LUAD dataset (2237 miRNAs) and extract the most informative data to use, we selected the most transcriptionally regulated miRNAs. We selected those miRNAs with raw counts > 0 in at least the 50% of patients either in C1 or nonC1, identifying a total of 382 miRNAs. We applied the complexity reduction also to genes and we selected the most varying across all samples (standard deviation in the top 25%), identifying a total of 4899 genes. Using DESeq2 R package, we identified a total of 2900 differentially expressed genes between C1 and nonC1 tumors.

BRB-ArrayTools [10] and DESeq2 (R package) [11] tools were used for class prediction (C1 cluster vs nonC1 clusters) according to miRNA expression. BRB-ArrayTools uses statistics based on two-sample T-test with multivariate permutations test (1000 random permutations); confidence level of false discovery rate assessment, 80%; maximum allowed proportion of false-positive genes, 0.05. DESeq2 is based on Wald test statistics to identify differentially expressed transcripts. Lists of miRNAs differentially expressed obtained from BRB-ArrayTools and DESeq2 tools were subsequently reduced via Lasso regularization. In details, a penalized unconditional logistic regression was applied considering cluster as discrete outcome (C1 cluster vs. nonC1 clusters) and miRNA expressions as explanatory variables. Cross-validated (10-fold) log-likelihood with optimization (50 simulations) of the tuning penalty parameter was used to control for potential overfitting.

Starting from differentially expressed genes (identified with DESeq2) and miRNAs (identified with both DESeq2 and BRB-ArrayTools), we used ARACNe [12] with 1000 bootstraps to infer direct regulatory relationships between transcriptional regulators (i.e. miRNAs) and target genes. ARACNe was performed using all patients, stage I patients and stage II-IV patients. miRNA target genes were retrieved using miRWalk 3.0 [13].

Probability of being in the C1 cluster was estimated using the unconditional logistic regression for the 3 signatures of 19, 14 and 7 miRNAs. Model performance was assessed using the cross-validated area under the receiver operating curve, and assessing the difference in C1 predicted probability between C1 and nonC1 patients (Wilcoxon-Mann-Whitney test). Cox regression model was used to evaluate the prognostic role of these miRNA signatures and their ability to recapitulate the risk-stratification of the original 10-genes signature.

To get insights in the biology of the 7-miRNA model, we verify the enrichment of cancer-relevant pathways associated to their target genes. We investigated the Molecular Signature Database (MSigDB; v7.2) (https://www.gsea-msigdb.org/gsea/msigdb/annotate.jsp) using the list of 87 targeted genes by interrogating the CGP (chemical and genetic perturbations, 3358 gene sets). Bubble plot analysis was performed using JMP 15.2.1 (SAS) software.

Hierarchical clustering analysis was performed on the 7-miRNA signature for 510 patients, those with available miRNA expression data. Clustering was done by using Cluster 3.0 for Mac OS X (C Clustering Library 1.56) with uncentered correlation and centroid linkage, and Java TreeView software environment (version 1.1.6r4; http://jtreeview.sourceforge.net).

The 7 miRNAs signature was identified as the best for risk-stratification and therefore validated in an external cohort of patients from IRCCS Casa Sollievo della Sofferenza Hospital (CSS, San Giovanni Rotondo, Italy). Between February 2017 and February 2020, 44 patients with lung adenocarcinoma underwent surgery at the CSS. Written informed consent was obtained from all study patients. None of these patients received preoperative chemotherapy. Clinical information was obtained through review of medical records. Vital status was assessed through the Vital Records Offices of the patients' towns of residence or by contacting directly the patients or their families.

One tissue core (1.5 mm in diameter) from FFPE blocks, in representative tumor areas with adequate tumor cellularity (>60%) selected by a pathologist, was processed for total RNA extraction. The AllPrep DNA/RNA FFPE kit (QIAGEN) was used for Total RNA extraction. Quantitative real-time PCR (qRT-PCR) was performed to analyze the 10-genes signature as described in Dama et al [6]. Briefly, RNA was quantified using Nanodrop ND-10000 Spectrophotometer and a total of 200 ng was retro-transcribed using SuperScript VILO cDNA Synthesis Kit (Thermo Fisher Scientific) and pre-amplified for 10 cycles with PreAmp Master Mix Kit (ThermoFisher Scientific), following manufacturer's instructions. qRT-PCR analysis was performed starting from 1:10 diluted pre-amplified cDNA, using the TaqMan Fast Advance Master Mix and hydrolysis probes (ThermoFisher Scientific; for primers see Dama et al [6]), in a QuantStudio 12k Flex (ThermoFisher Scientific). Thermal cycling amplification was performed with an initial incubation at 95°C for 30 seconds, followed by 45 cycles of 95°C for 5 seconds and 60°C for 30 seconds. For miRNA expression analysis, a total of 10 ng RNA was reverse-transcribed using the TaqMan Advanced miRNA cDNA Synthesis Kit (ThermoFisher Scientific). Poly(A) tailing, adapter ligation, RT reaction and miR-Amp (using TaqMan Advanced miRNA assays) were performed following manufacturer's instructions [15], i.e.: 95°C for 30 seconds, 45 cycles of 95°C for 5 seconds, and 60°C for 30 seconds, using a Card Custom Advance (ThermoFisher Scientific) in a QuantStudio 12k Flex (ThermoFisher Scientific).

The hsa-miR-16-5p was used as standard reference for CT normalization using a previously described methodology [6]. Briefly, the normalized CT of each miRNA (i) of each sample (j) was calculated as difference between the raw CTij and a scaling factor (SF) specific for each sample (j); the SFj represented the difference between the raw CT of the miRNA "hsa-miR-16-5p" used as a reference in the sample (j) and a constant equal to 21.87.

Risk-scores were assigned to each patient based to the 10-gene risk model described in Dama et al. [6]. Before applying the risk-model, data were rescaled (q1-q3 normalization). Patients with risk-scores higher than the $66^{th}$

percentiles (6) were classified as C1 tumors. Next, unconditional logistic regression (C1 vs nonC1 tumors) with 7 miRNAs as explanatory variables was applied, and the area under the receiver operating curve was calculated. Difference in C1 predicted probability between C1 and nonC1 patients was evaluated through Wilcoxon-Mann-Whitney test.

All statistical analyses were performed using SAS software, version 9.4 (SAS Institute, Inc., Cary, NC) and R 3.3.1 (R Core Team, 2016) and JMP 15 (SAS). P-values less than 0.05 were considered statistically significant.

**Table 12. Commercial assays used to amplify each miRNA of the 19-, 14- and 7-miRNA signatures.**

Assay refers to the code used to identify each miRNA in the qRT-PCR experiment; "Acc. mature-miRNA" refers to the miRBase accession number of the mature miRNA; "miRbase ID" refers to the name of the mature miRNA in the miRbase database; "Signature" refers to the specific signature each miRNA belongs to; "house-keeping miRNA" is the miRNA used to normalized the qRT-PCR experiments; "spike-in" miRNA is the miRNA used to control the performance of the qRT-PCR experiments.

| Assay | Acc. mature-miRNA | miRbase ID | Signature |
|---|---|---|---|
| 479365_mir | MIMAT0026472 | hsa-let-7c-3p | 19-miRNA |
| 478582_mir | MIMAT0000758 | hsa-miR-135b-5p | 14-miRNA |
| 477905_mir | MIMAT0000430 | hsa-miR-138-5p | 19-miRNA |
| 477941_mir | MIMAT0000262 | hsa-miR-187-3p | 14-miRNA |
| 478262_mir | MIMAT0000222 | hsa-miR-192-5p | 14-miRNA |
| 478314_mir | MIMAT0002819 | hsa-miR-193b-3p | 14- and 7-miRNA |
| 478742_mir | MIMAT0004767 | hsa-miR-193b-5p | 19- and 7-miRNA |
| 478230_mir | MIMAT0000226 | hsa-miR-196a-5p | 19-miRNA |
| 478585_mir | MIMAT0001080 | hsa-miR-196b-5p | 19-, 14-miRNA and 7-miRNA |
| 478316_mir | MIMAT0000264 | hsa-miR-203a-3p | 19-miRNA |
| 477970_mir | MIMAT0000267 | hsa-miR-210-3p | 14-miRNA |
| 478516_mir | MIMAT0000272 | hsa-miR-215-5p | 19-miRNA |
| 477989_mir | MIMAT0017950 | hsa-miR-2355-3p | 19-miRNA |
| 478003_mir | MIMAT0004515 | hsa-miR-29b-2-5p | 14-miRNA |
| 479605_mir | MIMAT0015378 | hsa-miR-3065-3p | 14-miRNA |
| 479362_mir | MIMAT0004551 | hsa-miR-30d-3p | 19-miRNA |
| 478606_mir | MIMAT0000245 | hsa-miR-30d-5p | 19- and 14-miRNA |
| 478012_mir | MIMAT0004504 | hsa-miR-31-3p | 19- and 7-miRNA |
| 478015_mir | MIMAT0000089 | hsa-miR-31-5p | 19- and 7-miRNA |
| 478074_mir | MIMAT0000728 | hsa-miR-375-3p | 14-miRNA |
| 478928_mir | MIMAT0019812 | hsa-miR-4709-3p | 19-miRNA |
| 479018_mir | MIMAT0003254 | hsa-miR-548b-3p | 19-miRNA |
| 477852_mir | MIMAT0004800 | hsa-miR-550a-5p | 19- and 7-miRNA |
| 479064_mir | MIMAT0004797 | hsa-miR-582-3p | 19- and 14-miRNA |
| 478167_mir | MIMAT0003249 | hsa-miR-584-5p | 19-, 14-miRNA and 7-miRNA |
| 478195_mir | MIMAT0006790 | hsa-miR-675-3p | 19-miRNA |
| 478197_mir | MIMAT0004926 | hsa-miR-708-5p | 14-miRNA |
| 478214_mir | MIMAT0000441 | hsa-miR-9-5p | 19- and 14-miRNA |
| 477860_mir | MIMAT0000069 | hsa-miR-16-5p | house-keeping miRNA |
| 478411_mir | MIMAT0000177 | ath-miR159a | spike-in miRNA |

**References**

1. Bray, F.; Ferlay, J.; Soerjomataram, I.; Siegel, R.L.; Torre, L.A.; Jemal, A. Global cancer statistics 2018: GLOBOCAN estimates of incidence and mortality worldwide for 36 cancers in 185 countries. CA: A Cancer Journal for Clinicians 2018, 68, 394-424, doi:10.3322/caac.21492.
2. The National Lung Screening Trial Research Team Reduced Lung-CancerMortality with Low-Dose Computed Tomographic Screening. N Engl J Med 2011, 365, 395-409, doi:10.1056/NEJMoa1102873.
3. de Koning, H.J.; van der Aalst, C.M.; de Jong, P.A.; Scholten, E.T.; Nackaerts, K.; Heuvelmans, M.A.; Lammers, J.-W.J.; Weenink, C.; Yousaf-Khan, U.; Horeweg, N.; et al. Reduced Lung-Cancer Mortality with Volume CT Screening in a Randomized Trial. New England Journal of Medicine 2020, 382, 503-513, doi:10.1056/NEJMoa1911793.
4. Siegel, R.L.; Miller, K.D.; Jemal, A. Cancer statistics, 2020. CA: A Cancer Journal for Clinicians 2020, 70, 7-30, doi:10.3322/caac.21590.
5. Bianchi, F.; Nuciforo, P.; Vecchi, M.; Bernard, L.; Tizzoni, L.; Marchetti, A.; Buttitta, F.; Felicioni, L.; Nicassio, F.; Fiore, P.P.D. Survival prediction of stage I lung adenocarcinomas by expression of 10 genes. J Clin Invest 2007, 117, 3436-3444, doi:10.1172/JCI32007.
6. Dama, E.; Melocchi, V.; Dezi, F.; Pirroni, S.; Carletti, R.M.; Brambilla, D.; Bertalot, G.; Casiraghi, M.; Maisonneuve, P.; Barberis, M.; et al. An Aggressive Subtype of Stage I Lung Adenocarcinoma with Molecular and Prognostic Characteristics Typical of Advanced Lung Cancers. Clin Cancer Res 2017, 23, 62-72, doi:10.1158/1078-0432.CCR-15-3005.
7. Li, J.; Smyth, P.; Flavin, R.; Cahill, S.; Denning, K.; Aherne, S.; Guenther, S.M.; O'Leary, J.J.; Sheils, O. Comparison of miRNA expression patterns using total RNA extracted from matched samples of formalin-fixed paraffin-embedded (FFPE) cells and snap frozen cells. BMC Biotechnology 2007, 7, 36, doi:10.1186/1472-6750-7-36.
8. Hall, J.S.; Taylor, J.; Valentine, H.R.; Irlam, J.J.; Eustace, A.; Hoskin, P.J.; Miller, C.J.; West, C.M.L. Enhanced stability of microRNA expression facilitates classification of FFPE tumour samples exhibiting near total mRNA degradation. British Journal of Cancer 2012, 107, 684-694, doi:10.1038/bjc.2012.294.
9. Jung, M.; Schaefer, A.; Steiner, I.; Kempkensteffen, C.; Stephan, C.; Erbersdobler, A.; Jung, K. Robust MicroRNA Stability in Degraded RNA Preparations from Human Tissue and Cell Samples. Clin Chem 2010, 56, 998-1006, doi:10.1373/clinchem.2009.141580.
10. Simon, R.; Lam, A.; Li, M.-C.; Ngan, M.; Menenzes, S.; Zhao, Y. Analysis of Gene Expression Data Using BRB-Array Tools. Cancer Inform 2007, 3, 11-17.
11. Love, M.I.; Huber, W.; Anders, S. Moderated estimation of fold change and dispersion for RNA-seq data with DESeq2. Genome Biology 2014, 15, 550, doi:10.1186/s13059-014-0550-8.
12. Lachmann, A.; Giorgi, F.M.; Lopez, G.; Califano, A. ARACNe-AP: gene network reverse engineering through adaptive partitioning inference of mutual information. Bioinformatics 2016, 32, 2233-2235, doi:10.1093/bioinformatics/btw216.
13. Sticht, C.; Torre, C.D.L.; Parveen, A.; Gretz, N. miRWalk: An online resource for prediction of microRNA binding sites. PLOS ONE 2018, 13, e0206239, doi: 1 0.1371/journal.pone.0206239.
14. Bianchi et al. A serum circulating miRNA diagnostic test to identify asymptomatic high-risk individuals with early stage lung cancer. Embo Mol. Med. 3 (2011), 495-503.
15. https://www.thermofisher.com/order/catalog/product/A25576
16. Vautrot V, Behm-Ansmant I. Enhanced Probe-Based RT-qPCR Quantification of MicroRNAs Using Poly(A) Tailing and 5' Adaptor Ligation. Methods Mol Biol. 2020;2065:39-54. doi: 10.1007/978-1-4939-9833-3_4. PMID: 31578686.
17. Schmittgen TD, Lee EJ, Jiang J, Sarkar A, Yang L, Elton TS, Chen C. Real-time PCR quantification of precursor and mature microRNA. Methods. 2008 Jan;44(1):31-8. doi: 10.1016/j.ymeth.2007.09.006. PMID: 18158130; PMCID: PMC2663046.

**Claims**

1. A method *in-vitro* or *ex-vivo* for identifying patients affected by aggressive lung adenocarcinoma (C1-ADC), comprising the steps of:

   a) detecting the amount of each of the **19 miRNAs** having sequence hsa-miR-193b-5p (SEQ ID NO. 3), hsa-miR-31-3p (SEQ ID NO. 11), hsa-miR-31-5p (SEQ ID NO. 12), hsa-miR-550a-5p (SEQ ID NO. 15), hsa-miR-196b-5p (SEQ ID NO. 5), hsa-miR-584-5p (SEQ ID NO. 17), hsa-miR-30d-5p (SEQ ID NO. 10), hsa-miR-582-3p (SEQ ID NO. 16), hsa-miR-9-5p (SEQ ID NO. 19), hsa-let-7c-3p (SEQ ID NO. 1), hsa-miR-138-5p (SEQ ID NO. 2), hsa-miR-196a-5p (SEQ ID NO. 4), hsa-miR-203a-3p (SEQ ID NO. 6), hsa-miR-215-5p (SEQ ID NO. 7), hsa-miR-2355-3p (SEQ ID NO. 3); hsa-miR-30d-3p (SEQ ID NO. 9), hsa-miR-4709-3p (SEQ ID NO. 13), hsa-miR-548b-3p (SEQ ID NO. 14) and hsa-miR-675-3p (SEQ ID NO. 18); or of each of the **14 miRNAs** having sequence hsa-miR-196b-5p (SEQ ID NO. 5), hsa-miR-584-5p (SEQ ID NO. 17), hsa-miR-30d-5p (SEQ ID NO. 10), hsa-miR-582-3p (SEQ ID NO. 16), hsa-miR-9-5p (SEQ ID NO. 19), hsa-miR-193b-3p (SEQ ID NO. 23), hsa-miR-135b-5p (SEQ ID NO. 20), hsa-miR-187-3p (SEQ ID NO. 21), hsa-miR-192-5p (SEQ ID NO. 22), hsa-miR-210-3p (SEQ ID NO. 24), hsa-miR-29b-2-5p (SEQ ID NO. 25), hsa-miR-3065-3p (SEQ ID NO. 26), hsa-miR-375-3p (SEQ ID NO. 27) and hsa-miR-708-5p (SEQ ID NO. 28), or of each of the **7 mi-RNAs** having sequence hsa-miR-31-5p (SEQ ID NO. 12), hsa-miR-31-3p (SEQ ID NO. 11), hsa-miR-193b-3p (SEQ ID NO. 23), hsa-miR-193b-5p (SEQ ID NO. 3), hsa-miR-196b-5p (SEQ ID NO. 5), hsa-miR-550a-5p (SEQ ID NO. 15) and hsa-miR-584-5p (SEQ ID NO. 17) in a biological sample from a subject.

2. Method according to claim 1 further comprising step b), wherein the data obtained in step a) is normalized.

3. Method according to claim 2 further comprising step c), wherein the patients are classified either in the predicted class of subjects affected by aggressive lung adenocarcinoma or in the class of subjects affected by non-aggressive lung adenocarcinoma (nonC1-ADC).

4. Method according to claim 3, wherein the patients affected by aggressive lung adenocarcinoma comprises patients at an early-stage of the disease (stage I).

5. Method according to any of the previous claims, wherein the biological sample is a tissue sample or a body fluid, preferably said tissue sample is a fresh tissue sample, a frozen tissue sample or a FFPE tissue sample, and said body fluid is serum or plasma.

6. Method according to any of previous claims wherein the lung adenocarcinoma is a non-small cell lung carcinoma (NSCLC).

7. Method according to claim 1, wherein the miRNAs in step a) is detected by quantitative RT-PCR (qRT-PCR), or digital PCR, or RNA sequencing, or Affymetrix microarray, or custom microarray, or digital detection through molecular barcoding selected from NanoString technology.

8. Method according to claim 1, wherein the normalization of the data reported in step b) is made according to the scheme reported in below, when the miRNAs are detected by RNA sequencing:

| | RNA sequencing | | |
| --- | --- | --- | --- |
| | **For 14-miRNAs signature** | **For 19-miRNAs signature** | **For 7-miRNAs signature** |
| **STEP b- raw data normalization** | For miRNA $i$ in sample $j$: Normalized count$_{ij}$=raw count$_{ij}$/SizeFactor$_j$ Where: SizeFactor$_j$=median$_j$ (across all RatioFactor$_{ij}$), RatioFactor$_{ij}$=raw count$_{ij}$/ geomean$_i$ (across all samples$_{ij}$) | For miRNA $i$ in sample j: Normalized count$_{ij}$=raw count$_{ij}$/SizeFactor$_j$ Where: SizeFactor$_j$=median$_j$ (across all RatioFactor$_{ij}$), RatioFactor$_{ij}$=raw count$_{ij}$/ geomean$_i$ (across all samples$_{ij}$) | For miRNA $i$ in sample j: Normalized count$_{ij}$=raw count$_{ij}$/SizeFactor$_j$ Where: SizeFactor$_j$=median$_j$ (across all RatioFactor$_{ij}$), RatioFactor$_{ij}$=raw count$_{ij}$/ geomean$_i$ (across all samples$_{ij}$) |

wherein

- the normalized count$_{ij}$ of each miRNA ($i$) of each sample ($j$) is calculated as ratio of the raw count$_{ij}$ and a size factor$_j$ specific for each sample$_j$; the size factor$_j$ specific for each sample is calculated as the median of all ratio factors$_{ij}$ of that sample; ratio factors$_{ij}$ represents the ratio between raw count$_{ij}$ and the geometric mean$_j$ of all raw count$_{ij}$ relative to a specific miRNA$_i$,

9. Method according to claim 1, wherein the normalization of the data reported in step b) is made according to the scheme reported in below, when the miRNAs are detected by qRT-PCR:

| | qRT-PCR | | |
| --- | --- | --- | --- |
| | **For 14-miRNA signature** | **For 19-miRNA signature** | **For 7-miRNA signature** |
| **STEP b- raw data normalization** | For miRNA *i* in sample *j*: Normalized **Ct$_{ij}$=raw Ct$_{ij}$-SF$_j$** **where** SF$_j$=hsa-**miR-16-5p$_j$**-21.87 | For miRNA *i* in sample *j*: Normalized Ct$_{ij}$=raw Ct$_{ij}$-SF$_j$ where SF$_j$=hsa-miR-16-5p$_j$- 21.87 | For miRNA *i* in sample *j*: Normalized Ct$_{ij}$=raw Ct$_{ij}$-SF$_j$ where SF$_j$=hsa-miR-16-5p$_j$- 21.87 |

wherein:

- the normalized Ct$_{ij}$ (cycle threshold) of each miRNA (*i*) of each sample (*j*) is calculated as difference between the raw Ct$_{ij}$ and a scaling factor$_j$ (SF) specific for each sample$_j$; the scaling factor represents the difference between the raw Ct of the miRNA "hsa-miR-16-5p" used as a reference in the sample and a constant equal to 21.87.

**10.** Method according to any of the previous claims, wherein the classification of the class of subjects affected by aggressive lung adenocarcinoma or of subjects affected by non-aggressive lung adenocarcinoma of step c) is calculated by the following formula:

$$
\text{predicted class} = \begin{Bmatrix} C1, & \dfrac{1}{1+e^{-z}} \geq 0.5 \\ \text{nonC1}, & \dfrac{1}{1+e^{-z}} < 0.5 \end{Bmatrix}
$$

wherein:

- in a model of 19-miRNAs analyzed by RNA sequencing :

z=-8.2029+(-0.2651*hsa-let-7c-3p)+(0.1709*hsa-miR-138-5p)+(0.1443*hsa-miR-193b-5p)+(0.0200*hsa-miR-196a-5p)+(0.0464*hsa-miR-196b-5p)+(0.2297*hsa-miR-203a-3p)+(0.1285*hsa-miR-215-5p)+(0.3933*hsa-miR-2355-3p)+(-0.2220*hsa-miR-30d-3p)+(-0.1874*hsa-miR-30d-5p)+(0.1535*hsa-miR-31-3p)+(0.0326*hsa-miR-31-5p)+(-0.3032*hsa-miR-4709-3p)+(-0.1672*hsa-miR-548b-3p)+(0.1529*hsa-miR-550a-5p) +(0.1132*hsa-miR-582-3p)+(0.5229*hsa-miR-584-5p)+(0.1314*hsa-miR-675-3p)+(0.0497*hsa-miR-9-5p);

- in a model of 14-miRNAs analyzed by RNA sequencing:

z=-5.4414+(-0.1028*hsa-miR-135b-5p)+(-0.0486*hsa-miR-187-3p)+(0.2828*hsa-  miR-192-5p)+(0.1977*hsa-miR-193b-3p)+(0.0201*hsa-miR-196b-5p)+(0.1908*hsa-          miR-210-3p)+(-0.5074*hsa-miR-29b-2-5p) +(0.0384*hsa-miR-3065-3p)+(-0.3312*hsa-miR-30d-5p)+(-0.0475*hsa-miR-375-3p)+(0.1895*hsa-miR-582-3p)+(0.5606*hsa-miR-584-5p)+(0.2663*hsa-miR-708-5p)+(0.0435*hsa-miR-9-5p); and

- in model 7-miRNAs analyzed by RNA sequencing:

z      =      -8.5210+(0.1171*hsa-miR-193b-3p)+(0.2233*hsa-miR-193b-5p)+(0.1341*hsa-miR-196b-5p) +(0.1554*hsa-miR-31-3p)+(0.0584*hsa-miR-31-5p)+(0.3622*hsa-miR-550a-5p)+(0.4683*hsa-miR-584-5p);

or in alternative,
- in a model of 7-miRNAs analyzed by qRT-PCR :

z= 2.2920 +(hsa-miR-193b-3p*0.1295)+(hsa-miR-193b-5p*0.0920)+(hsa-miR-196b-5p*-0.1310)+(hsa-miR-31-3p*-0.2116)+(hsa-miR-31-5p*-0.2724)+(hsa-miR-550a-5p*0.2717)+(hsa-miR-584-5p*0.0413).

11. Method according to any of the previous claims, for the identification of patients included in the group of aggressive lung adenocarcinoma with a poor-prognosis, selected from patients with shorter overall survival, and/or patients with shorter disease-free survival, and/or patients responsive to a treatment, and/or with patients with metastatic disease which can include, but not limited to, patients with early-stage disease (stage I).

12. Method according to any of the previous claims, for the identification of patients included in the group of non aggressive lung adenocarcinoma with a good-prognosis, selected from patients with longer overall survival, and/or patients with longer disease-free survival, and/or patients responsive to treatment, patients and/or without metastatic disease which can include, but not limited to, patients with early-stage disease (stage I).

13. Method according to any of the previous claims for the prognostic risk stratification of lung adenocarcinomas (C1-ADC) and/or for the identification of alternative therapeutic options after surgery, selected from systemic adjuvant chemotherapy, selected from platinum-based combinations, preferably cisplatin, carboplatin plus a third generation agents such as gemcitabine, vinorelbine, a taxane, or camptothecin, molecular targeted therapeutics, immunotherapy, radiotherapy, or a combination thereof.

**Patentansprüche**

1. Verfahren *in vitro* oder ex *vivo* zum Identifizieren von Patienten, die von einem aggressiven Lungenadenokarzinom (C1-ADC) betroffen sind, umfassend die folgenden Schritte:

   a) Nachweisen der Menge jeder der **19 miRNAs** mit der Sequenz hsa-miR- 193b-5p (SEQ ID NO. 3), hsa-miR-31-3p (SEQ ID NO. 11), hsa-miR-31-5p (SEQ ID NO. 12), hsa-miR-550a-5p (SEQ ID NO. 15), hsa-miR-196b-5p (SEQ ID NO. 5), hsa-miR-584-5p (SEQ ID NO. 17), hsa-miR-30d-5p (SEQ ID NO. 10), hsa-miR-582-3p (SEQ ID NO. 16), hsa-miR-9-5p (SEQ ID NO. 19), hsa-let-7c-3p (SEQ ID NO. 1), hsa-miR-138-5p (SEQ ID NO. 2), hsa-miR-196a-5p (SEQ ID NO. 4), hsa-miR-203a-3p (SEQ ID NO. 6), hsa-miR-215-5p (SEQ ID NO. 7), hsa-miR-2355-3p (SEQ ID NO. 3); hsa-miR-30d-3p (SEQ ID NO. 9), hsa-miR-4709-3p (SEQ ID NO. 13), hsa-miR-548b-3p (SEQ ID NO. 14) und hsa-miR-675-3p (SEQ ID NO. 18); oder von jeder der **14 miRNAs** mit der Sequenz hsa-miR-196b-5p (SEQ ID NO. 5), hsa-miR-584-5p (SEQ ID NO. 17), hsa-miR-30d-5p (SEQ ID NO. 10), hsa-miR-582-3p (SEQ ID NO. 16), hsa-miR-9-5p (SEQ ID NO. 19), hsa-miR-193b-3p (SEQ ID NO. 23), hsa-miR- 135b-5p (SEQ ID NO. 20), hsa-miR-187-3p (SEQ ID NO. 21), hsa-miR-192-5p (SEQ ID NO. 22), hsa-miR-210-3p (SEQ ID NO. 24), hsa-miR-29b-2-5p (SEQ ID NO. 25), hsa-miR-3065-3p (SEQ ID NO. 26), hsa-miR-375-3p (SEQ ID NO. 27) und hsa-miR-708-5p (SEQ ID NO. 28), oder von jeder der **7 mi-RNAs** mit der Sequenz hsa-miR-31-5p (SEQ ID NO. 12), hsa-miR-31-3p (SEQ ID NO. 11), hsa-miR-193b-3p (SEQ ID NO. 23), hsa-miR-193b-5p (SEQ ID NO. 3), hsa-miR-196b-5p (SEQ ID NO. 5), hsa-miR-550a-5p (SEQ ID NO. 15) und hsa-miR-584-5p (SEQ ID NO. 17) in einer biologischen Probe eines Subjekts.

2. Verfahren nach Anspruch 1, ferner umfassend Schritt b), wobei die in Schritt a) erhaltenen Daten normalisiert werden.

3. Verfahren nach Anspruch 2, ferner umfassend Schritt c), wobei die Patienten entweder in die vorhergesagte Klasse von Subjekten, die von einem aggressiven Lungenadenokarzinom betroffen sind, oder in die Klasse von Subjekten, die von einem nichtaggressiven Lungenadenokarzinom betroffen sind (nonC1-ADC), eingestuft werden.

4. Verfahren nach Anspruch 3, wobei die von einem aggressiven Lungenadenokarzinom betroffenen Patienten Patienten in einem frühen Stadium der Krankheit (Stadium I) umfassen.

5. Verfahren nach einem der vorstehenden Ansprüche, wobei die biologische Probe eine Gewebeprobe oder ein Körperfluid ist, vorzugsweise ist die Gewebeprobe eine Frischgewebeprobe, eine gefrorene Gewebeprobe oder eine FFPE-Gewebeprobe, und das Körperfluid ist Serum oder Plasma.

6. Verfahren nach einem der vorstehenden Ansprüche, wobei das Lungenadenokarzinom ein nicht-kleinzelliges Lungenkarzinom (NSCLC) ist.

7. Verfahren nach Anspruch 1, wobei die miRNAs in Schritt a) durch quantitative RT-PCR (qRT-PCR) oder digitale PCR oder RNA-Sequenzierung oder Affymetrix-Microarray oder kundenspezifisches Microarray oder digitale Detektion durch molekulares Barcoding, ausgewählt aus der NanoString-Technologie, nachgewiesen wird.

8. Verfahren nach Anspruch 1, wobei die Normalisierung der in Schritt b) berichteten Daten nach dem unten angegebenen Schema erfolgt, wenn die miRNAs durch RNA-Sequenzierung nachgewiesen werden:

| | RNA-Sequenzierung | | |
| | Für 14-miRNAs-Signatur | Für 19-miRNAs-Signatur | Für 7-miRNAs-Signatur |
| --- | --- | --- | --- |
| **SCHRITT b - Normalisierung der Rohdaten** | Für miRNA i in Probe $j$:<br>Normalisierte Anzahl$_{ij}$=Rohzahl$_{ij}$/Größenfaktor$_j$<br>Wobei: Größenfaktor$_j$=Mittel$_j$ (über alle Verhältnisfaktor $_{ij}$),<br>Verhältnisfaktor$_{ij}$=Rohzahl$_{ij}$/Geom ittel$_i$ (über alle Proben$_{ij}$) | Für miRNA i in Probe $j$:<br>Normalisierte Anzahl$_{ij}$=Rohzahl$_{ij}$/GrbBenfaktor$_j$<br>Wobei: GröBenfaktor$_j$=Mittel$_j$ (über alle Verhältnisfaktor $_{ij}$),<br>Verhältnisfaktor$_j$=Rohzahl$_{ij}$/Geom ittel$_i$ (über alle Proben$_{ij}$) | Für miRNA i in Probe $j$:<br>Normalisierte Anzahl$_{ij}$=Rohzahl$_{ij}$/Größenfaktor$_j$<br>Wobei: Größenfaktor$_j$=Mittel$_j$ (über alle Verhältnisfaktor$_{ij}$),<br>Verhältnisfaktor$_{ij}$=Rohzahl$_{ij}$/Geomi ttel$_i$ (über alle Proben$_{ij}$) |

wobei

- die normalisierte Zahl$_{ij}$ jeder miRNA ($i$) jeder Probe ($j$) als Verhältnis der Rohzahl$_{ij}$ und eines für jede Probe$_j$ spezifischen Größenfaktor$_j$ berechnet wird, der für jede Probe spezifische Größenfaktor$_j$ als das Mittel aller Verhältnisfaktoren$_{ij}$ dieser Probe berechnet wird; Verhältnisfaktoren$_{ij}$ das Verhältnis zwischen Rohzahl$_{ij}$ und dem geometrischen Mittel$_j$ aller Rohzahlen$_{ij}$ relativ zu einer spezifischen miRNA$_i$ darstellt,

9. Verfahren nach Anspruch 1, wobei die Normalisierung der in Schritt b) berichteten Daten nach dem unten angegebenen Schema erfolgt, wenn die miRNAs durch qRT-PCR nachgewiesen werden:

| | qRT-PCR | | |
| | Für 14-miRNA-Signatur | Für 19-miRNA-Signatur | Für 7-miRNA-Signatur |
| --- | --- | --- | --- |
| **SCHRITT b - Normalisierung der Rohdaten** | Für miRNA $i$ in Probe $j$:<br>Normalisierte Ct$_{ij}$=Roh-Ct$_{ij}$-SF$_j$<br>wobei SF$_j$=hsa-miR-16-5p$_j$-21,87 | Für miRNA $i$ in Probe $j$:<br>Normalisierte Ct$_{ij}$=Roh-Ct$_{ij}$-SF$_j$<br>wobei SF$_j$=hsa-miR-16-5p$_j$-21, 87 | Für miRNA $i$ in Probe $j$:<br>Normalisierte Ct$_{ij}$-Roh-Ct$_{ij}$-SF$_j$<br>wobei SF$_j$=hsa-miR-16-5p$_j$-21, 87 |

wobei:

- die normalisierte Ct$_{ij}$ (Zyklusschwelle) jeder miRNA ($i$) jeder Probe ($j$) als Differenz zwischen der Roh-Ct$_{ij}$ und einem Skalierungsfaktor$_j$ (SF), der für jede Probe$_j$ spezifisch ist, berechnet wird, wobei der Skalierungsfaktor die Differenz zwischen der Roh-Ct der miRNA "hsa-miR-16-5p", die als Referenz in der Probe verwendet wird, und einer Konstante gleich 21,87 darstellt.

10. Verfahren nach einem der vorstehenden Ansprüche, wobei die Klassifizierung der Klasse von Subjekten, die von einem aggressiven Lungenadenokarzinom betroffen sind, oder von Subjekten, die von einem nichtaggressiven Lungenadenokarzinom betroffen sind, in Schritt c) nach folgender Formel berechnet wird:

$$\text{vorhergesagte Klasse} = \begin{cases} C1, & \frac{1}{1+e^{-z}} \geq 0{,}5 \\ nonC1, & \frac{1}{1+e^{-z}} < 0{,}5 \end{cases}$$

wobei:

- in einem Modell von 19-miRNAs, die durch RNA-Sequenzierung analysiert wurden:

z=-8,2029+(-0,2651\*hsa-let-7c-3p)+(0,1709\*hsa-miR-138-5p)+(0,1443\*hsa-miR-193b-5p)+(0,0200\*hsa-miR-196a-5p)+(0,0464\*hsa-miR-196b-5p)+(0,2297\*hsa-miR-203a-3p)+(0,1285\*hsa-miR-215-5p)+(0,3933\*hsa-miR-2355-3p)+(-0,2220\*hsa-miR-30d-3p)+(-0,1874\*hsa-miR-30d-5p)+(0,1535\*hsa-miR-31-3p)+(0,0326\*hsa-miR-31-5p)+(-0,3032\*hsa-miR-4709-3p)+(-0,1672\*hsa-miR-548b-3p)+(0,1529\*hsa-miR-550a-5p) +(0,1132\*hsa-miR-582-3p)+(0,5229\*hsa-miR-584-5p)+(0,1314\*hsa-miR-675-3p)+(0,0497\*hsa-miR-9-5p);

- in einem Modell von 14-miRNAs, die durch RNA-Sequenzierung analysiert wurden:

z=-5,4414+(-0,1028\*hsa-miR-135b-5p)+(-0,0486\*hsa-miR- 187-3p)+(0,2828\*hsa-miR-192-5p)+(0,1977\*hsa-miR-193b-        3p)+(0,0201\*hsa-miR-196b-5p)+(0,1908\*hsa-miR-210-3p)+(-        0,5074\*hsa-miR-29b-2-5p) +(0,0384\*hsa-miR-3065-3p)+(- 0,3312\*hsa-miR-30d-5p)+(-0,0475\*hsa-miR-375- 3p)+(0,1895\*hsa-miR-582-3p)+(0,5606\*hsa-miR-584-5p)+(0,2663\*hsa-miR-708-5p)+(0,0435\*hsa-miR-9-5p); und

- in Modell 7-miRNAs, die durch RNA-Sequenzierung analysiert wurden:

z=-8,5210+(0,1171\*hsa-miR-193b-3p)+(0,2233\*hsa-miR-193b-5p)+(0,1341\*hsa-miR-196b-5p)+(0,1554\*hsa-miR-31-3p)+(0,0584\*hsa-miR-31-5p)+(0,3622\*hsa-miR-550a-5p)+(0,4683\*hsa-miR-584-5p);

oder alternativ,
- in einem Modell von 7-miRNAs, die durch qRT-PCR analysiert wurden:

z=2,2920+(hsa-miR-193b-3p\*0,1295)+(hsa-miR-193b-5p\*0,0920)+(hsa-miR-196b-5p\*-0,1310)+(hsa-miR-31-3p\*-0,2116)+(hsa-miR-31-5p\*-0,2724)+(hsa-miR-550a-5p\*0,2717)+(hsa-miR-584-5p\*0,0413).

11. Verfahren nach einem der vorstehenden Ansprüche zur Identifizierung von Patienten, die in die Gruppe der aggressiven Lungenadenokarzinome mit schlechter Prognose eingeschlossen sind, ausgewählt aus Patienten mit kürzerem Gesamtüberleben und/oder Patienten mit kürzerem krankheitsfreien Überleben und/oder Patienten, die auf eine Behandlung ansprechen, und/oder mit Patienten mit metastasierter Krankheit, die Patienten mit einem frühen Krankheitsstadium (Stadium I) einschließen können, aber nicht darauf beschränkt sind.

12. Verfahren nach einem der vorstehenden Ansprüche zur Identifizierung von Patienten, die zur Gruppe der nicht aggressiven Lungenadenokarzinome mit guter Prognose gehören, ausgewählt aus Patienten mit längerem Gesamtüberleben und/oder Patienten mit längerem krankheitsfreien Überleben und/oder Patienten, die auf die Behandlung ansprechen, und/oder Patienten ohne metastatische Krankheit, die Patienten mit einem frühen Krankheitsstadium (Stadium I) einschließen können, aber nicht darauf beschränkt sind.

13. Verfahren nach einem der vorstehenden Ansprüche zur prognostischen Risikostratifizierung von Lungenadenokarzinomen (C1-ADC) und/oder zur Ermittlung alternativer therapeutischer Optionen nach der Operation, ausgewählt aus systemischer adjuvanter Chemotherapie, ausgewählt aus Kombinationen auf Platinbasis, vorzugsweise Cisplatin, Carboplatin plus einem Mittel der dritten Generation wie Gemcitabin, Vinorelbin, einem Taxan oder Camptothecin, molekularen zielgerichteten Therapeutika, Immuntherapie, Strahlentherapie oder einer Kombination davon.

**Revendications**

1. Procédé *in vitro* ou *ex vivo* permettant l'identification des patients atteints d'un adénocarcinome pulmonaire agressif (C1-ADC), comprenant les étapes consistant à :

   a) détecter la quantité de chacune des **19 molécules miARN** ayant la séquence hsa-miR- 193b-5p (SEQ ID NO. 3), hsa-miR-31-3p (SEQ ID NO. 11), hsa-miR-31-5p (SEQ ID NO. 12), hsa-miR-550a-5p (SEQ ID NO. 15), hsa-miR-196b-5p (SEQ ID NO. 5), hsa-miR-584-5p (SEQ ID NO. 17), hsa-miR-30d-5p (SEQ ID NO. 10), hsa-miR-582-3p (SEQ ID NO. 16), hsa-miR-9-5p (SEQ ID NO. 19), hsa-let-7c-3p (SEQ ID NO. 1), hsa-miR-138-5p (SEQ ID NO. 2), hsa-miR-196a-5p (SEQ ID NO. 4), hsa-miR-203a-3p (SEQ ID NO. 6), hsa-miR-215-5p (SEQ ID NO. 7), hsa-miR-2355-3p (SEQ ID NO. 3) ; hsa-miR-30d-3p (SEQ ID NO. 9), hsa-miR-4709-3p (SEQ ID NO. 13), hsa-miR-548b-3p (SEQ ID NO. 14) et hsa-miR-675-3p (SEQ ID NO. 18) ; ou de chacune des **14 molécules miARN** ayant la séquence hsa-miR-196b-5p (SEQ ID NO. 5), hsa-miR-584-5p (SEQ ID NO. 17), hsa-miR-30d-5p (SEQ ID NO. 10), hsa-miR-582-3p (SEQ ID NO. 16), hsa-miR-9-5p (SEQ ID NO. 19), hsa-miR-193b-3p (SEQ ID NO. 23), hsa-miR- 135b-5p (SEQ ID NO. 20), hsa-miR-187-3p (SEQ ID NO. 21), hsa-miR-192-5p (SEQ ID NO. 22), hsa-miR-210-3p (SEQ ID NO. 24), hsa-miR-29b-2-5p (SEQ ID NO. 25), hsa-miR-3065-3p (SEQ ID NO. 26), hsa-miR-375-3p (SEQ ID NO. 27) et hsa-miR-708-5p (SEQ ID NO. 28), ou de chacune des **7 molécules miARN** ayant la séquence hsa-miR-31-5p (SEQ ID NO. 12), hsa-miR-31-3p (SEQ ID NO. 11), hsa-miR-193b-3p (SEQ ID NO. 23), hsa-miR-193b-5p (SEQ ID NO. 3), hsa-miR-196b-5p (SEQ ID NO. 5), hsa-miR-550a-5p (SEQ ID NO. 15) et hsa-miR-584-5p (SEQ ID NO. 17) dans un échantillon biologique prélevé sur un sujet.

2. Procédé selon la revendication 1, comprenant en outre l'étape b), dans laquelle les données obtenues à l'étape a) sont normalisées.

3. Procédé selon la revendication 2 comprenant en outre l'étape c), dans lequel les patients sont classés soit dans la classe prédite des sujets atteints d'un adénocarcinome pulmonaire agressif, soit dans la classe des sujets atteints d'un adénocarcinome pulmonaire non agressif (nonC1-ADC).

4. Procédé selon la revendication 3, dans lequel les patients atteints d'un adénocarcinome pulmonaire agressif comprennent des patients se trouvant à un stade précoce de la maladie (stade I).

5. Procédé selon l'une quelconque des revendications précédentes, dans lequel l'échantillon biologique est un échantillon de tissu ou un fluide corporel, de préférence ledit échantillon de tissu est un échantillon de tissu frais, un échantillon de tissu congelé ou un échantillon de tissu FFPE, et ledit fluide corporel est du sérum ou du plasma.

6. Procédé selon l'une quelconque des revendications précédentes dans lequel l'adénocarcinome pulmonaire est un carcinome pulmonaire non à petites cellules (CPNPC).

7. Procédé selon la revendication 1, dans lequel les molécules miARN à l'étape a) sont détectées par RT-PCR quantitative (qRT-PCR), ou par PCR numérique, ou par séquençage d'ARN, ou par puce à ADN Affymetrix, ou par puce à ADN personnalisée, ou par détection numérique au moyen d'un codage à barres moléculaire sélectionné à partir de la technologie NanoString.

8. Procédé selon la revendication 1, dans lequel la normalisation des données rapportées à l'étape b) est effectuée selon le schéma rapporté ci-dessous, lorsque les molécules miARN sont détectées par séquençage d'ARN :

| Séquençage d'ARN | | |
|---|---|---|
| miARN Pour la signature de 14 molécules miARN | Pour la signature de 19 molécules miARN | Pour la signature de 7 molécules miARN |
| Pour la molécule miARN $i$ dans l'échantillon $j$ : Nombre normalisé$_{ij}$=nombre brut$_{ij}$/facteur de taille$_j$ | Pour la molécule miARN $i$ dans l'échantillon $j$ : Nombre normalisé$_{ij}$=nombre brut$_{ij}$/facteur de taille$_j$ | Pour la molécule miARN $i$ dans l'échantillon $j$ : Nombre normalisé$_{ij}$=nombre brut$_{ij}$/facteur de taille$_j$ |

(continued)

|  | Séquençage d'ARN | | |
|  | miARN Pour la signature de 14 molécules miARN | Pour la signature de 19 molécules miARN | Pour la signature de 7 molécules miARN |
| --- | --- | --- | --- |
| ÉTAPE b - Normalisation des données brutes | Où : <br><br> Facteur de taille$_j$=médiane$_j$ (sur tous les facteurs de rapport$_{ij}$), <br><br> Facteur de rapport$_{ij}$=nombre brut$_{ij}$/moyenne géométrique$_i$ (pour tous les échantillons$_{ij}$) | Où : <br><br> Facteur de taille$_j$=médiane$_j$ (sur tous les facteurs de rapport$_{ij}$), <br><br> Facteur de rapport$_{ij}$=nombre brut$_{ij}$/moyenne géométrique$_i$ (sur tous les échantillons$_{ij}$) | Où : <br><br> Facteur de taille$_j$=médiane$_j$ (sur tous les facteurs de rapport$_{ij}$), <br><br> Facteur de rapport$_{ij}$=nombre brut$_{ij}$/moyenne géométrique$_i$ (sur tous les échantillons$_{ij}$) |

dans lequel

- le nombre normalisé$_{ij}$ de chaque molécule miARN ($i$) de chaque échantillon ($j$) est calculé comme le rapport entre le nombre brut$_{ij}$ et un facteur de taille$_j$ spécifique pour chaque échantillon$_j$, le facteur de taille$_j$ spécifique pour chaque échantillon est calculé comme la médiane de tous les facteurs de rapport$_{ij}$ de cet échantillon ; les facteurs de rapport$_{ij}$ représentent le rapport entre le nombre brut$_{ij}$ ; et la moyenne$_j$ géométrique de tous les nombres bruts$_{ij}$ relatifs à une molécule miARN$_i$ spécifique,

9. Procédé selon la revendication 1, dans lequel la normalisation des données rapportées à l'étape b) est effectuée selon le schéma rapporté ci-dessous, lorsque les molécules miARN sont détectées par qRT-PCR :

|  | qRT-PCR | | |
|  | *miARN* Pour la signature de 14 molécules *miARN* | Pour la signature de 19 molécules *miARN* | Pour la signature *de* 7 molécules *miARN* |
| --- | --- | --- | --- |
| ÉTAPE b - Normalisation des données brutes | Pour la molécule miARN $i$ dans l'échantillon $j$ : <br> Ct$_{ij}$ normalisée=Ct$_{ij}$ brute-SF$_j$ <br><br> où SF$_j$=hsa-miR-16-5p$_j$-21,87 | Pour la molécule miARN $i$ dans l'échantillon $j$ : <br> Ct$_{ij}$ normalisée=Ct$_{ij}$ brute-SF$_j$ <br><br> où SF$_j$=hsa-miR-16-5p$_j$-21,87 | Pour la molécule miARN $i$ dans l'échantillon $j$ : <br> Ct$_{ij}$ normalisée=Ct$_{ij}$ brute-SF$_j$ <br><br> ou SF$_j$=hsa-miR-16-5p$_j$-21,87 |

dans lequel :

- la valeur Ct$_{ij}$ normalisée (seuil de cycle) de chaque miARN ($i$) de chaque échantillon ($j$) est calculée comme étant la différence entre la valeur Ct$_{ij}$ brute et un facteur d'échelle$_j$ (SF) spécifique pour chaque échantillon$_j$, le facteur d'échelle représente la différence entre la valeur Ct brute de la molécule miARN « hsa-miR-16-5p » utilisée comme référence dans l'échantillon et une constante égale à 21,87.

10. Procédé selon l'une quelconque des revendications précédentes, dans lequel la classification de la classe de sujets atteints d'un adénocarcinome pulmonaire agressif ou de sujets atteints d'un adénocarcinome pulmonaire non agressif à l'étape c) est calculée à l'aide de la formule suivante :

$$
\text{classe prédite} = \begin{cases} C1, & \frac{1}{1+e^{-z}} \geq 0,5 \\ nonC1, & \frac{1}{1+e^{-z}} < 0,5 \end{cases}
$$

dans lequel :

- dans un modèle de 19 molécules miARN analysées par séquençage d'ARN :

z=-8,2029+(-0,2651*hsa-let-7c-3p)+(0,1709*hsa-miR-138-        5p)+(0,1443*hsa-miR-193b-5p)+(0,0200*hsa-miR-196a-        5p)+(0,0464*hsa-miR-196b-5p)+(0,2297*hsa-miR-203a-        3p)+(0,1285*hsa-miR-215-5p) +(0,3933*hsa-miR-2355-3p)+(-        0,2220*hsa-miR-30d-3p)+(-0,1874*hsa-miR-30d-5p)+(0,1535*hsa-miR-31-3p)+(0,0326*hsa-miR-31-5p)+(-0,3032*hsa-miR-4709-3p)+(-0,1672*hsa-miR-548b-3p)+(0,1529*hsa-miR-550a-5p)+(0,1132*hsa-miR-582-3p)+(0,5229*hsa-miR-584-5p)+(0,1314*hsa-miR-675-3p)+(0,0497*hsa-miR-9-5p) ;

- dans un modèle de 14 molécules miARN analysées par séquençage d'ARN :

z=-5,4414+(-0,1028*hsa-miR-135b-5p)+(-0,0486*hsa-miR-187-3p)+(0,2828*hsa-miR-192-5p)+(0,1977*hsa-miR-193b-3p)+(0,0201*hsa-miR-196b-5p)+(0,1908*hsa-miR-210-3p)+(-0,5074*hsa-miR-29b-2-5p) +(0,0384*hsa-miR-3065-3p)+(-0,3312*hsa-miR-30d-5p)+(-0,0475*hsa-miR-375-3p)+(0,1895*hsa-miR-582-3p)+(0,5606*hsa-miR-584-5p)+(0,2663*hsa-miR-708-5p)+(0,0435*hsa-miR-9-5p) ; et

- dans un modèle de 7 molécules miARN analysées par séquençage d'ARN :

z=-8,5210+(0,1171*hsa-miR-193b-3p)+(0,2233*hsa-miR-193b-5p)+(0,1341*hsa-miR-196b-5p)+(0,1554*hsa-miR-31-3p)+(0,0584*hsa-miR-31-5p)+(0,3622*hsa-miR-550a-5p)+(0,4683*hsa-miR-584-5p) ;

ou bien,
- dans un modèle de 7 molécules miARN analysées par qRT-PCR :

z=2,2920+(hsa-miR-193b-3p*0,1295)+(hsa-miR-193b-5p*0,0920)+(hsa-miR-196b-5p*-0,1310)+(hsa-miR-31-3p*-0,2116)+(hsa-miR-31-5p*-0,2724)+(hsa-miR-550a-5p*0,2717)+(hsa-miR-584-5p*0,0413).

11. Procédé selon l'une quelconque des revendications précédentes, permettant l'identification de patients inclus dans le groupe d'adénocarcinome pulmonaire agressif de mauvais pronostic sélectionnés parmi les patients avec une survie globale plus courte, et/ou les patients avec une survie sans récidive plus courte, et/ou les patients répondant à un traitement, et/ou les patients avec une maladie métastatique, ce qui peut inclure, sans s'y limiter, les patients avec une maladie à un stade précoce (stade I).

12. Procédé selon l'une quelconque des revendications précédentes, permettant l'identification de patients inclus dans le groupe d'adénocarcinome pulmonaire non agressif de bon pronostic sélectionnés parmi les patients avec une survie globale plus longue, et/ou les patients avec une survie sans récidive plus longue, et/ou les patients répondant au traitement, et/ou les patients sans maladie métastatique, ce qui peut inclure, sans s'y limiter, les patients avec une maladie à un stade précoce (stade I).

13. Procédé selon l'une quelconque des revendications précédentes permettant la stratification du risque pronostique des adénocarcinomes pulmonaires (C1-ADC) et/ou permettant l'identification d'options thérapeutiques alternatives après une intervention chirurgicale, sélectionnées parmi la chimiothérapie adjuvante systémique, sélectionnées parmi des associations à base de platine, de préférence le cisplatine, le carboplatine associé à des agents de troisième génération tels que la gemcitabine, la vinorelbine, un taxane ou la camptothécine, des thérapies moléculaires ciblées, l'immunothérapie, la radiothérapie ou une combinaison de celles-ci.

EP 4 214 335 B1

# Figure 1. Flow chart of study design with data sets and analysis.

Figure 1. Flow chart of study design with data sets and analysis.

**Figures 2.a), b), c), d) and e)  mRNA and miRNA expression profile analysis of the TCGA-LUAD cohort.**

(Figure 2a)

EP 4 214 335 B1

# (Figure 2b)

(Figure 2c)

(Figure 2d)

## (Figure 2e)

**Figures 3.a), b), c), d), e) and f). Validation of the 7-miRNAs model.**

## (Figure 3a)

(Figure 3b)

(Figure 3c)

EP 4 214 335 B1

(Figure 3d)

Log$_2$ Ratio

3 2 1 0 -1 -2 -3

E2F4
HOXB7
E2F1
MCM6
RRM2
NUDCD1
SERPINB5
SF3B1
HSPG2
SCGB3A1

Risk Score

C1-like   nonC1-like

(Figure 3e)

(Figure 3f)

7-miRNA model

**Figure 4. AUC obtained from signatures of 14 and 19 miRNAs.**

EP 4 214 335 B1

**Figure 5. Table S1A. TCGA-LUAD cohort. 200 miRNAs significantly regulated by DESeq2 in C1 vs. nonC1 patients comparison.**

| miRNA Name | Fold Change C1 vs. nonC1 patients | Wald test p-value | Wald test adjusted p-value (Benjamini-Hochberg method) |
|---|---|---|---|
| hsa-miR-584-5p | 2.78 | 3.24E-43 | 1.24E-40 |
| hsa-miR-215-5p | 4.99 | 6.34E-40 | 1.21E-37 |
| hsa-miR-194-5p | 3.31 | 1.35E-26 | 1.72E-24 |
| hsa-miR-192-5p | 3.10 | 1.04E-22 | 9.81E-21 |
| hsa-miR-196b-5p | 3.15 | 1.28E-22 | 9.81E-21 |
| hsa-miR-31-3p | 3.21 | 2.92E-22 | 1.86E-20 |
| hsa-miR-4709-3p | 0.48 | 2.32E-21 | 1.26E-19 |
| hsa-miR-31-5p | 3.15 | 3.57E-20 | 1.71E-18 |
| hsa-miR-582-3p | 2.22 | 5.92E-20 | 2.51E-18 |
| hsa-miR-30d-5p | 0.61 | 1.25E-17 | 4.79E-16 |
| hsa-miR-30d-3p | 0.65 | 7.06E-17 | 2.45E-15 |
| hsa-miR-664a-3p | 0.68 | 1.15E-15 | 3.65E-14 |
| hsa-miR-664b-3p | 0.70 | 5.78E-14 | 1.70E-12 |
| hsa-miR-181a-5p | 0.70 | 1.05E-13 | 2.87E-12 |
| hsa-miR-30b-5p | 0.66 | 4.61E-13 | 1.17E-11 |
| hsa-miR-153-5p | 1.93 | 3.34E-12 | 7.61E-11 |
| hsa-miR-664a-5p | 0.69 | 3.39E-12 | 7.61E-11 |
| hsa-miR-138-5p | 1.90 | 5.81E-12 | 1.23E-10 |
| hsa-miR-548b-3p | 0.59 | 3.59E-11 | 7.21E-10 |
| hsa-miR-99a-5p | 0.72 | 1.07E-10 | 2.04E-09 |
| hsa-miR-421 | 1.51 | 1.84E-10 | 3.35E-09 |
| hsa-miR-550a-5p | 1.51 | 3.60E-10 | 5.98E-09 |
| hsa-miR-582-5p | 1.67 | 3.50E-10 | 5.98E-09 |
| hsa-miR-30c-5p | 0.74 | 5.25E-10 | 8.35E-09 |
| hsa-miR-128-3p | 1.31 | 7.67E-10 | 1.17E-08 |
| hsa-miR-675-3p | 2.13 | 1.07E-09 | 1.51E-08 |
| hsa-miR-101-3p | 0.76 | 1.05E-09 | 1.51E-08 |

Figure 5 continued

| | | | |
|---|---|---|---|
| hsa-miR-18a-5p | 1.47 | 1.33E-09 | 1.82E-08 |
| hsa-miR-432-5p | 0.56 | 1.51E-09 | 1.98E-08 |
| hsa-miR-942-5p | 1.37 | 3.77E-09 | 4.80E-08 |
| hsa-miR-29b-2-5p | 0.72 | 1.02E-08 | 1.23E-07 |
| hsa-miR-130b-3p | 1.43 | 1.06E-08 | 1.23E-07 |
| hsa-let-7c-5p | 0.72 | 1.06E-08 | 1.23E-07 |
| hsa-miR-3677-3p | 1.50 | 2.64E-08 | 2.96E-07 |
| hsa-miR-193b-5p | 1.46 | 3.01E-08 | 3.29E-07 |
| hsa-miR-362-5p | 0.69 | 4.33E-08 | 4.59E-07 |
| hsa-miR-195-5p | 0.75 | 5.90E-08 | 6.09E-07 |
| hsa-miR-629-5p | 1.29 | 8.99E-08 | 9.03E-07 |
| hsa-miR-181b-5p | 0.78 | 1.17E-07 | 1.15E-06 |
| hsa-miR-106b-3p | 1.34 | 1.37E-07 | 1.31E-06 |
| hsa-miR-9-5p | 1.83 | 1.78E-07 | 1.66E-06 |
| hsa-miR-766-3p | 1.30 | 2.03E-07 | 1.85E-06 |
| hsa-miR-1306-5p | 1.33 | 3.56E-07 | 3.16E-06 |
| hsa-miR-135b-5p | 0.67 | 4.26E-07 | 3.70E-06 |
| hsa-miR-30b-3p | 0.73 | 5.23E-07 | 4.44E-06 |
| hsa-miR-146b-5p | 0.74 | 5.47E-07 | 4.54E-06 |
| hsa-miR-1307-3p | 1.28 | 6.02E-07 | 4.90E-06 |
| hsa-miR-320b | 1.33 | 7.61E-07 | 6.06E-06 |
| hsa-miR-101-5p | 0.79 | 7.87E-07 | 6.14E-06 |
| hsa-miR-193b-3p | 1.43 | 1.12E-06 | 8.55E-06 |
| hsa-miR-26a-5p | 0.82 | 1.15E-06 | 8.62E-06 |
| hsa-miR-181a-3p | 0.81 | 1.22E-06 | 8.98E-06 |
| hsa-miR-497-5p | 0.81 | 1.75E-06 | 1.26E-05 |
| hsa-miR-30e-5p | 0.83 | 2.05E-06 | 1.45E-05 |
| hsa-miR-139-5p | 0.74 | 2.35E-06 | 1.63E-05 |
| hsa-miR-30e-3p | 0.82 | 2.47E-06 | 1.68E-05 |
| hsa-miR-146b-3p | 0.77 | 3.02E-06 | 2.02E-05 |

Figure 5 continued

| | | | |
|---|---|---|---|
| hsa-miR-629-3p | 1.37 | 4.10E-06 | 2.70E-05 |
| hsa-miR-362-3p | 0.76 | 5.03E-06 | 3.25E-05 |
| hsa-miR-151b | 0.77 | 5.12E-06 | 3.26E-05 |
| hsa-miR-29c-5p | 0.79 | 5.32E-06 | 3.33E-05 |
| hsa-miR-125a-5p | 0.83 | 5.55E-06 | 3.42E-05 |
| hsa-miR-3065-3p | 0.67 | 6.90E-06 | 4.18E-05 |
| hsa-miR-660-5p | 0.79 | 7.88E-06 | 4.70E-05 |
| hsa-miR-2355-5p | 1.27 | 8.33E-06 | 4.89E-05 |
| hsa-miR-205-5p | 1.75 | 9.15E-06 | 5.30E-05 |
| hsa-miR-2355-3p | 1.30 | 9.39E-06 | 5.36E-05 |
| hsa-miR-23a-3p | 1.15 | 9.87E-06 | 5.50E-05 |
| hsa-miR-338-3p | 0.68 | 9.94E-06 | 5.50E-05 |
| hsa-miR-106b-5p | 1.21 | 1.01E-05 | 5.51E-05 |
| hsa-miR-1301-3p | 1.28 | 1.11E-05 | 5.99E-05 |
| hsa-miR-223-3p | 1.34 | 1.17E-05 | 6.23E-05 |
| hsa-miR-365b-3p | 1.28 | 1.40E-05 | 7.30E-05 |
| hsa-miR-365a-3p | 1.28 | 1.43E-05 | 7.35E-05 |
| hsa-miR-576-5p | 1.25 | 1.44E-05 | 7.35E-05 |
| hsa-miR-193a-5p | 1.25 | 1.81E-05 | 9.08E-05 |
| hsa-miR-4772-3p | 1.31 | 1.88E-05 | 9.34E-05 |
| hsa-miR-4661-5p | 1.37 | 2.01E-05 | 9.73E-05 |
| hsa-miR-769-5p | 1.20 | 1.99E-05 | 9.73E-05 |
| hsa-miR-34a-5p | 0.82 | 2.12E-05 | 1.01E-04 |
| hsa-miR-148a-5p | 0.77 | 2.50E-05 | 1.18E-04 |
| hsa-miR-20b-5p | 1.51 | 2.62E-05 | 1.22E-04 |
| hsa-miR-1266-5p | 1.33 | 2.98E-05 | 1.37E-04 |
| hsa-miR-195-3p | 0.81 | 3.04E-05 | 1.38E-04 |
| hsa-miR-95-3p | 1.39 | 4.35E-05 | 1.96E-04 |
| hsa-miR-493-5p | 0.71 | 4.48E-05 | 1.99E-04 |
| hsa-miR-187-3p | 0.65 | 5.35E-05 | 2.35E-04 |

Figure 5 continued

| | | | |
|---|---|---|---|
| hsa-miR-222-5p | 1.30 | 6.66E-05 | 2.89E-04 |
| hsa-miR-203a-3p | 1.39 | 7.33E-05 | 3.15E-04 |
| hsa-miR-431-3p | 0.68 | 7.49E-05 | 3.18E-04 |
| hsa-miR-29a-3p | 0.82 | 7.61E-05 | 3.19E-04 |
| hsa-miR-27b-3p | 0.85 | 8.41E-05 | 3.49E-04 |
| hsa-miR-331-5p | 1.18 | 8.52E-05 | 3.50E-04 |
| hsa-miR-185-3p | 1.24 | 9.59E-05 | 3.90E-04 |
| hsa-miR-193a-3p | 1.23 | 9.88E-05 | 3.97E-04 |
| hsa-miR-1296-5p | 1.24 | 1.19E-04 | 4.72E-04 |
| hsa-miR-495-3p | 0.69 | 1.26E-04 | 4.98E-04 |
| hsa-miR-532-5p | 0.83 | 1.66E-04 | 6.47E-04 |
| hsa-miR-125b-2-3p | 0.79 | 2.03E-04 | 7.83E-04 |
| hsa-miR-146a-5p | 0.79 | 2.38E-04 | 9.09E-04 |
| hsa-miR-130b-5p | 1.25 | 2.61E-04 | 9.88E-04 |
| hsa-miR-29c-3p | 0.81 | 2.84E-04 | 1.06E-03 |
| hsa-miR-27a-3p | 1.16 | 3.18E-04 | 1.18E-03 |
| hsa-miR-550a-3p | 1.26 | 3.33E-04 | 1.22E-03 |
| hsa-miR-15b-3p | 1.19 | 3.59E-04 | 1.31E-03 |
| hsa-miR-502-3p | 0.85 | 3.81E-04 | 1.37E-03 |
| hsa-miR-328-3p | 0.83 | 3.92E-04 | 1.40E-03 |
| hsa-miR-330-5p | 1.20 | 4.03E-04 | 1.43E-03 |
| hsa-miR-4662a-5p | 1.31 | 4.80E-04 | 1.68E-03 |
| hsa-miR-200a-5p | 1.24 | 4.91E-04 | 1.69E-03 |
| hsa-miR-625-3p | 1.20 | 4.92E-04 | 1.69E-03 |
| hsa-miR-26a-2-3p | 0.83 | 5.06E-04 | 1.73E-03 |
| hsa-miR-218-5p | 0.84 | 5.12E-04 | 1.73E-03 |
| hsa-miR-128-1-5p | 1.19 | 5.23E-04 | 1.75E-03 |
| hsa-miR-487b-3p | 0.72 | 5.34E-04 | 1.77E-03 |
| hsa-miR-148a-3p | 0.83 | 5.67E-04 | 1.87E-03 |
| hsa-let-7a-2-3p | 1.34 | 5.81E-04 | 1.90E-03 |

Figure 5 continued

| | | | |
|---|---|---|---|
| hsa-miR-133a-3p | 0.74 | 6.01E-04 | 1.94E-03 |
| hsa-miR-197-3p | 1.15 | 6.28E-04 | 2.02E-03 |
| hsa-miR-93-3p | 1.19 | 6.56E-04 | 2.07E-03 |
| hsa-miR-874-3p | 1.18 | 6.56E-04 | 2.07E-03 |
| hsa-miR-132-3p | 1.16 | 7.00E-04 | 2.19E-03 |
| hsa-miR-182-5p | 0.83 | 8.16E-04 | 2.53E-03 |
| hsa-miR-3934-3p | 0.81 | 8.80E-04 | 2.70E-03 |
| hsa-miR-224-3p | 0.76 | 8.83E-04 | 2.70E-03 |
| hsa-miR-708-5p | 1.25 | 8.98E-04 | 2.72E-03 |
| hsa-miR-25-3p | 1.15 | 9.72E-04 | 2.92E-03 |
| hsa-miR-21-3p | 1.18 | 1.08E-03 | 3.21E-03 |
| hsa-miR-15a-5p | 0.88 | 1.08E-03 | 3.21E-03 |
| hsa-miR-642a-5p | 1.25 | 1.13E-03 | 3.33E-03 |
| hsa-miR-339-3p | 0.86 | 1.18E-03 | 3.44E-03 |
| hsa-miR-1-3p | 0.75 | 1.26E-03 | 3.64E-03 |
| hsa-miR-1180-3p | 1.23 | 1.29E-03 | 3.71E-03 |
| hsa-miR-425-5p | 1.17 | 1.56E-03 | 4.46E-03 |
| hsa-miR-877-5p | 1.25 | 1.62E-03 | 4.54E-03 |
| hsa-miR-221-3p | 1.20 | 1.62E-03 | 4.54E-03 |
| hsa-miR-23b-3p | 0.88 | 1.74E-03 | 4.85E-03 |
| hsa-miR-363-3p | 1.32 | 1.85E-03 | 5.12E-03 |
| hsa-miR-24-2-5p | 1.16 | 2.02E-03 | 5.56E-03 |
| hsa-miR-493-3p | 0.76 | 2.08E-03 | 5.67E-03 |
| hsa-miR-22-3p | 1.10 | 2.28E-03 | 6.19E-03 |
| hsa-miR-222-3p | 1.18 | 2.61E-03 | 6.98E-03 |
| hsa-miR-99b-3p | 1.18 | 2.62E-03 | 6.98E-03 |
| hsa-miR-500b-5p | 0.84 | 2.65E-03 | 6.98E-03 |
| hsa-miR-542-3p | 0.82 | 2.63E-03 | 6.98E-03 |
| hsa-miR-212-3p | 1.22 | 2.72E-03 | 7.01E-03 |
| hsa-miR-136-5p | 0.77 | 2.71E-03 | 7.01E-03 |

Figure 5 continued

| | | | |
|---|---|---|---|
| hsa-miR-758-5p | 0.75 | 2.70E-03 | 7.01E-03 |
| hsa-miR-500a-5p | 0.84 | 2.76E-03 | 7.08E-03 |
| hsa-miR-93-5p | 1.17 | 2.86E-03 | 7.27E-03 |
| hsa-miR-339-5p | 0.86 | 2.94E-03 | 7.43E-03 |
| hsa-miR-500a-3p | 0.86 | 3.07E-03 | 7.72E-03 |
| hsa-miR-532-3p | 0.86 | 3.26E-03 | 8.14E-03 |
| hsa-miR-509-3p | 0.70 | 3.36E-03 | 8.34E-03 |
| hsa-miR-1976 | 0.88 | 3.48E-03 | 8.57E-03 |
| hsa-miR-590-5p | 1.15 | 3.74E-03 | 9.16E-03 |
| hsa-miR-200c-5p | 1.18 | 3.83E-03 | 9.31E-03 |
| hsa-miR-3065-5p | 0.78 | 4.00E-03 | 9.68E-03 |
| hsa-miR-577 | 1.38 | 4.03E-03 | 9.68E-03 |
| hsa-miR-1247-3p | 1.33 | 4.26E-03 | 1.02E-02 |
| hsa-miR-151a-5p | 0.89 | 4.46E-03 | 1.06E-02 |
| hsa-miR-1287-5p | 0.86 | 4.75E-03 | 1.12E-02 |
| hsa-miR-132-5p | 1.15 | 6.45E-03 | 1.51E-02 |
| hsa-miR-1287-3p | 0.83 | 6.66E-03 | 1.55E-02 |
| hsa-miR-217 | 0.80 | 6.86E-03 | 1.59E-02 |
| hsa-miR-106a-5p | 1.24 | 6.92E-03 | 1.59E-02 |
| hsa-miR-29b-3p | 0.87 | 7.78E-03 | 1.78E-02 |
| hsa-miR-17-5p | 1.15 | 7.89E-03 | 1.79E-02 |
| hsa-miR-508-3p | 0.74 | 8.32E-03 | 1.88E-02 |
| hsa-let-7c-3p | 0.82 | 8.45E-03 | 1.90E-02 |
| hsa-miR-514a-3p | 0.73 | 9.70E-03 | 2.17E-02 |
| hsa-miR-455-5p | 0.88 | 9.79E-03 | 2.17E-02 |
| hsa-miR-22-5p | 1.14 | 1.03E-02 | 2.27E-02 |
| hsa-miR-590-3p | 1.15 | 1.08E-02 | 2.38E-02 |
| hsa-miR-3074-5p | 1.18 | 1.09E-02 | 2.39E-02 |
| hsa-miR-181a-2-3p | 0.89 | 1.11E-02 | 2.40E-02 |
| hsa-miR-141-5p | 1.16 | 1.14E-02 | 2.45E-02 |

Figure 5 continued

| hsa-miR-708-3p | 1.17 | 1.15E-02 | 2.46E-02 |
|---|---|---|---|
| hsa-miR-29a-5p | 0.87 | 1.16E-02 | 2.47E-02 |
| hsa-miR-410-3p | 0.78 | 1.18E-02 | 2.50E-02 |
| hsa-miR-196a-5p | 1.40 | 1.28E-02 | 2.71E-02 |
| hsa-miR-625-5p | 1.15 | 1.39E-02 | 2.92E-02 |
| hsa-miR-24-1-5p | 0.88 | 1.43E-02 | 2.98E-02 |
| hsa-miR-331-3p | 1.13 | 1.49E-02 | 3.09E-02 |
| hsa-miR-4326 | 1.26 | 1.52E-02 | 3.14E-02 |
| hsa-miR-149-5p | 1.23 | 1.57E-02 | 3.23E-02 |
| hsa-miR-1307-5p | 1.18 | 1.63E-02 | 3.33E-02 |
| hsa-let-7g-5p | 0.90 | 1.64E-02 | 3.34E-02 |
| hsa-miR-4677-3p | 1.11 | 1.73E-02 | 3.50E-02 |
| hsa-miR-125a-3p | 1.13 | 1.74E-02 | 3.51E-02 |
| hsa-miR-181c-5p | 0.88 | 1.76E-02 | 3.52E-02 |
| hsa-miR-199b-5p | 0.88 | 1.84E-02 | 3.65E-02 |
| hsa-miR-501-5p | 0.87 | 1.87E-02 | 3.70E-02 |
| hsa-miR-19a-3p | 1.17 | 2.10E-02 | 4.13E-02 |
| hsa-miR-152-3p | 1.12 | 2.17E-02 | 4.25E-02 |
| hsa-miR-326 | 0.84 | 2.24E-02 | 4.37E-02 |
| hsa-miR-345-5p | 1.16 | 2.32E-02 | 4.49E-02 |
| hsa-miR-100-5p | 1.18 | 2.35E-02 | 4.50E-02 |
| hsa-miR-150-5p | 0.86 | 2.34E-02 | 4.50E-02 |
| hsa-miR-671-5p | 1.13 | 2.40E-02 | 4.58E-02 |

**Figure 6. Table S1B. TCGA-LUAD cohort. 90 miRNAs significantly regulated by BRB-ArrayTools in C1 vs. nonC1 patients comparison.**

| miRNA Name | Fold Change C1 vs nonC1 patients | Parametric p-value |
|---|---|---|
| hsa-miR-30d-5p | 0.59 | < 1.00E-07 |
| hsa-miR-30b-5p | 0.62 | < 1.00E-07 |
| hsa-miR-584-5p | 1.79 | < 1.00E-07 |
| hsa-miR-664a-3p | 0.70 | < 1.00E-07 |
| hsa-miR-192-5p | 2.17 | < 1.00E-07 |
| hsa-miR-181a-5p | 0.73 | < 1.00E-07 |
| hsa-miR-196b-5p | 2.53 | < 1.00E-07 |
| hsa-miR-29b-2-5p | 0.70 | < 1.00E-07 |
| hsa-miR-99a-5p | 0.73 | < 1.00E-07 |
| hsa-miR-128-3p | 1.28 | < 1.00E-07 |
| hsa-miR-101-3p | 0.78 | < 1.00E-07 |
| hsa-miR-30c-5p | 0.77 | < 1.00E-07 |
| hsa-miR-582-3p | 1.63 | 1.00E-07 |
| hsa-miR-194-5p | 1.84 | 5.00E-07 |
| hsa-miR-193b-3p | 1.49 | 5.00E-07 |
| hsa-miR-30e-5p | 0.82 | 7.00E-07 |
| hsa-let-7c-5p | 0.73 | 8.00E-07 |
| hsa-miR-181b-5p | 0.78 | 1.20E-06 |
| hsa-miR-193a-5p | 1.31 | 1.30E-06 |
| hsa-miR-3065-3p | 0.60 | 1.40E-06 |
| hsa-miR-629-5p | 1.25 | 3.70E-06 |
| hsa-miR-135b-5p | 0.63 | 4.40E-06 |
| hsa-miR-9-5p | 1.93 | 7.40E-06 |
| hsa-miR-106b-3p | 1.30 | 8.60E-06 |
| hsa-miR-26a-5p | 0.83 | 9.80E-06 |
| hsa-miR-497-5p | 0.81 | 1.03E-05 |
| hsa-miR-29c-5p | 0.79 | 1.20E-05 |
| hsa-miR-2355-5p | 1.27 | 1.26E-05 |
| hsa-miR-34a-5p | 0.81 | 3.06E-05 |
| hsa-miR-125a-5p | 0.84 | 3.82E-05 |
| hsa-miR-210-3p | 1.52 | 4.06E-05 |
| hsa-miR-1307-3p | 1.26 | 4.47E-05 |
| hsa-miR-375 | 0.62 | 4.53E-05 |
| hsa-miR-29a-3p | 0.81 | 5.19E-05 |
| hsa-miR-146b-3p | 0.79 | 5.72E-05 |
| hsa-miR-195-5p | 0.80 | 6.41E-05 |
| hsa-miR-181a-3p | 0.83 | 6.53E-05 |
| hsa-miR-365b-3p | 1.25 | 1.05E-04 |
| hsa-miR-27b-3p | 0.84 | 1.07E-04 |
| hsa-miR-365a-3p | 1.25 | 1.07E-04 |
| hsa-miR-1301-3p | 1.24 | 1.83E-04 |
| hsa-miR-23a-3p | 1.13 | 1.93E-04 |
| hsa-miR-660-5p | 0.81 | 1.94E-04 |
| hsa-miR-769-5p | 1.16 | 2.52E-04 |

Figure 6 continued

| | | |
|---|---|---|
| hsa-miR-1976 | 0.84 | 2.68E-04 |
| hsa-miR-590-5p | 1.21 | 2.72E-04 |
| hsa-miR-21-3p | 1.21 | 2.73E-04 |
| hsa-miR-146b-5p | 0.79 | 2.91E-04 |
| hsa-miR-30e-3p | 0.86 | 2.93E-04 |
| hsa-miR-328-3p | 0.83 | 3.63E-04 |
| hsa-miR-223-3p | 1.28 | 3.70E-04 |
| hsa-miR-197-3p | 1.16 | 4.18E-04 |
| hsa-miR-130b-3p | 1.24 | 4.30E-04 |
| hsa-miR-29c-3p | 0.81 | 6.43E-04 |
| hsa-miR-106b-5p | 1.15 | 9.18E-04 |
| hsa-miR-24-2-5p | 1.18 | 1.21E-03 |
| hsa-miR-15b-3p | 1.17 | 1.25E-03 |
| hsa-miR-23b-3p | 0.88 | 1.37E-03 |
| hsa-miR-27a-3p | 1.14 | 1.41E-03 |
| hsa-miR-187-3p | 0.64 | 1.42E-03 |
| hsa-miR-532-5p | 0.86 | 1.57E-03 |
| hsa-miR-148a-3p | 0.83 | 1.74E-03 |
| hsa-miR-218-5p | 0.84 | 1.84E-03 |
| hsa-miR-132-3p | 1.15 | 2.05E-03 |
| hsa-miR-182-5p | 0.83 | 2.15E-03 |
| hsa-miR-708-5p | 1.26 | 2.18E-03 |
| hsa-miR-542-3p | 0.81 | 2.19E-03 |
| hsa-miR-29b-3p | 0.84 | 2.30E-03 |
| hsa-miR-148a-5p | 0.81 | 2.48E-03 |
| hsa-miR-134-5p | 1.33 | 2.54E-03 |
| hsa-miR-425-5p | 1.17 | 2.73E-03 |
| hsa-miR-139-5p | 0.82 | 3.01E-03 |
| hsa-miR-339-3p | 0.87 | 3.17E-03 |
| hsa-miR-22-5p | 1.17 | 3.53E-03 |
| hsa-miR-339-5p | 0.85 | 3.87E-03 |
| hsa-miR-24-1-5p | 0.85 | 4.15E-03 |
| hsa-miR-874-3p | 1.17 | 4.33E-03 |
| hsa-miR-625-3p | 1.17 | 4.59E-03 |
| hsa-miR-326 | 0.79 | 4.71E-03 |
| hsa-miR-1287-5p | 0.85 | 5.46E-03 |
| hsa-miR-708-3p | 1.21 | 5.54E-03 |
| hsa-miR-150-5p | 0.81 | 6.66E-03 |
| hsa-miR-330-5p | 1.16 | 7.09E-03 |
| hsa-miR-151a-5p | 0.90 | 8.81E-03 |
| hsa-let-7g-5p | 0.89 | 1.09E-02 |
| hsa-miR-200c-5p | 1.17 | 1.13E-02 |
| hsa-miR-1266-5p | 1.20 | 1.15E-02 |
| hsa-miR-500a-3p | 0.88 | 1.15E-02 |
| hsa-miR-93-5p | 1.14 | 1.20E-02 |
| hsa-miR-331-3p | 1.14 | 1.23E-02 |

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2012089630 A1
- WO 2016038119 A, Bianchi F.
- AU 2012238336 B2
- CN 104818334 B

**Non-patent literature cited in the description**

- **XUELIAN LI et al.** *Journal of Translational Medicine*, 04 June 2014
- **SIRIWARDHANA CHATHURA et al.** *BMC Cancer*, 01 December 2019
- **PENG ZHUO et al.** *Oncotarget*, 08 September 2017
- **YERUKALA SATHIPATI SRINIVASULU et al.** *Scientific Reports*, 01 December 2017
- **BRAY, F.** ; **FERLAY, J.** ; **SOERJOMATARAM, I** ; **SIEGEL, R.L** ; **TORRE, L.A.** ; **JEMAL, A.** Global cancer statistics 2018: GLOBOCAN estimates of incidence and mortality worldwide for 36 cancers in 185 countries. *CA: A Cancer Journal for Clinicians*, 2018, vol. 68, 394-424
- The National Lung Screening Trial Research Team Reduced Lung-CancerMortality with Low-Dose Computed Tomographic Screening. *N Engl J Med*, 2011, vol. 365, 395-409
- **KONING, H.J** ; **VAN DER AALST, C.M.** ; **DE JONG, P.A.** ; **SCHOLTEN, E.T** ; **NACKAERTS, K.** ; **HEU-VELMANS, M.A.** ; **LAMMERS, J.-W.J** ; **WEENINK, C** ; **YOUSAF-KHAN, U** ; **HOREWEG, N. et al.** Reduced Lung-Cancer Mortality with Volume CT Screening in a Randomized Trial. *New England Journal of Medicine*, 2020, vol. 382, 503-513
- **SIEGEL, R.L** ; **MILLER, K.D.** ; **JEMAL, A.** CA: A Cancer Journal for Clinicians. *Cancer statistics*, 2020, vol. 2020 (70), 7-30
- **BIANCHI, F.** ; **NUCIFORO, P.** ; **VECCHI, M** ; **BERNARD, L** ; **TIZZONI, L.** ; **MARCHETTI, A** ; **BUTTITTA, F** ; **FELICIONI, L** ; **NICASSIO, F.** ; **FIORE, P.P.D**. Survival prediction of stage I lung adenocarcinomas by expression of 10 genes. *J Clin Invest*, 2007, vol. 117, 3436-3444
- **DAMA, E.** ; **MELOCCHI, V** ; **DEZI, F** ; **PIRRONI, S** ; **CARLETTI, R.M** ; **BRAMBILLA, D** ; **BERTALOT, G** ; **CASIRAGHI, M** ; **MAISONNEUVE, P.** ; **BARBERIS, M. et al.** An Aggressive Subtype of Stage I Lung Adenocarcinoma with Molecular and Prognostic Characteristics Typical of Advanced Lung Cancers. *Clin Cancer Res*, 2017, vol. 23, 62-72
- **LI, J** ; **SMYTH, P** ; **FLAVIN, R** ; **CAHILL, S.** ; **DENNING, K** ; **AHERNE, S** ; **GUENTHER, S.M** ; **O'LEARY, J.J.** ; **SHEILS, O**. Comparison of miRNA expression patterns using total RNA extracted from matched samples of formalin-fixed paraffin-embedded (FFPE) cells and snap frozen cells. *BMC Biotechnology*, 2007, vol. 7, 36
- **HALL, J.S** ; **TAYLOR, J.** ; **VALENTINE, H.R** ; **IRLAM, J.J.** ; **EUSTACE, A** ; **HOSKIN, P.J.** ; **MILLER, C.J** ; **WEST, C.M.L**. Enhanced stability of microRNA expression facilitates classification of FFPE tumour samples exhibiting near total mRNA degradation. *British Journal of Cancer*, 2012, vol. 107, 684-694
- **JUNG, M.** ; **SCHAEFER, A.** ; **STEINER, I.** ; **KEMP-KENSTEFFEN, C.** ; **STEPHAN, C.** ; **ERBERSDO-BLER, A.** ; **JUNG, K**. Robust MicroRNA Stability in Degraded RNA Preparations from Human Tissue and Cell Samples. *Clin Chem*, 2010, vol. 56, 998-1006
- **SIMON, R.** ; **LAM, A** ; **LI, M.-C.** ; **NGAN, M** ; **MENENZES, S** ; **ZHAO, Y**. Analysis of Gene Expression Data Using BRB-Array Tools. *Cancer Inform*, 2007, vol. 3, 11-17
- **LOVE, M.I** ; **HUBER, W.** ; **ANDERS, S**. Moderated estimation of fold change and dispersion for RNA-seq data with DESeq2. *Genome Biology*, 2014, vol. 15, 550
- **LACHMANN, A.** ; **GIORGI, F.M** ; **LOPEZ, G.** ; **CALIFANO, A**. ARACNe-AP: gene network reverse engineering through adaptive partitioning inference of mutual information. *Bioinformatics*, 2016, vol. 32, 2233-2235
- **STICHT, C.** ; **TORRE, C.D.L** ; **PARVEEN, A** ; **GRETZ, N**. miRWalk: An online resource for prediction of microRNA binding sites. *PLOS ONE*, 2018, vol. 13, e0206239
- **BIANCHI et al.** A serum circulating miRNA diagnostic test to identify asymptomatic high-risk individuals with early stage lung cancer. *Embo Mol. Med.*, 2011, vol. 3, 495-503

- **VAUTROT V** ; **BEHM-ANSMANT I**. Enhanced Probe-Based RT-qPCR Quantification of MicroRNAs Using Poly(A) Tailing and 5' Adaptor Ligation. *Methods Mol Biol.*, 2020, vol. 2065, 39-54

- **SCHMITTGEN TD** ; **LEE EJ** ; **JIANG J** ; **SARKAR A** ; **YANG L** ; **ELTON TS** ; **CHEN C**. Real-time PCR quantification of precursor and mature microRNA. *Methods*, January 2008, vol. 44 (1), 31-8